(19) Europäisches Patentamt
European Patent Office
Office européen des brevets

(11) EP 1 929 010 B1

(12) EUROPEAN PATENT SPECIFICATION

(45) Date of publication and mention of the grant of the patent:
**18.12.2013 Bulletin 2013/51**

(21) Application number: **06799601.7**

(22) Date of filing: **27.09.2006**

(51) Int Cl.:
***C12N 15/09*** *(2006.01)* ***C12P 7/62*** *(2006.01)*

(86) International application number:
**PCT/NZ2006/000251**

(87) International publication number:
**WO 2007/037706 (05.04.2007 Gazette 2007/14)**

(54) **USES OF POLYMER PARTICLES**

VERWENDUNG VON POLYMERTEILCHEN

APPLICATIONS DES PARTICULES POLYMERIQUES

(84) Designated Contracting States:
**AT BE BG CH CY CZ DE DK EE ES FI FR GB GR HU IE IS IT LI LT LU LV MC NL PL PT RO SE SI SK TR**

(30) Priority: **27.09.2005 NZ 54264405**
**12.12.2005 NZ 54409705**
**12.12.2005 NZ 54409605**

(43) Date of publication of application:
**11.06.2008 Bulletin 2008/24**

(73) Proprietor: **PolyBatics Limited**
**Palmerston North (NZ)**

(72) Inventors:
- **Rehm, Bernd, Helmut, Adam**
  **Palmerston North (NZ)**
- **Backstrom, Bjorn, Thomas**
  **Wellington (NZ)**

(74) Representative: **Walker, Ross Thomson et al**
**Forresters**
**Skygarden**
**Erika-Mann-Strasse 11**
**80636 München (DE)**

(56) References cited:

| | |
|---|---|
| **WO-A-00/06747** | **WO-A-99/35278** |
| **WO-A-2004/020623** | **WO-A-2008/006832** |
| **WO-A1-99/35278** | **AU-A1- 2003 266 922** |
| **US-A1- 2006 141 570** | |

- **BANKI MAHMOUD REZA ET AL: "Novel and economical purification of recombinant proteins: Intein-mediated protein purification using in vivo polyhydroxybutyrate (PHB) matrix association" PROTEIN SCIENCE, vol. 14, no. 6, June 2005 (2005-06), pages 1387-1395, XP002523624 ISSN: 0961-8368**
- **SANG JUN SIM ET AL: "PHA synthase activity controls the molecular weight and polydispersity of polyhydroxybutyrate in vivo" NATURE BIOTECHNOLOGY, NATURE PUBLISHING GROUP, NEW YORK, NY, US, vol. 15, no. 1, 1 January 1997 (1997-01-01), pages 63-67, XP002268360 ISSN: 1087-0156**
- **MADISON L L ET AL: "METABOLIC ENGINEERING OF POLY(3-HYDROXYALKANOATES): FROM DNA TO PLASTIC" MICROBIOLOGY AND MOLECULAR BIOLOGY REVIEWS, AMERICAN SOCIETY FOR MICROBIOLOGY, US, vol. 63, no. 1, 1 March 1999 (1999-03-01), pages 21-53, XP000869647 ISSN: 1092-2172**
- **JENDROSSEK D: "Fluorescence microscopical investigation of poly(3-hydroxybutyrate) granule formation in bacteria" BIOMACROMOLECULES MARCH/APRIL 2005 AMERICAN CHEMICAL SOCIETY US, vol. 6, no. 2, March 2005 (2005-03), pages 598-603, XP002523625**
- **BROCKELBANK J A ET AL: "Recombinant Escherichia coli strain produces a ZZ domain displaying biopolyester granules suitable for immunoglobulin G purification" APPLIED AND ENVIRONMENTAL MICROBIOLOGY, AMERICAN SOCIETY FOR MICROBIOLOGY, US, vol. 72, no. 11, 25 August 2006 (2006-08-25), - 1 November 2006 (2006-11-01) pages 7394-7397, XP003014827 ISSN: 0099-2240**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- BÄCKSTRÖM B THOMAS ET AL: "Recombinant Escherichia coli produces tailor-made biopolyester granules for applications in fluorescence activated cell sorting: functional display of the mouse interleukin-2 and myelin oligodendrocyte glycoprotein" BMC BIOTECHNOLOGY, BIOMED CENTRAL LTD. LONDON, GB, vol. 7, no. 1, 4 January 2007 (2007-01-04), page 3, XP021023603 ISSN: 1472-6750
- JANE A ATWOOD ET AL: "Protein engineering towards biotechnological production of bifunctional polyester beads" BIOTECHNOLOGY LETTERS, KLUWER ACADEMIC PUBLISHERS, DO, vol. 31, no. 1, 18 September 2008 (2008-09-18), pages 131-137, XP019639520 ISSN: 1573-6776
- KOURTZ L. ET AL.: 'A novel thiolase-reductase gene fusion promotes the production of polyhydroxybutyrate in Arabidopsis' PLANT BIOTECHNOLOGY JOURNAL vol. 3, no. 4, 2005, pages 435 - 447, XP003014824
- PETERS V. ET AL.: 'In vivo monitoring of PHA granule formation using GFP-labeled PHA synthases' FEMS MICROBIOLOGY LETTERS vol. 248, no. 1, 2005, pages 93 - 100, XP004943694
- MOLDES C. ET AL.: 'In vivo immobilisation of fusion protein on bioplastics by the novel tag BioF' APPLIED AND ENVIRONMENTAL MICROBIOLOGY vol. 70, no. 6, 2004, pages 3205 - 3212, XP003014825
- PETERS V. ET AL.: 'In vivo enzyme immobilization by use of engineered polyhydroxyalkanoate synthase' APPLIED AND ENVIRONMENTAL MICROBIOLOGY vol. 72, no. 3, 2006, pages 1777 - 1783, XP003014826
- BROCKELBANK J.A. ET AL.: 'Recombinant Escherichia coli strain produces a ZZ domain displaying biopolyester granules suitable for immunoglobulin G purification' APPLIED AND ENVIRONMENTAL MICROBIOLOGY vol. 72, no. 11, 2006, pages 7394 - 7397, XP003014827
- V\LLENKLE C ET AL: "Construction of a functional Slayer fusion protein comprising an immunoglobulin G-binding domain for development of specific adsorbents for extracorporeal blood purification", APPLIED AND ENVIRONMENTAL MICROBIOLOGY, AMERICAN SOCIETY FOR MICROBIOLOGY, US LNKD- DOI:10.1128/AEM.70.3.1514-1521.2004, vol. 70, no. 3, 1 March 2004 (2004-03-01), pages 1514-1521, XP003011043, ISSN: 0099-2240
- X ZHANG ET AL: "Control of the Escherichia coli rrnB P1 promoter strength by ppGpp", JOURNAL OF BIOLOGICAL CHEMISTRY, vol. 270, no. 19, 12 May 1995 (1995-05-12) , pages 11181-11189, XP55050600, ISSN: 0021-9258, DOI: 10.1074/jbc.270.19.11181
- KAJITANI M ET AL: "Determination of the promoter strength in the mixed transcription system: promoters of lactose, tryptophan and ribosomal protein L10 operons from Escherichia coli.", NUCLEIC ACIDS RESEARCH 11 FEB 1983, vol. 11, no. 3, 11 February 1983 (1983-02-11), pages 671-686, ISSN: 0305-1048
- DEKHTYAR MICHAEL ET AL: "Triad pattern algorithm for predicting strong promoter candidates in bacterial genomes", BMC BIOINFORMATICS, BIOMED CENTRAL, LONDON, GB, vol. 9, no. 1, 9 May 2008 (2008-05-09), page 233, XP021031810, ISSN: 1471-2105

## Description

### TECHNICAL FIELD

The present invention relates to the use of polymer particles. In particular the present invention relates to the use of functionalised polymer particles.

### BACKGROUND OF THE INVENTION

Many bacterial species produce macromolecules to store excess nutrients intracellularly, which are known to play a role in the storage of carbon, when growth may be impaired or restricted by the lack of other nutrients. These polymer particles are deposited as cytoplasmic inclusions in the cell. The core of these polymer particles typically consists of polyhydroxyalkyl carboxylates, in particular polyhydroxy alkanoates (PHAs). The particles are presumably enclosed by a phospholipid membrane.

The properties of PHAs have been widely investigated for their applications as bioplastics, in addition to their use as a matrix for the transport of drugs and other active agents in medical, pharmaceutical and food industry applications.

A number of proteins are also known to be embedded within the phospholipid membrane surrounding these polymer particles. Immobilising therapeutic proteins within the phospholipid layer has been contemplated. Such functionalised polymer particles have been contemplated as suitable for transporting therapeutic agents, including through the blood brain barrier (WO 04/020623, Rehm).

Polymeric beads such as agarose beads are commonly used for the separation of target components from a mixture. For example, affinity separation techniques typically involve the passing of a mobile phase over or through a solid or gel stationary phase consisting of beads to which an affinity ligands such as a protein or antibody is covalently linked. Separation is based on the ability of the affinity ligand to bind specifically to a target component in the mobile phase, while unbound components are washed out. The target component can later be eluted and the affinity matrix regenerated for further use.

Alternatively, following contact with the mobile phase the beads can be separated using a cell sorter, centrifugation or filtration.

Flow cytometry can be used to separate and simultaneously characterise particles such as cells or synthetic beads that possess a number of pre-selected properties. Measurable properties include size, volume, viscosity, light scatter characteristics, content of DNA or RNA and surface antigens.

The use of different fluorescent markers allows multiparameter analyses of a single particle to be simultaneously conducted and measured in a single, small-volume sample. Examples of such particle-based flow cytometric immunoassays include BD cytometric bead arrays available from BD Biosciences that allow the simultaneous measurement of multiple analytes (http://www.bdbiosciences.com/pharmingen/products/display product. php?keyID=9). These bead arrays consist of different bead populations each labelled with distinct fluorescent intensities and covalently coupled to capture antibodies specific for various analytes. The captured analyte can then be specifically detected by the addition of fluorescent antibodies. Such bead arrays are particularly valuable in the diagnosis of disease, allowing the immunophenotyping of abnormal cells, determination of the ratio of different cell types and discrimination between graft rejection and viral infections, e.g., in transplant patients.

Bead arrays can also be used in ligand screening methods. WO 2004/048922 describes the bead-based detection of G protein coupled receptors compatible with flow cytometry. Synthetic beads were coated with nickel to bind hexahistidine-tagged G proteins, or with streptavidin to bind biotinylated anti-FLAG antibodies followed by FLAG-tagged G proteins. Ternary complexes were assembled on the beads using fluorescent ligand with wild-type receptor or fluorescent receptor fusion polypeptides with unlabelled ligands to determine affinity measurements of the complex.

The generation and screening of combinatorial peptide libraries provides a powerful tool for the study of biological systems, as well as in the identification of candidate molecules for drug therapy. For useful reviews of such methods see Ruiwu Liu et al (2003) and Adda et al., (2002).

Methods for designing peptide libraries, screening target molecules, and isolating compound of interest are well known in the art (Diaz J et al., 2003; Wolkowicz and Nolan, 2003; Doi and Yanagawa 2001)

Phage display technology has been used extensively to display and screen large libraries by exploiting the capability of bacteriophage to express and display peptides on their surface (Rhyner C et al., 2002). The many applications of phage display can be broken down into three general categories depending on the nature of the peptide being displayed. These are display of (1) proteins or protein fragments; (2) antibody fragments; and (3) random peptides.

Phage display of proteins or protein fragments can be used to identify catalytic and non-catalytic proteins or fragments thereof that bind other proteins, nucleic acids (DNA and RNA), carbohydrates, lipids or small chemical compounds (organic or inorganic) including compounds that are agonists, antagonists or substrates of the protein of interest. This type of phage-display has been used to identify enzymes that catalyze particular reactions, to study the interaction between protein domains and DNA and to explore protein-protein interactions, especially interactions with multifunctional proteins, antibodies, receptors and proteins in signalling cascades. Phage display of random peptides can be used in similar ways, particularly to identify novel peptides that bind to target molecules of interest.

Phage display of antibody or antibody fragments (particularly variable region fragments such as Fab and scFv) can be used to identify antibodies that bind to an epitope of interest.

However, immobilising ligands to synthetic beads either directly or indirectly typically presents a number of problems. For instance, the biological activity of the ligand may be impaired during immobilisation. Further, the amount of ligand bound and its biological activity may vary within each bead population, between different bead populations and between one bead array batch to another, presenting problems with standardisation.

Due to the extensive production procedures required, bead-based isolation, detection or screening kits tend to be very expensive.

Ion exchange chromatography matrices have also been investigated for the refolding of recombinant proteins expressed as inclusion bodies in cellular expression systems.

The completion of numerous micro-organism, plant and animal genome sequencing projects has led to the identification of large numbers of potentially useful proteins. Advances in protein expression systems have made the production of recombinant polypeptides possible in a variety of host cells.

In the absence of specific folding or post-translational modification requirements, E. coli-based fermentation systems are often the expression host of choice. While such fermentations produce good yields at a laboratory scale however, the scale-up to an industrial scale is problematic.

Recombinant protein productivity can be proved by i) increasing the amount of recombinant protein per cell; and ii) increasing the amount of cell mass per unit of volume. However, high-level expression of recombinant proteins in *E. coli* often results in the formation of inactive protein, aggregated as inclusion bodies that comprise partially folded intermediates rather than correctly folded native protein.

Inclusion bodies consist mainly of the protein of interest and can be isolated from disrupted host cells by centrifugation. Accordingly, despite being inactive the production of recombinant proteins as inclusion bodies is often exploited as a simple method of purification. Additionally, inclusion bodies often allow the accumulation of recombinant protein in the cytoplasm to a much higher level and provide increased protection against proteolytic degradation compared to soluble forms. Inclusion bodies also have no biological activity, allowing production of proteins which may otherwise be toxic to the *E. coli* host.

Recovery of biologically active products from inclusion bodies typically involves unfolding with chaotropic agents or acids, followed by dilution or dialysis into optimised refolding buffers. However, while such recovery is possible at a laboratory scale, the recovery of biologically active proteins from inclusion bodies is typically too expensive for large-scale production. Furthermore, many oligomeric or structurally complex polypeptides, or those containing cysteine residues, do not easily adopt active confirmations during *in vitro* refolding. Maximising the yields of recombinant proteins in a soluble and active form *in vivo* is the focus of much investigation.

WO2004/020623 relates to a method for producing biodegradable, functionalised polymer particles, and to the use of the same as medicament carriers.

An article by VOLLENKLE C ET AL: entitled "Construction of a functional Slayer fusion protein comprising an immunoglobulin G-binding domain for development of specific adsorbents for extracorporeal blood purification" APPLIED AND ENVIRONMENTAL MICROBIOLOGY, AMERICAN SOCIETY FOR MICROBIOLOGY, US LINKD-DOI: 10.1128/AEM.70.3 1514-1521.2004, vol. 70, no. 3, 1 March 2004 (2004-03.01), pages 1514-1521, discloses a microbead that should find application in a microsphere-based detoxification system.

It is an object of the present invention to provide improved polymer peptides and methods of their use or to at least provide the public with a useful choice.

Further aspects and advantages of the present invention will become apparent from the ensuing description which is given by way of example only.

## SUMMARY OF THE INVENTION

In accordance with the present invention there is provided a method of detecting and optionally isolating at least one target component as set forth in the claims.

One aspect of the present disclosure relates to a process for producing polymer particles, the process comprising:

A) providing a host cell comprising at least one expression construct operably linked to a strong promoter, the expression construct comprising:

(1) at least one nucleic acid sequence encoding a polymer synthase; or

(2) at least one nucleic acid sequence encoding a fusion polypeptide that comprises a polymer synthase and at least one fusion partner;

B) cultivating the host cell under conditions suitable for expression of the expression construct and for formation of polymer particles by the polymer synthase; and

C) separating the polymer particles from the cultivated host cells to produce a composition comprising polymer particles.

Another aspect of the present disclosure relates to a process for producing polymer particles, the process comprising:

A) providing a host cell comprising at least one expression construct operably linked to a strong promoter, the expression construct comprising:

(1) at least one nucleic acid sequence encoding a polymer synthase; or

(2) at least one nucleic acid sequence encoding a fusion polypeptide that comprises a polymer synthase and at least one fusion partner; and

(3) at least one nucleic acid sequence encoding a particle forming protein or at least one nucleic acid sequence encoding an additional fusion polypeptide or a combination thereof, wherein the additional fusion polypeptide comprises

(a) a polymer particle binding domain, a protein that comprises a polymer particle binding domain or a particle forming protein, and at least one fusion partner, or

(b) at least one polypeptide and a binding domain that binds the fusion partner of the fusion polypeptide;

B) cultivating the host cell under conditions suitable for expression of the expression construct and for formation of polymer particles by the polymer synthase; and

C) separating the polymer particles from the cultivated cells to produce a composition comprising polymer particles.

Another aspect of the present disclosure relates to a process for producing polymer particles, the process comprising:

A) providing a host cell comprising at least one expression construct operably linked to a strong promoter, the expression construct comprising:

(1) at least one nucleic acid sequence encoding a polymer synthase; or

(2) at least one nucleic acid sequence encoding a fusion polypeptide that comprises a polymer synthase and at least one fusion partner; and

(3) at least one nucleic acid sequence encoding an additional fusion polypeptide that comprises a polymer particle binding domain, a protein that comprises a polymer particle binding domain or a particle forming protein, and at least one fusion partner; and

(4) at least one nucleic acid sequence encoding a further fusion polypeptide that comprises a polypeptide and a binding domain that binds the fusion partner of the fusion polypeptide or the additional fusion polypeptide;

B) cultivating the host cell under conditions suitable for expression of the expression construct and for formation of polymer particles by the polymer synthase; and

C) separating the polymer particles from the cultivated cells to produce a composition comprising polymer particles.

In one embodiment the host cell further comprises an additional expression construct comprising at least one nucleic acid sequence encoding an additional fusion polypeptide that comprises a polymer particle binding domain, a protein that comprises a polymer particle binding domain, or a particle forming protein and at least one fusion partner.

In one embodiment the host cell further comprises an additional expression construct comprising at least one nucleic acid sequence encoding a particle forming protein.

In one embodiment the host cell further comprises an additional expression construct comprising at least one nucleic acid sequence encoding a further fusion polypeptide that comprises a polypeptide and a binding domain that binds the fusion partner of the fusion polypeptide.

Another aspect of the present disclosure relates to a process for producing polymer particles, the process comprising:

A) providing a cell comprising at least one expression construct operably linked to a strong promoter, the expression construct comprising:

(1) at least one nucleic acid sequence encoding a polymer synthase, the polymer synthase comprising a polymer particle binding domain; or

(2) at least one nucleic acid sequence encoding a fusion polypeptide, the fusion polypeptide comprising a polymer synthase and at least one fusion partner, the polymer synthase comprising a polymer particle binding domain; and

(3) optionally, at least one nucleic acid sequence encoding an additional fusion polypeptide that comprises a polymer particle binding domain, a protein that comprises a polymer particle binding domain or a particle forming protein, and at least one fusion partner;

B) cultivating the cell under conditions suitable for expression of the expression construct and for formation of polymer particles by the polymer synthase, wherein the polymer synthase remains associated with the particle it forms and wherein any fusion polypeptide present binds a polymer particle; and

C) separating the polymer particles from the cultivated cells to produce a composition comprising polymer particles.

In one embodiment the expression construct is in a high copy number vector. A high copy number number vector comprises a related origin of replication

In one embodiment the strong promoter is a viral promoter or a phage promoter.

In one embodiment the promoter is a phage promoter.

In one embodiment the promoter is a T7 phage promoter.

In one embodiment the host cell comprises two or more different expression constructs that each encode a different fusion polypeptide.

In one embodiment the host cell comprises three or more different expression constructs that each encode a different fusion polypeptide.

In one embodiment at least about 1% of the surface area of the polymer particles is covered by surface-bound proteins.

In one embodiment at least about 10% of the surface area of the polymer particles is covered by surface-bound proteins.

In one embodiment at least about 50% of the surface area of the polymer particles is covered by surface-bound proteins.

In one embodiment the process produces polymer particles with an average diameter less than about 200 nm.

In one embodiment the process produces polymer particles with an average diameter less than about 150 nm.

In one embodiment the process produces polymer particles with an average diameter less than about 110 nm.

In one embodiment the process produces at least about 20 particles per host cell.

In one embodiment the process produces at least about 40 particles per host cell.

In one embodiment the process produces at least about 60 particles per host cell.

In one embodiment the process further comprises adding at least one polypeptide or at least one substance or a combination thereof while cultivating the host cells so that the at least one polypeptide or at least one substance binds to or is incorporated into the polymer particles.

In one embodiment the process further comprises contacting the polymer particles with at least one polypeptide or at least one substance or a combination thereof that binds to or is adsorbed into the polymer particles.

In one embodiment the substance is a lipophilic substance that is incorporated into or adsorbed into the polymer particles.

In one embodiment the substance is one or more skin care agents selected from sunscreen agents, particulate materials, conditioning agents, thickening agents, water-soluble vitamins, water-dispersible vitamins, oil-dispersible vitamins, emulsifying elastomers comprising dimethicone copolyol crosspolymers, non-emulsifying elastomers comprising dimethicone/vinyl dimethicone crosspolymers, oil-soluble skin care actives comprising oil-soluble terpene alcohols, phytosterols, anti-acne actives, beta-hydroxy acids, vitamin $B_3$ compounds, retinoids, anti-oxidants/radical scavengers, chelators, flavonoids, antiinflammatory agents, anti-cellulite agents, topical anesthetics, antiperspirants, and fragrances, or a combination thereof.

In one embodiment the substance is one or more cleaning agents selected from

a) enzymes including cellulases, peroxidases, proteases, glucoamylases, amylases, lipases, cutinases, pectinases, reductases, oxidases, phenoloxidases, lipoxygenases, ligninases, pullulanases, tannases, pentosanases, malanases, β-glucanases, and arabinosidases, or

b) anti-redeposition agents including methylcellulose, carboxymethylcellulose, hydroxyethylcellulose, polyacrylate polymers, copolymers of maleic anhydride and acrylic acid, copolymers of maleic anhydride and ethylene, copolymers of maleic anhydride and methylvinyl ether, copolymers of maleic anhydride, and methacrylic acid, or

c) a combination thereof.

In one embodiment the fusion partner is an enzyme selected from the list comprising cellulases, peroxidases, proteases, glucoamylases, amylases, lipases, cutinases, pectinases, reductases, oxidases, phenoloxidases, lipoxygenases, ligninases, pullulanases, tannases, pentosanases, malanases, β-glucanases, arabinosidases, racemases, hydrolases, dehydrogenases, polymerases, dioxygenases, monoxgenases, lyases, synthetases, epimerases, hydroxylases, transferases, transacylases and synthases.

In one embodiment the substance is a coloured or fluorescent molecule, a radioisotope, one or more metal ions or a combination thereof.

In one embodiment the fusion partner is selected from a protein, a protein fragment, a binding domain, a target-binding domain, a binding protein, a binding protein fragment, an antibody, an antibody fragment, an antibody heavy chain, an antibody light chain, a single chain antibody, a single-domain antibody (a VHH for example), a Fab antibody fragment, an Fc antibody fragment, an Fv antibody fragment, a F(ab')2 antibody fragment, a Fab' antibody fragment, a single-chain Fv (scFv) antibody fragment, an antibody binding domain (a ZZ domain for example), an antigen, an antigenic determinant, an epitope, a hapten, an immunogen, an immunogen fragment, biotin, a biotin derivative, an avidin, a streptavidin, a substrate, an enzyme, an abzyme, a co-factor, a receptor, a receptor fragment, a receptor subunit, a receptor subunit fragment, a ligand, an inhibitor, a hormone, a lectin, a polyhistidine, a coupling domain, a DNA binding domain, a FLAG epitope, a cysteine residue, a library peptide, a reporter peptide, and an affinity purification peptide, or a combination thereof.

In one embodiment the fusion partner comprises

a) an antigen, an antigenic determinant, an epitope, or an immunogen, or

b) an antibody or an antibody fragment, or

c) an antibody binding domain.

In one embodiment the fusion partner comprises an antibody binding domain.

In one embodiment the process produces at least about 20 particles per host cell with an average diameter less than about 110 nm.

In one embodiment the process further comprises:

a. binding a coupling reagent to the fusion partner, or

b. binding a coupling reagent to the fusion partner and binding a substance to the coupling reagent, or

c. binding a substance to the fusion partner, or

d. chemically modifying the polymer synthase or the particle forming protein to form at least one binding domain by contacting the polymer synthase or the protein with a coupling reagent,

e. or a combination thereof.

In one embodiment the fusion partner comprises a binding domain, the process further comprising

a. binding a coupling reagent to the binding domain, or

b. binding a coupling reagent to the binding domain and binding a substance to the coupling reagent, or

c. binding a substance to the binding domain,

d. or a combination thereof.

Another aspect of the present disclosure relates to a composition comprising a plurality of polymer particles having an average diameter of less than about 200 nm, the polymer particles comprising

A) at least one polymer synthase, or

B) at least one fusion polypeptide comprising a polymer synthase and at least one fusion partner, and

C) optionally,

(1) at least one additional particle forming protein, or

(2) at least one additional fusion polypeptide comprising a polymer particle binding domain, a protein that comprises a polymer particle binding domain or a particle forming protein, and at least one fusion partner, or

(3) at least one of (1) and at least one of (2).

Another aspect of the present invention relates to a composition comprising a plurality of polymer particles having an average diameter of less than about 150 nm, the polymer particles comprising

A) at least one polymer synthase, or

B) at least one fusion polypeptide comprising a polymer synthase and at least one fusion partner, and

C) optionally,

(1) at least one additional particle forming protein, or

(2) at least one additional fusion polypeptide comprising a polymer particle binding domain, a protein that comprises a polymer particle binding domain or a particle forming protein, and at least one fusion partner, or

(3) at least one of (1) and at least one of (2).

Another aspect of the present disclosure relates to a composition comprising a plurality of polymer particles having an average diameter of less than about 110 nm, the polymer particles comprising

A) at least one polymer synthase, or

B) at least one fusion polypeptide comprising a polymer synthase and at least one fusion partner, and

C) optionally,

(1) at least one additional particle forming protein, or

(2) at least one additional fusion polypeptide comprising a polymer particle binding domain, a protein that comprises a polymer particle binding domain or a particle forming protein, and at least one fusion partner, or

(3) at least one of (1) and at least one of (2).

In one embodiment at least about 1% of the surface area of the polymer particles is covered by surface-bound proteins.

In one embodiment at least about 10% of the surface area of the polymer particles is covered by surface-bound proteins.

In one embodiment at least about 50% of the surface area of the polymer particles is covered by surface-bound proteins.

In one embodiment the composition further comprises at least one substance bound to or incorporated into the polymer particles or a combination thereof.

In one embodiment the substance is a lipophilic substance.

In one embodiment the substance is one or more skin care agents selected from sunscreen agents, particulate materials, conditioning agents, thickening agents, water-soluble vitamins, water-dispersible vitamins, oil-dispersible vitamins, emulsifying elastomers comprising dimethicone copolyol crosspolymers, non-emulsifying elastomers comprising dimethicone/vinyl dimethicone crosspolymers, oil-soluble skin care actives comprising oil-soluble terpene alcohols, phytosterols, anti-acne actives, beta-hydroxy acids, vitamin $B_3$ compounds, retinoids, anti-oxidants/radical scavengers, chelators, flavonoids, antiinflammatory agents, anti-cellulite agents, topical anesthetics, antiperspirants, and fragrances, or a combination thereof.

In one embodiment the substance is one or more cleaning agents selected from

a) enzymes including cellulases, peroxidases, proteases, glucoamylases, amylases, lipases, cutinases, pectinases, reductases, oxidases, phenoloxidases, lipoxygenases, ligninases, pullulanases, tannases, pentosanases, malanases, β-glucanases, and arabinosidases, or

b) anti-redeposition agents including methylcellulose, carboxymethylcellulose, hydroxyethylcellulose, polyacrylate polymers, copolymers of maleic anhydride and acrylic acid, copolymers of maleic anhydride and ethylene, copolymers of maleic anhydride and methylvinyl ether, copolymers of maleic anhydride, and methacrylic acid, or

c) a combination thereof.

In one embodiment the fusion partner is an enzyme selected from the list comprising cellulases, peroxidases, proteases, glucoamylases, amylases, lipases, cutinases, pectinases, reductases, oxidases, phenoloxidases, lipoxygenases, ligninases, pullulanases, tannases, pentosanases, malanases, β-glucanases, arabinosidases, racemases, hydrolases, dehydrogenases, polymerases, dioxygenases, monoxgenases, lyases, synthetases, epimerases, hydroxylases, transferases, transacylases and synthases.

In one embodiment the substance is a coloured or fluorescent molecule, a radioisotope, one or more metal ions, or a combination thereof.

In one embodiment the fusion partner is selected from a protein, a protein fragment, a binding domain, a target-binding domain, a binding protein, a binding protein fragment, an antibody, an antibody fragment, an antibody heavy chain, an antibody light chain, a single chain antibody, a single-domain antibody (a VHH for example), a Fab antibody fragment, an Fc antibody fragment, an Fv antibody fragment, a F(ab')2 antibody fragment, a Fab' antibody fragment, a single-chain Fv (scFv) antibody fragment, an antibody binding domain (a ZZ domain for example), an antigen, an antigenic determinant, an epitope, a hapten, an immunogen, an immunogen fragment, biotin, a biotin derivative, an avidin, a streptavidin, a substrate, an enzyme, an abzyme, a co-factor, a receptor, a receptor fragment, a receptor subunit, a receptor subunit fragment, a ligand, an inhibitor, a hormone, a lectin, a polyhistidine, a coupling domain, a DNA binding domain, a FLAG epitope, a cysteine residue, a library peptide, a reporter peptide, and an affinity purification peptide, or a combination thereof.

In one embodiment the fusion partner is

a) an antigen, an antigenic determinant, an epitope, or an immunogen, or

b) an antibody or antibody fragment, or

c) an antibody binding domain.

In one embodiment the fusion partner comprises an antibody binding domain.

In one embodiment:

a. a coupling reagent is bound to the fusion partner, or

b. a coupling reagent is bound to the fusion partner and a substance is bound to the coupling reagent, or

c. a substance is bound to the fusion partner, or

d. the polymer synthase or the particle forming protein has been chemically modified with a coupling reagent to form at least one binding domain, or

e. a combination thereof.

In one embodiment the fusion partner comprises a binding domain and

a. a coupling reagent is bound to the binding domain, or

b. a coupling reagent is bound to the binding domain and a substance is bound to the coupling reagent, or

c. a substance is bound to the binding domain,

d. or a combination thereof.

Another aspect of the present disclosure relates to a composition of polymer particles produced according to a process defined above.

Another aspect of the present disclosure relates to a polymer particle comprising:

A) at least one polypeptide comprising a polymer synthase; or

B) at least one fusion polypeptide comprising a polymer synthase and at least one fusion partner; and

C) optionally,

(1) at least one polypeptide comprising a particle forming protein, or

(2) at least one additional fusion polypeptide comprising a polymer particle binding domain, a protein that comprises a polymer particle binding domain, or a particle forming protein, and at least one fusion partner, or

(3) a combination thereof;

wherein at least about 1% of the surface area of the polymer particle is covered by polypeptide.

Another aspect of the present disclosure relates to a polymer particle comprising:

A) at least one polypeptide comprising a polymer synthase; or

B) at least one fusion polypeptide comprising a polymer synthase and at least one fusion partner; and

C) optionally,

(1) at least one polypeptide comprising a particle forming protein, or

(2) at least one additional fusion polypeptide comprising a polymer particle binding domain, a protein that comprises a polymer particle binding domain, or a particle forming protein, and at least one fusion partner, or

(3) a combination thereof;

wherein at least about 10% of the surface area of the polymer particle is covered by polypeptide.

Another aspect of the present disclosure relates to a polymer particle comprising:

A) at least one polypeptide comprising a polymer synthase; or

B) at least one fusion polypeptide comprising a polymer synthase and at least one fusion partner; and

C) optionally,

(1) at least one polypeptide comprising a particle forming protein, or

(2) at least one additional fusion polypeptide comprising a polymer particle binding domain, a protein that comprises a polymer particle binding domain, or a particle forming protein, and at least one fusion partner, or

(3) a combination thereof;

wherein at least about 50% of the surface area of the polymer particle is covered by polypeptide.

In one embodiment the polymer particle further comprises at least one substance bound to or incorporated into the polymer particle, or a combination thereof.

In one embodiment the substance is a protein or protein fragment, a peptide, a polypeptide, an antibody or antibody fragment, an antibody binding domain, an antigen, an antigenic determinant, an epitope, an immunogen or fragment thereof, a metal ion, a metal ion-coated molecule, biotin, avidin, streptavidin or derivatives thereof, an inhibitor, a co-factor, a substrate, an enzyme, a co-factor, a receptor, receptor subunit or fragment thereof, a ligand, an inhibitor, a monosaccharide, an oligosaccharide, a polysaccharide, a glycoprotein, a lipid, a cell or fragment thereof, a cell extract, a virus, a hormone, a serum protein, a milk protein, a macromolecule, a drug of abuse, or a combination thereof.

In one embodiment the substance is one or more skin care agents selected from sunscreen agents, particulate materials, conditioning agents, thickening agents, water-soluble vitamins, water-dispersible vitamins, oil-dispersible vitamins, emulsifying elastomers comprising dimethicone copolyol crosspolymers, non-emulsifying elastomers comprising dimethicone/vinyl dimethicone crosspolymers, oil-soluble skin care actives comprising oil-soluble terpene alcohols, phytosterols, anti-acne actives, beta-hydroxy acids, vitamin $B_3$ compounds, retinoids, anti-oxidants/radical scavengers, chelators, flavonoids, antiinflammatory agents, anti-cellulite agents, topical anesthetics, antiperspirants and fragrances, or a combination thereof.

In one embodiment the substance is one or more cleaning agents selected from

a) enzymes including cellulases, peroxidases, proteases, glucoamylases, amylases, lipases, cutinases, pectinases, reductases, oxidases, phenoloxidases, lipoxygenases, ligninases, pullulanases, tannases, pentosanases, malanases, β-glucanases, and arabinosidases, or

b) anti-redeposition agents including methylcellulose, carboxymethylcellulose, hydroxyethylcellulose, polyacrylate polymers, copolymers of maleic anhydride and acrylic acid, copolymers of maleic anhydride and ethylene, copolymers of maleic anhydride and methylvinyl ether, copolymers of maleic anhydride, and methacrylic acid, or

c) a combination thereof.

In one embodiment the fusion partner is an enzyme selected from the list comprising cellulases, peroxidases, proteases, glucoamylases, amylases, lipases, cutinases, pectinases, reductases, oxidases, phenoloxidases, lipoxygenases, ligninases, pullulanases, tannases, pentosanases, malanases, β-glucanases, arabinosidases, racemases, hydrolases, dehydrogenases, polymerases, dioxygenases, monoxgenases, lyases, synthetases, epimerases, hydroxylases, transferases, transacylases and synthases.

In one embodiment the substance is a coloured or fluorescent molecule, a radioisotope, one or more metal ions, or a combination thereof.

In one embodiment wherein the fusion partner is selected from a protein, a protein fragment, a binding domain, a target-binding domain, a binding protein, a binding protein fragment, an antibody, an antibody fragment, an antibody heavy chain, an antibody light chain, a single chain antibody, a single-domain antibody (a VHH for example), a Fab antibody fragment, an Fc antibody fragment, an Fv antibody fragment, a F(ab')2 antibody fragment, a Fab' antibody fragment, a single-chain Fv (scFv) antibody fragment, an antibody binding domain (a ZZ domain for example), an antigen, an antigenic determinant, an epitope, a hapten, an immunogen, an immunogen fragment, biotin, a biotin derivative, an avidin, a streptavidin, a substrate, an enzyme, an abzyme, a co-factor, a receptor, a receptor fragment, a receptor subunit, a receptor subunit fragment, a ligand, an inhibitor, a hormone, a lectin, a polyhistidine, a coupling domain, a DNA binding domain, a FLAG epitope, a cysteine residue, a library peptide, a reporter peptide, and an affinity purification peptide, or a combination thereof.

In one embodiment the fusion partner is:

a) an antigen, an antigenic determinant, an epitope, or an immunogen, or

b) an antibody or antibody fragment, or

c) an antibody binding domain.

In one embodiment the fusion partner comprises an antibody binding domain.

In one embodiment:

a. a coupling reagent is bound to the fusion partner, or

b. a coupling reagent is bound to the fusion partner and a substance is bound to the coupling reagent, or

c. a substance is bound to the fusion partner, or

d. the polymer synthase or the particle forming protein has been chemically modified with a coupling reagent to form at least one binding domain, or

e. a combination thereof.

In one embodiment the fusion partner comprises a binding domain and

a) a coupling reagent is bound to the binding domain, or

b) a coupling reagent is bound to the binding domain and a substance is bound to the coupling reagent, or

c) a substance is bound to the binding domain,

d) or a combination thereof.

Another aspect of the present disclosure relates to a polymer particle produced according to a process defined above.

Another aspect of the present disclosure relates to a composition comprising a polymer particle as defined above that is produced according to a process as defined above.

Another aspect of the present disclosure relates to a diagnostic reagent comprising a composition of polymer particles as defined above or comprising one or more polymer particles as defined above.

Another aspect of the present disclosure relates to a diagnostic kit comprising a composition of polymer particles as defined above or comprising one or more polymer particles as defined above.

In one embodiment the polymer particles in the diagnostic kit are immobilised on a substrate comprising an ELISA plate, microarray slide or a chromatography matrix, or a combination thereof.

Another aspect of the present invention relates to a method of detecting and optionally isolating at least one target component in a sample, the method comprising:

(a) providing at least one polymer particle comprising at least one fusion polypeptide covalently or non-covalently bound to the polymer particle, the fusion polypeptide comprising

(i) a polymer particle binding domain, a polypeptide that comprises a polymer particle binding domain or a particle forming protein that comprises a polymer particle binding domain, and
(ii) at least one binding domain that will bind a target component,

(b) contacting the polymer particle with a sample comprising a target component such that the binding domain binds the target component to form a complex,

(c) detecting the complex, and

(d) optionally separating the complex from the sample.

In one embodiment detecting the complex comprises contacting the polymer particle with a labelled molecule that will bind to the complex, to the at least one fusion polypeptide or to the polymer particle, and detecting the labelled molecule.

In one embodiment the labelled molecule is a labelled antibody.

In one embodiment the polymer particle comprises

a) a label bound to or incorporated into the polymer particle, or

b) at least one additional fusion polypeptide comprising

(i) a polymer a polymer particle binding domain, a polypeptide that comprises a polymer particle binding domain or a particle forming protein that comprises a polymer particle binding domain, and

(ii) a binding domain that will bind a labelled molecule, or

c) at least one additional fusion polypeptide comprising a reporter protein.

In one embodiment the polymer particle comprises two or more different fusion polypeptides.

In one embodiment the polymer particle comprises two or more different fusion polypeptides on the polymer particle surface.

In one embodiment the polymer particle comprises three or more different fusion polypeptides on the polymer particle surface.

In one embodiment the polymer particle further comprises at least one substance bound to or incorporated into the polymer particle, or a combination thereof.

In one embodiment the substance is a coloured fluorescent molecule, a radioisotope, or one or more metal ions, or a combination thereof.

In one embodiment the binding domain is

a) an antigen, an antigenic determinant, an epitope or an immunogen, or

b) an antibody or antibody fragment, or

c) an antibody binding domain.

In one embodiment the binding domain is Protein A or a ZZ domain.

In one embodiment the target component is selected from a protein, a protein fragment, a peptide, a polypeptide, a polypeptide fragment, an antibody, an antibody fragment, an antibody binding domain, an antigen, an antigen fragment, an antigenic determinant, an epitope, a hapten, an immunogen, an immunogen fragment, a metal ion, a metal ion-coated molecule, biotin, a biotin derivative, avidin, streptavidin, an inhibitor, a co-factor, a substrate, an enzyme, an abzyme, a receptor, a receptor fragment, a receptor subunit, a receptor subunit fragment, a ligand, a receptor ligand, a receptor agonist, a receptor antagonist, a signalling molecule, a signalling protein, a signalling protein fragment, a growth factor, a growth factor fragment, a transcription factor, a transcription factor firagment, an inhibitor, a cytokine, a chemokine, an inflammatory mediator, a monosaccharide, an oligosaccharide, a polysaccharide, a glycoprotein, a lipid, a cell, a cell-surface protein, a cell-surface lipid, a cell-surface carbohydrate, a cell-surface glycoprotein, a cell extract, a virus, a virus coat protein, a hormone, a serum protein, a milk protein, a macromolecule, a drug of abuse, a coupling reagent, a polyhistidine, a pharmaceutically active agent, a biologically active agent, a label, a coupling reagent, a library peptide, an expression construct, a nucleic acid or a combination thereof.

In one embodiment the target component is selected from Interleukin-3 (IL-3), Interleukin-4 (IL-4), Interleukin-5 (IL-5), Interleukin-10 (IL-10), Interleukin-7 (IL-7), Interleukin-1β (IL-1β), Interleukin-6 (IL-6), Interleukin-12p70 (IL-12p70), Granulocyte Macrophage-Colony Stimulating Factor (GM-CSF), cleaved PARP, Bcl-2, and active Caspase-3 protein levels, Interleukin-8 (IL-8), basic Fibroblast Growth Factor (bFGF), Angiogenin (ANG), Vascular Endothelial Growth Factor (VEGF), and Tumor Necrosis Factor (TNF), Interleukin-8 (CXCL8/IL-8), RANTES (CCL5/RANTES), Monokine-induced by Interferon-γ (CXCL9/MIG), Monocyte Chemoattractant Protein-1 (CCL2/MCP-1), or Interferon-γ-induced Protein-10 (CXCL10/IP-10), or a mixture thereof.

In one embodiment the at least one polymer particle is immobilised on a substrate comprising an ELISA plate, microarray slide or a chromatography matrix.

In one embodiment the at least one polymer particle comprises

a. a polymer particle produced according to a method of the invention,

b. a composition of the invention, or

c. a polymer particle of the invention.

Another aspect of the present disclosure relates to a method of producing recombinant polypeptides, the method comprising culturing a particle-producing host cell that comprises an expression construct comprising a nucleic acid sequence encoding a fusion polypeptide, the fusion polypeptide comprising

(a) a polymer particle binding domain, a polypeptide that comprises a polymer particle binding domain or a particle forming protein that comprises a polymer particle binding domain, and

(b) a polypeptide that forms inclusion bodies when expressed in a cellular expression system,

wherein the culture conditions are suitable for expression of the fusion polypeptide from the expression construct and for formation of polymer particles.

Another aspect of the present disclosure relates to a method of producing recombinant polypeptides comprising

(a) providing at least one fusion polypeptide comprising

(i) a polymer particle binding domain, a polypeptide that comprises a polymer particle binding domain or a particle forming protein that comprises a polymer particle binding domain, and

(ii) a polypeptide that forms inclusion bodies when expressed in a cellular expression system, and

(b) contacting the fusion polypeptide with a particle-forming reaction mixture to form at least one polymer particle.

In one embodiment the particle forming protein comprises a polymer synthase.

In one embodiment the particle-forming reaction mixture comprises a polymer synthase.

In one embodiment the fusion partner is a polypeptide that forms inclusion bodies when expressed in a cellular expression system.

In one embodiment the host cell comprises two or more different expression constructs that each encode a different fusion polypeptide.

In one embodiment the method comprises the initial step of selecting a polypeptide that forms inclusion bodies when expressed in a cellular expression system by:

(a) conducting a literature search to identify a polypeptide that has previously been determined to form inclusion bodies when expressed in a cellular expression system; or
(b) expressing a candidate polypeptide in a cell that can not form polymer particles and examining the cell microscopically to determine whether or not the expressed polypeptide forms inclusion bodies.

In one embodiment the method further comprises

(1) separating the polymer particles from the cells or the reaction mixture to produce a composition comprising polymer particles, and

(2) optionally separating the fusion polypeptide from the polymer particles, and

(2) optionally separating the polypeptide from the fusion polypeptide.

In one embodiment the recombinant polypeptide does not require refolding.

Another aspect of the present invention relates to a method of identifying a target molecule that binds a receptor polypeptide, the method comprising:

a) providing at least one polymer particle comprising at least one fusion polypeptide, the fusion polypeptide comprising

(i) a polymer particle binding domain, a polypeptide that comprises a polymer particle binding domain or a particle forming protein that comprises a polymer particle binding domain, and

(ii) at least one receptor polypeptide,

b) contacting the at least one polymer particle with at least one target molecule, and

c) identifying a target molecule that binds the receptor polypeptide.

Another aspect of the present disclosure relates to a method of identifying a target molecule that binds a receptor ligand, the method comprising:

(a) providing at least one polymer particle comprising at least one fusion polypeptide the fusion polypeptide comprising

(i) a polymer particle binding domain, a polypeptide that comprises a polymer particle binding domain or a particle forming protein that comprises a polymer particle binding domain, and

(ii) at least one receptor ligand,

(a) contacting the at least one polymer particle with at least one target molecule, and

(b) identifying a target molecule that binds the receptor ligand.

Another aspect of the present disclosure relates to a method of producing a mixed population of polymer particles comprising:

a) providing a particle-producing host cell containing a mixed population of expression constructs wherein each expression construct comprises a nucleic acid sequence encoding a fusion polypeptide, the fusion polypeptide comprising

(i) a polymer particle binding domain, a polypeptide that comprises a polymer particle binding domain or a particle forming protein that comprises a polymer particle binding domain, and

(ii) at least one polypeptide of interest;

b) inducing the host cell to produce polymer particles and express the expression constructs to produce a mixed population of fusion polypeptides that bind the polymer particles; and

c) optionally separating the mixed population of polymer particles from the host cell.

Another aspect of the present disclosure relates to a method of identifying a target molecule that binds a library polypeptide comprising:

(a) providing a mixed population of polymer particles comprising a mixed population of fusion polypeptides, the fusion polypeptides comprising

(i) a polymer particle binding domain, a polypeptide that comprises a polymer particle binding domain or a particle forming protein that comprises a polymer particle binding domain, and

(ii) at least one library polypeptide;

(b) contacting the polymer particles with at least one target molecule;

(c) identifying a target molecule that binds a library polypeptide.

In one embodiment identifying the target molecule comprises contacting the polymer particle with a labelled molecule that will bind to the target molecule, to the at least one fusion polypeptide or to the polymer particle, and detecting the labelled molecule.

In one embodiment the polymer particle is immobilised on a substrate comprising an ELISA plate, microarray slide or a chromatography matrix.

In one embodiment the at least one polypeptide of interest, the at least one library polypeptide or the at least one receptor ligand is selected from a protein, a protein fragment, a binding domain, a target-binding domain, a binding protein, a binding protein fragment, an antibody, an antibody fragment, an antibody heavy chain, an antibody light chain, a single chain antibody, a single-domain antibody, a Fab antibody fragment, an Fc antibody fragment, an Fv antibody fragment, a F(ab')2 antibody fragment, a Fab' antibody fragment, a single-chain Fv (scFv) antibody fragment, an antibody binding domain, an antigen, an antigenic determinant, an epitope, a hapten, an immunogen, an immunogen fragment, biotin, a biotin derivative, an avidin, a streptavidin, a substrate, an enzyme, an abzyme, a co-factor, a receptor, a receptor fragment, a receptor subunit, a receptor subunit fragment, an inhibitor, a coupling domain, or a combination thereof.

Another aspect of the present disclosure relates to a method of identifying a target molecule that binds a fusion polypeptide comprising:

(a) providing a composition of the invention wherein the fusion partner comprises at least one receptor polypeptide, at least one receptor ligand, at least one polypeptide of interest, at least one library polypeptide, or a combination thereof,

(b) contacting the composition with at least one target molecule, and

(c) identifying a target molecule that binds the fusion partner.

In one embodiment identifying the target molecule comprises contacting the polymer particle with a labelled molecule that will bind to the target molecule, to the at least one fusion polypeptide or to the polymer particle, and detecting the labelled molecule.

In one embodiment the polymer particle is immobilised on a substrate comprising an ELISA plate, microarray slide or a chromatography matrix.

Another aspect of the present disclosure relates to a diagnostic reagent comprising a polymers particle as defined above.

Another aspect of the present disclosure relates to diagnostic kit comprising a polymer particle as defined above.

Another aspect of the present disclosure relates to a method as defined above or a composition as defined above wherein:

(a) 80 % of the particles in the composition have a diameter of between about 10 nm to about 150 nm;

(b) 60 % of the particles in the composition have a diameter of between about 10 nm to about 100 nm;

(c) 45 % of the particles in the composition have a diameter of between about 10 nm to about 80 nm;

(d) 40 % of the particles in the composition have a diameter of between about 10 nm to about 60 nm;

(e) 25 % of the particles in the composition have a diameter of between about 10 nm to about 50 nm; or

(f) 5 % of the particles in the composition have a diameter of between about 10 nm to about 35 nm.

Another aspect of the present disclosure relates to an expression construct operably linked to a strong promoter, the expression construct comprising:

(1) at least one nucleic acid sequence encoding a polymer synthase; or

(2) at least one nucleic acid sequence encoding a fusion polypeptide that comprises a polymer synthase and at least one fusion partner.

Another aspect of the present disclosure relates to an expression construct operably linked to a strong promoter, the expression construct comprising:

(1) at least one nucleic acid sequence encoding a polymer synthase; or

(2) at least one nucleic acid sequence encoding a fusion polypeptide that comprises a polymer synthase and at least one fusion partner; and

(3) at least one nucleic acid sequence encoding a particle forming protein or at least one nucleic acid sequence encoding an additional fusion polypeptide or a combination thereof, wherein the additional fusion polypeptide comprises

(a) a polymer particle binding domain, a polypeptide that comprises a polymer particle binding domain or a particle forming protein that comprises a polymer particle binding domain, and

(b) at least one fusion partner.

Another aspect of the present disclosure relates to an expression construct operably linked to a strong promoter, the expression construct comprising:

(1) at least one nucleic acid sequence encoding a fusion polypeptide that comprises a polymer synthase and at least one fusion partner; and

(2) at least one nucleic acid sequence encoding an additional fusion polypeptide that comprises at least one polypeptide and a binding domain that binds the fusion partner of the fusion polypeptide.

Another aspect of the present disclosure relates to a vector comprising an expression construct of the invention.

In one embodiment the expression construct is on a high compy number vector.

Another aspect of the present disclosure relates to a host cell comprising an expression construct or a vector as defined above.

The following embodiments may relate to any of the above aspects.

In one embodiment a fusion polypeptide may comprise at least one polypeptide and a binding domain that binds the fusion partner of the fusion polypeptide or the additional fusion polypeptide. Such fusion polypeptides allow the polypeptide to be separated with the polymer particles from host cells.

In one embodiment the polymer core comprises a polymer selected from poly-beta-amino acids, polylactates, polythioesters and polyesters. Most preferably the polymer comprises polyhydroxyalkanoate (PHA), preferably poly(3-hydroxybutyrate) (PHB).

In one embodiment the polymer particle comprises a polymer core encapsulated by a phospholipid monolayer.

In one embodiment the particle forming protein is bound to the polymer core or to the phospholipid monolayer or is bound to both.

In one embodiment the particle forming protein is covalently or non-covalently bound to the polymer particle it forms.

In one embodiment the particle forming protein is selected from the group of proteins which comprises a polymer depolymerase, a polymer regulator, a polymer synthase and a particle size-determining protein. These proteins preferably originate from microorganisms that are capable of forming polymer particles, in particular those from the genera *Ralstonia, Alcaligenes* and *Pseudomonas,* more preferably selected from the group comprising *Ralstonia eutropha, Alcaligenes latus, Pseudomonas putida, Pseudomonas oleovorans, Pseudomonas aeruginosa,* and *Halobiforma haloterrestris.*

In one embodiment the polymer synthase is a PHA polymer synthase from *Ralstonia eutropha, Pseudomonas oleovorans, Pseudomonas putida, Pseudomonas aeruginosa, Aeromonas punctata, Thiocapsa pfennigii* or *Haloarcula marismortui.*

The nucleotide sequences of 59 PHA synthase genes from 45 different bacteria have been obtained, differing in primary structure, substrate specificity and subunit composition. Polymer synthases for use in the present invention are described in detail in Rehm B. H. A., Biochem J., (2003), 376(1):15-33, which is herein incorporated by reference. For example, the polymer synthase may comprise a PHA polymer synthase from C. necator, P. aeruginosa, A. vinosum, B. megaterium, *H. marismortui, P. aureofaciens,* or *P. putida,* which have Accession No.s AY836680, AE004091, AB205104, AF109909, YP137339, AB049413 and AF150670, respectively.

In one embodiment the particle forming protein is a phasin. Most preferably the phasin is selected from the group comprising a phasin from *R. eutropha* and *P. oleovorans,* preferably the phasin phaP from *R. eutropha* and the phasin phaF from *P. oleovorans.*

In one embodiment the particle forming protein can be used for the *in vitro* production of polymer particles by polymerising or facilitating the polymerisation of the substrates (R)-Hydroxyacyl-CoA or other CoA thioester or derivatives thereof.

In one embodiment the substrate or the substrate mixture comprises at least one optionally substituted amino acid, lactate, ester or saturated or unsaturated fatty acid, preferably acetyl-CoA.

In one embodiment the nucleic acid sequence that codes for a fusion polypeptide comprises:

In one embodiment the nucleic acid sequence that codes for a fusion polypeptide comprises:

(1) a nucleic acid sequence that codes for a fusion partner contiguous with the 5' or 3' end of the nucleic acid sequence that codes for a polymer synthase, a polymer particle binding domain, a protein comprising a polymer particle binding domain, or a particle forming protein, or

(2) a nucleic acid sequence that codes for a fusion partner indirectly fused with the 5' or 3' end of the nucleic acid sequence that codes for a polymer synthase, a polymer particle binding domain, a protein comprising a polymer particle binding domain, or a particle forming protein through a polynucleotide linker or spacer sequence of a desired length, or

(3) a nucleic acid sequence that codes for a fusion partner that is inserted into the nucleic acid sequence that codes for a polymer synthase, a polymer particle binding domain, a protein comprising a polymer particle binding domain, or a particle forming protein through a polynucleotide linker or spacer sequence of a desired length

(4) a nucleic acid sequence that codes for a protease cleavage site spaced between the nucleic acid sequence that codes for a fusion partner and the nucleic acid sequence that codes for a polymer synthase, a polymer particle binding domain, a protein comprising a polymer particle binding domain, or a particle forming protein; or

(5) a nucleic acid sequence that codes for a self-splicing element spaced between the nucleic acid sequence that codes for a fusion partner and the nucleic acid sequence that codes for a polymer synthase, a polymer particle binding domain, a protein comprising a polymer particle binding domain, or a particle forming protein; or

(6) any combination of two or more thereof.

In one embodiment the at least one fusion polypeptide comprises:

(1) an amino acid sequence that codes for a fusion partner contiguous with the N- or C- terminal end of the amino acid sequence that codes for a polymer synthase, a polymer particle binding domain, a protein comprising a polymer particle binding domain, or a particle forming protein, or

(2) an amino acid sequence that codes for a fusion partner indirectly fused with the N- or C- terminal of the amino acid sequence that codes for a polymer synthase, a polymer particle binding domain, a protein comprising a polymer particle binding domain, or a particle forming protein through a peptide linker or spacer sequence of a desired length, or

(3) an amino acid sequence sequence that codes for a fusion partner that is inserted into the amino acid sequence that codes for a polymer synthase, a polymer particle binding domain, a protein comprising a polymer particle binding domain, or a particle forming protein through a peptide linker or spacer sequence of a desired length, or

(4) an amino acid sequence that codes for a protease cleavage site spaced between the amino acid sequence that codes for a fusion partner and the amino acid sequence that codes for a polymer synthase, a polymer particle binding domain, a protein comprising a polymer particle binding domain, or a particle forming protein; or

(5) an amino acid sequence that codes for a self-splicing element spaced between the amino acid sequence that codes for a fusion partner and the amino acid sequence that codes for a polymer synthase, a polymer particle binding domain, a protein comprising a polymer particle binding domain, or a particle forming protein; or

(6) any combination of two or more thereof.

In one embodiment the polymer particle binding domain is a polymer particle binding domain of a polymer synthase.

In one embodiment the polymer synthase is a PHA polymer synthase from *Ralstonia eutropha, Pseudomonas oleovorans, Pseudomonas putida, Pseudomonas aeruginosa, Aeromonas punctata* or *Thiocapsa pfennigii.*

In one embodiment the expression construct comprises a constitutive or regulatable promoter system.

In one embodiment the regulatable promoter system is an inducible or repressible promoter system.

In one embodiment the regulatable promoter system is selected from LacI, Trp, phage $\gamma$ and phage RNA polymerase.

In one embodiment the promoter is any strong promoter known to those skilled in the art. Suitable strong promoters comprise adenoviral promoters, such as the adenoviral major late promoter; or heterologous promoters, such as the cytomegalovirus (CMV) promoter; the respiratory syncytial virus (RSV) promoter; the simian virus 40 (SV40) promoter; inducible promoters, such as the MMT promoter, the metallothionein promoter; heat shock promoters; the albumin promoter; the ApoAI promoter; human globin promoters; viral thymidine kinase promoters, such as the Herpes Simplex thymidine kinase promoter; retroviral LTRs; the b-actin promoter; human growth hormone promoters; phage promoters such as the T7, SP6 and T3 RNA polymerase promoters and the cauliflower mosaic 35S (CaMV 35S) promoter.

In one embodiment the promoter is a T7 RNA polymerase promoter.

In one embodiment the promoter is a promoter having the sequence as shown in SEQ ID NO: 24.

In one embodiment the cell comprises two or more different expression constructs that each encode a different fusion polypeptide.

In one embodiment a substrate is added to the cell culture or *in vitro* solution in such a quantity that it is sufficient to ensure control of the size of the polymer particles.

In one embodiment the fusion partner is selected from the list comprising a protein, a protein fragment, a binding domain, a target-binding domain, a binding protein, a binding protein fragment, an antibody, an antibody fragment, an antibody heavy chain, an antibody light chain, a single chain antibody, a single-domain antibody (a VHH for example), a Fab antibody fragment, an Fc antibody fragment, an Fv antibody fragment, a F(ab')2 antibody fragment, a Fab' antibody fragment, a single-chain Fv (scFv) antibody fragment, an antibody binding domain (a ZZ domain for example), an antigen, an antigenic determinant, an epitope, a hapten, an immunogen, an immunogen fragment, biotin, a biotin derivative, an avidin, a streptavidin, a substrate, an enzyme, an abzyme, a co-factor, a receptor, a receptor fragment, a receptor subunit, a receptor subunit fragment, a ligand, an inhibitor, a hormone, a lectin, a polyhistidine, a coupling domain, a DNA binding domain, a FLAG epitope, a cysteine residue, a library peptide, a reporter peptide, an affinity purification peptide, or any combination of any two or more thereof.

In one embodiment the binding domain encodes Myelin Oligodendrocyte Glycoprotein (MOG) or fragments thereof. A binding domain encoding MOG allows the detection of antibodies raised against MOG in samples of antisera, for example.

In one embodiment the target component is an anti-Myelin Oligodendrocyte Glycoprotein (MOG) antibody or fragments thereof.

In one embodiment the binding domain encodes an antibody or fragment thereof that will bind a target component related to Type 1 and Type 2 immune responses, apoptosis, and/or angiogenesis.

In one embodiment the coupling domain is selected from the group comprising oligopeptides, enzymes, abzymes and non-catalytic proteins. This group particularly preferably comprises FLAG epitopes or at least one cysteine to which the peptide of interest can bind.

The coupling domain may also be obtained after production of the polymer particles by chemically modifying the at least one binding domain located on the surface with coupling reagents.

In one embodiment the label is a detectable label such as a coloured dye, a fluorescent molecule such as a fluorophore or fluorochrome, a radioisotope; or one or more metal ions that is bound to or absorbed into or incorporated within the polymer particle.

In one embodiment the recombinant polypeptide is a difficult folder polypeptide.

In one embodiment the difficult folder polypeptide is a polypeptide that forms inclusion bodies when expressed in a cellular expression system.

In one embodiment the difficult folder polypeptide is selected by conducting a literature search to identify a polypeptide that has previously been determined to form inclusion bodies when expressed in a cellular expression system.

In one embodiment the difficult folder polypeptide is selected by expressing a candidate polypeptide in a host cell that can not form polymer particles and examining the cell microscopically to determine whether or not the expressed polypeptide forms inclusion bodies.

Preferably the particle forming protein remains associated with the particle it forms to assist the correct folding of the difficult folder polypeptide on the surface of the polymer particle.

In one embodiment at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95%, 99% or 100% of the surface area of the polymer particles are covered by surface-bound proteins.

In one embodiment 90% of the particles in the composition have a diameter of between about 10 nm to about 200 nm. Preferably:

(1) 80 % of the particles in the composition have a diameter of between about 10 nm to about 150 nm;

(2) 60 % of the particles in the composition have a diameter of between about 10 nm to about 100 nm;

(3) 45 % of the particles in the composition have a diameter of between about 10 nm to about 80 nm;

(4) 40 % of the particles in the composition have a diameter of between about 10 nm to about 60 nm;

(5) 25 % of the particles in the composition have a diameter of between about 10 nm to about 50 nm; or

(6) 5 % of the particles in the composition have a diameter of between about 10 nm to about 35 nm.

In one embodiment the process further comprises:

A) adding to the cell culture or solution at least one substance that binds to or is incorporated into the polymer particles, or

B) contacting the polymer particles with adding at least one substance that binds to or is incorporated into the polymer particles, or

C) a combination thereof.

In one embodiment the polymer particles comprise at least one substance bound to or incorporated into the polymer particle.

In one embodiment the process further comprises:

A) binding a coupling reagent to the fusion partner binding domain.

In another embodiment the process further comprises:

A) binding a coupling reagent to the fusion partner binding domain and

B) binding at least one substance to the coupling reagent

In one embodiment the substance that binds to or is incorporated into the polymer particles comprises:

(1) an antigen, an antigenic determinant, an epitope, or an immunogen of fragment thereof, or

(2) an antibody or antibody fragment, or

(3) an antibody binding domain.

Another aspect of the disclosure relates to a diagnostic reagent comprising of polymer particles of the disclosure, and diagnostic kits comprising such reagents.

In one embodiment the polymer particles are immobilised on a substrate comprising an ELISA plate, microarray slide or a chromatography matrix.

In one embodiment the substance is a coloured or fluorescent molecule, a radioisotope, or one or more metal ions to allow the particles to be distinguished from other sets of polymer particles by the mean intensity of the label.

In another embodiment the substance comprises metal ions to allow the particle to be separated by magnetism or distinguished from other sets of polymer particles by MRI or X-Ray.

In one embodiment the substance that binds to or is incorporated into the polymer particles is selected from the list comprising a protein or protein fragment, a peptide, a polypeptide, an antibody or antibody fragment, an antibody binding domain, an antigen, an antigenic determinant, an epitope, an immunogen or fragment thereof, a metal ion, a metal ion-coated molecule, biotin, avidin, streptavidin or derivatives thereof, an inhibitor, a co-factor, a substrate, an enzyme, a co-factor, a receptor, receptor subunit or fragment thereof, a ligand, an inhibitor, a monosaccharide, an oligosaccharide, a polysaccharide, a glycoprotein, a lipid, a cell or fragment thereof, a cell extract, a virus, a hormone, a serum protein, a milk protein, a macromolecule, a drug of abuse, or any combination of any two or more thereof.

In one embodiment the substance that binds to or is incorporated into the polymer particles is a skin care active selected from the group comprising sunscreen agents, particulate materials, conditioning agents, thickening agents, water-soluble vitamins, water-dispersible vitamins, oil-dispersible vitamins, emulsifying elastomers comprising dimethicone copolyol crosspolymers, non-emulsifying elastomers comprising dimethicone/vinyl dimethicone crosspolymers, oil-soluble skin care actives comprising oil-soluble terpene alcohols, phytosterols, anti-acne actives, beta-hydroxy acids, vitamin $B_3$ compounds, retinoids, anti-oxidants/radical scavengers, chelators, flavonoids, antiinflammatory agents, anti-cellulite agents, topical anesthetics, antiperspirants and fragrances, or any combination of any two or more thereof.

In one embodiment the substance that binds to or is incorporated into the polymer particles is a cleaning agent comprising:

(1) an enzyme selected from the list comprising cellulases, peroxidases, proteases, glucoamylases, amylases, lipases, cutinases, pectinases, reductases, oxidases, phenoloxidases, lipoxygenases, ligninases, pullulanases, tannases, pentosanases, malanases, β-glucanases, and arabinosidases, or

(2) an anti-redeposition agent selected from the group comprising methylcellulose, carboxymethylcellulose, hydroxyethylcellulose, polyacrylate polymers, copolymers of maleic anhydride and acrylic acid, copolymers of maleic anhydride and ethylene, copolymers of maleic anhydride and methylvinyl ether, copolymers of maleic anhydride and methacrylic acid, or

(3) any combination of two or more thereof.

In one embodiment the fusion partner is an enzyme selected from the list comprising cellulases, peroxidases, proteases, glucoamylases, amylases, lipases, cutinases, pectinases, reductases, oxidases, phenoloxidases, lipoxygenases, ligninases, pullulanases, tannases, pentosanases, malanases, β-glucanases, arabinosidases, racemases, hydrolases, dehydrogenases, polymerases, dioxygenases, monoxgenases, lyases, synthetases, epimerases, hydroxylases, transferases, transacylases and synthases.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

## DESCRIPTION OF THE DRAWINGS

Further aspects of the present invention will become apparent from the following description which is given by way of example only and with reference to the accompanying drawings.

Figure 1 shows a schematic overview of an *in vivo* produced polymer particle and the proteins and lipids which are associated with the particle.

Figure 2 shows an example of synthesis of polyhydroxyalkanoate polymer in *R. eutropha.* Polyhydroxyalkanoate polyhydroxybutyric acid (PHB) is produced in a three-stage process starting from the substrate acetyl-CoA. The C4 repeat unit in PHB is β-hydroxybutyric acid. The final step in the synthesis results in the formation of a polymer particle with a particle binding protein bound to the surface thereof.

Figure 3 shows the plasmid pBHR68-phaP-IL-2 which encodes a PHA operan for production of a polyhydroxybutyrate core β-hydroxybutyrate), and a fusion polypeptide comprising the phasin phaP and IL-2.

Figure 4 shows the plasmid pBHR68-phaP-MOG which encodes a PHA operan for production of a polyhydroxybutyrate core (3-hydroxybutyrate), and a fusion polypeptide comprising the phasin phaP and MOG.

Figure 5 shows a schematic view of the expression constructs of the plasmids shown in Figure 3 and Figure 4. A triangle represents the *lac* promoter; *phaP*, phasin gene; IL2, interleukin 2 gene; MOG, myelin oligodendrocyte glycoprotein encoding *gene*; *phaC*, PHA synthase encoding gene; *phaA*, gene encoding β-kethothiolase; *phaB*, gene encoding acetoacetyl-CoA reductase.

Figure 6 shows the plasmid pCWE (Peters, V. and Rehm, B.H.A. 2005, FEMS Microbiol. Lett. 248, 93-100) which encodes the PHA synthase from *Cupriavidus necator.*

Figure 7 shows the plasmid pBHR80 (Qi Q., Steinbüchel A., Rehm B.H.A. 2000, Appl. Microbiol. Biotechnol. 54: 37-43), which encodes a polyhydroxyalkanoate core (medium chain length 3-hydroxy fatty acids).

Figure 8 shows the detection of antigen-specific antibodies using antigen displaying PHA granules. In FIG 8A, MOG-phaP granules were incubated with serial dilutions of pooled antisera from five MOG (MOG) or OVA (OVA) immunized mice. Granules were extensively washed and then incubated with biotinylated anti-mouse IgG, followed by PE-conjugated streptavidin and FACS-based detection. The data was depicted in a "normalized" fashion (% of Max). One representative experiment, of at least two experiments performed, is depicted. In Figure 8B, the mean channel fluorescent for each dilution of antisera from MOG or OVA immunized mice binding to MOG-phaP was measured and depicted. In Figure 8C, ELISA was performed on anti-sera from MOG-and OVA-immunized mice. Serial dilutions of the anti-sera were added to MOG- or OVA-coated wells and incubated for 30 minutes. Biotinylated anti-mouse IgG was then added to the washed wells, followed by HRP-conjugated streptavidin and TMB substrate. The optical density was read at 450nm and the results from one representative experiment, of at least two experiments performed, are shown. In Figure 8C, data are displayed as mean ±SEM from triplicate samples.

Figure 9 shows MOG-phaP (column A) and IL-2-phaP (column B) fusion polypeptides on the surface of polymer particles can be detected using monoclonal antibodies specific for conformational epitopes and FACS. (i) particles incubated with directly labeled anti-IL-2-phycoerythrin. (ii) particles incubated with anti-MOG + biotinylated anti-mouse IgG + streptavidin-phycoerythrin, (iii) particles incubated with anti-MOG + directly labeled anti-mouse IgG-allophycocyanin. The filled histograms show the fluorescence of the MOG- or IL-2 polymer particles incubated with the anti-IL-2 or anti-MOG mAbs, respectively, as a negative control. The empty histograms show the fluorescence of the MOG- or IL-2 polymer particles incubated with anti-MOG mAbs or anti-IL-2, respectively.

Figure 10 shows ELISA using various PHA granules and anti-IgG antibodies for the detection of IgG bound to PHA granules. PHA granules were isolated from recombinant *E. coli* harboring various plasmids. Plasmids contained either the *lac* promoter or the T7 phage promoter for gene expression. The following versions of the PHA synthase mediated production of PHA granules: WT, wildtype PHA synthase; A(-), ZZ domain-PHA synthase fusion without signal peptide; A(+), ZZ domain-PHA synthase fusion plus signal peptide. Goat polyclonal anti-human IgG-horse radish peroxidase conjugates were used for detection of bound human IgG. Equal amounts of PHA granule protein (0.37 $\mu$g) corresponding to 2.6 $\mu$g polyhydroxybutyrate were added to each well. Measurements were conducted in quadruplets and the mean value and the standard deviation are indicated.

Figure 11 shows SDS-PAGE analysis of proteins bound *in vitro* either to ZZ domain-PHA synthase fusion granules or protein A sepharose and released after elution. M, molecular weight standard; 1, Human serum; 2, proteins eluted from protein A sepharose beads; 3, proteins eluted from wildtype PHA granules; 4, proteins eluted from ZZ domain-PHA synthase fusion granules displaying the ZZ domain without signal sequence. "A" and "B" indicates the heavy and light chains of IgG, respectively.

Figure 12 shows IL-2-phaP fusion polypeptides on the surface of polymer particles and cleavage of the IL-2 from the polymer particles using enterokinase. The histograms show IL-2-phaP expressing polymer particles labeled with anti-IL-2 mAbs directly coupled to phycoerythrin and exposed to enterokinase after (i) 0 hours, (ii) 1 hour, and (iii) 16 hours.

Figure 13 shows the vector pBAD-P-AChR. In this structure, the soluble domain of human acetylcholin-receptor (International Immunology (2000), Vol. 12, No. 9, pp. 1255-1265) was subcloned using Xho1 to the C-terminus of PhaP to create PhaP-AChR fusion polypeptides.

Figure 14 shows the vector pBAD-P-Mpl. In this structure, the soluble domain of human Thrombopoietin-Receptor (Mpl) (Biol. Pharm. Bull. (2004) 27(2): 219-221) was subcloned to the C-terminus of PhaP to create PhaP-Mpl fusion polypeptides.

Figure 15 shows the temperature stability of ZZ-PHA granules assessed by ELISA. ▲, ZZ-PHA granules displaying ZZ domain; ■, wildtype PHA granules. Measurements were conducted in triplicates and the mean value and the standard deviation are indicated.

Figure 16 shows ELISA applying the antibody capture assay using anti-$\beta$-galactosidase antibodies conjugated to HRP and PHA granules. PHA granules displaying LacZ-PhaC fusion polypeptides (A) were isolated from *P. aeruginosa* $\Delta$phaC1-Z-C2 (pBBR1JO5-lacZphaC1) and from wildtype *P. aeruginosa* PAO1 (B). After binding anti-$\beta$-galactosidase antibody conjugates to PHA granules attached to the microtiter plate, bound antibodies were quantified by using o-phenylenediamine solution and measuring the absorbance at a wavelength of 405 nm.

Figure 17 shows the IgG binding performance of polymer particles displaying ZZ domain-PHA synthase fusion polypeptides without signal peptide by FACS-based detection. PHA granules displaying ZZ domain-PhaC fusion polypeptides were isolated from cells containing pCWE-ZZ(-)phaC and pMCS69 (A), pBHR69-ZZ(-)phaC (B), and pET14b-ZZ (-)phaC (C). The isolated polymer particles were incubated with labeled mouse IgG2b monoclonal antibodies conjugated to phycoerythrin at three different concentrations: (i), 0 $\mu$g/mL IgG-PE; (ii), 1.0 $\mu$g/mL IgG-PE; and (iii), 10 $\mu$g/mL IgG-PE.

Figure 18 shows the IgG binding performance of polymer particles displaying ZZ domain-PHA synthase fusion polypeptides (A) compared with that of commercially available Protein A BioMag beads (B), using labeled mouse IgG2b monoclonal antibodies conjugated to phycoerythrin.

Figure 19 shows the IgG binding performance of polymer particles displaying ZZ domain-PHA synthase fusion polypeptides as a function of polymer size. PHA granules displaying ZZ domain-PhaC fusion polypeptides were isolated from cells containing pCWE-ZZ(-)phaC and pMCS69 (A), pBHR69-ZZ(-)phaC (B), and pET14b-ZZ(-)phaC (C). The isolated polymer particles in (A) and (B) had an average size of about 150 nm, whereas the polymer particles in (C) had an average size of about 100 nm. The polymer particles were incubated with labeled mouse IgG2b monoclonal antibodies conjugated to phycoerythrin a concentration of 10 $\mu$g/mL IgG-PE.

Figure 20 shows the size distribution of polymer particles displaying the IgG binding domain ZZ from protein A derived from pET14b-ZZ(-) phaC.

Figure 21 shows a schematic view of the microbial production of antigen displaying PHA granules, their use in binding antigen-specific antibodies followed by detection using labeled secondary antibodies.

## DETAILED DESCRIPTION OF THE INVENTION

The present invention relates to the use of polymer particles. In particular the present invention relates to the use of functionalised polymer particles. uses thereof. Functionalised polymer particles may comprise one or more surface-bound fusion polypeptides, one or more substances incorporated or adsorbed into the polymer particle core, one or more substances bound to surface bound fusion polypeptides, or a combination thereof.

## 1. Definitions

The terms "to alter expression of" and "altered expression" of a polynucleotide or polypeptide, are intended to encompass the situation where a polynucleotide is modified thus leading to altered expression of a polynucleotide or polypeptide. Modification of the polynucleotide may be through genetic transformation or other methods known in the art for inducing mutations. The "altered expression" can be related to an increase or decrease in the amount of messenger RNA and/or polypeptide produced and may also result in altered activity of a polypeptide due to alterations in the sequence of a polynucleotide and polypeptide produced.

The term "coding region" or "open reading frame" (ORF) refers to the sense strand of a genomic DNA sequence or a cDNA sequence that is capable of producing a transcription product and/or a polypeptide under the control of appropriate regulatory sequences. The coding sequence is identified by the presence of a 5' translation start codon and a 3' translation stop codon. When inserted into a genetic construct, a "coding sequence" is capable of being expressed when it is operably linked to promoter and terminator sequences.

The term "complex" as used herein refers to a polymer particle comprising a polymer core and at least one fusion polypeptide comprising an amino acid sequence encoding at least one particle binding domain and an amino acid sequence encoding at least one binding domain, wherein the binding domain is bound to a target component.

The term "comprising" as used in this specification means "consisting at least in part of". When interpreting statements in this specification which include that term, the features, prefaced by that term in each statement, all need to be present but other features can also be present.

The term "coupling reagent" as used herein refers to an inorganic or organic compound that is suitable for binding at least one substance or a further coupling reagent that is suitable for binding a coupling reagent on one side and at least one substance on the other side.

As used herein, the term "difficult folder polypeptide" refers to a polypeptide that when expressed recombinantly in a cellular expression system forms insoluble aggregations known as inclusion bodies, primarily of unfolded or partially-folded full or partial length polypeptides rather than correctly folded, native protein. The terms" insoluble aggregate" and "inclusion body" are used herein interchangeably.

The term "expression construct" refers to a genetic construct that includes the necessary elements that permit transcribing the insert polynucleotide molecule, and, optionally, translating the transcript into a polypeptide. An expression construct typically comprises in a 5' to 3' direction:

(1) a promoter, functional in the host cell into which the construct will be transformed,

(2) the polynucleotide to be expressed, and

(3) a terminator functional in the host cell into which the construct will be transformed.

Expression constructs of the disclosure may be inserted into a replicable vector for cloning or for expression, or may be incorporated into the host genome.

The terms "form a polymer particle" and "formation of polymer particles", as used herein, refer to the activity of a particle forming protein as discussed above.

A "fragment" of a polypeptide is a subsequence of the polypeptide that performs a function that is required for the enzymatic or binding activity and/or provides three dimensional structure of the polypeptide.

The term "fusion polypeptide", as used herein, refers to a polypeptide comprising two or more polypeptides fused through respective amino and carboxyl residues by a peptide linkage to form a single continuous polypeptide. It should be understood that the two or more polypeptides can either be directly fused or indirectly fused through their respective amino and carboxyl terimini through a linker or spacer or an additional polypeptide.

A fusion polypeptide according to the disclosure may comprise an amino acid sequence encoding a particle binding domain and an amino acid sequence encoding at least one fusion partner.

In one embodiment the amino acid sequences of the fusion polypeptide may be indirectly fused through a linker or spacer, the amino acid sequences of said fusion polypeptide arranged in the order of particle binding domain-linker-fusion partner, for example. In other embodiments the amino acid sequences of the fusion polypeptide may be indirectly fused through or comprise an additional polypeptide arranged in the order of particle binding domain-additional polypeptide-fusion partner, or particle binding domain-linker-fusion partner-additional polypeptide.

A fusion polypeptide according to the disclosure may also comprise one or more polypeptide sequences inserted within the sequence of another polypeptide. For example, a polypeptide sequence such as a protease recognition sequence may be inserted into a variable region of a protein comprising a particle binding domain.

The term "fusion partner" as used herein refers to a polypeptide such as a protein, a protein fragment, a binding domain, a target-binding domain, a binding protein, a binding protein fragment, an antibody, an antibody fragment, an antibody heavy chain, an antibody light chain, a single chain antibody, a single-domain antibody (a VHH for example), a Fab antibody fragment, an Fc antibody fragment, an Fv antibody fragment, a F(ab')2 antibody fragment, a Fab' antibody fragment, a single-chain Fv (scFv) antibody fragment, an antibody binding domain (a ZZ domain for example), an antigen, an antigenic determinant, an epitope, a hapten, an immunogen, an immunogen fragment, biotin, a biotin derivative, an avidin, a streptavidin, a substrate, an enzyme, an abzyme, a co-factor, a receptor, a receptor fragment, a receptor subunit, a receptor subunit fragment, a ligand, an inhibitor, a hormone, a lectin, a polyhistidine, a coupling domain, a DNA binding domain, a FLAG epitope, a cysteine residue, a library peptide, a reporter peptide, an affinity purification peptide, or any combination of any two or more thereof.

It should be understood that two or more polypeptides listed above can form the fusion partner.

Reference to a "binding domain" is intended to mean one half of a complementary binding pair and may include binding pairs from the list above. For example, antibody-antigen, antibody-antibody binding domain, biotin-streptavidin, receptor-ligand, enzyme-inhibitor pairs. A target-binding domain will bind a target molecule in a sample, and may be an antibody or antibody fragment, for example. A polypeptide-binding domain will bind a polypeptide, and may be an antibody or antibody fragment, or a binding domain from a receptor or signalling protein, for example.

Examples of substances that may be bound by a binding domain include a protein, a protein fragment, a peptide, a polypeptide, a polypeptide fragment, an antibody, an antibody fragment, an antibody binding domain, an antigen, an antigen fragment, an antigenic determinant, an epitope, a hapten, an immunogen, an immunogen fragment, a metal ion, a metal ion-coated molecule, biotin, a biotin derivative, avidin, streptavidin, an inhibitor, a co-factor, a substrate, an enzyme, an abzyme, a receptor, a receptor fragment, a receptor subunit, a receptor subunit fragment, a ligand, a receptor ligand, a receptor agonist, a receptor antagonist, a signalling molecule, a signalling protein, a signalling protein fragment, a growth factor, a growth factor fragment, a transcription factor, a transcription factor fragment, an inhibitor, a monosaccharide, an oligosaccharide, a polysaccharide, a glycoprotein, a lipid, a cell, a cell-surface protein, a cell-surface lipid, a cell-surface carbohydrate, a cell-surface glycoprotein, a cell extract, a virus, a virus coat protein, a hormone, a serum protein, a milk protein, a macromolecule, a drug of abuse, a coupling reagent, a polyhistidine, a pharmaceutically active agent, a biologically active agent, a label, a coupling reagent, a library peptide, an expression construct, a nucleic acid or any combination of any two or more thereof.

Examples of DNA binding domains include TraI and methyl transferase.

Such substances may be "target components" in a sample that is analysed according to a method of the invention.

Any reference herein to an antibody or antibody fragment is also intended to encompass a labeled antibody or antibody fragment, for example a colorimetric enzyme-labeled antibody or antibody fragment, a dye-labeled antibody or antibody fragment, a fluorescently-labeled antibody or antibody fragment or a quantum dot-labeled antibody or antibody fragment.

The term "genetic construct" refers to a polynucleotide molecule, usually double-stranded DNA, which may have inserted into it another polynucleotide molecule (the insert polynucleotide molecule) such as, but not limited to, a cDNA molecule. A genetic construct may contain the necessary elements that permit transcribing the insert polynucleotide molecule, and, optionally, translating the transcript into a polypeptide. The insert polynucleotide molecule may be derived from the host cell, or may be derived from a different cell or organism and/or may be a recombinant polynucleotide. Once inside the host cell the genetic construct may become integrated in the host chromosomal DNA. The genetic construct may be linked to a vector.

The term "host cell" refers to a bacterial cell, a fungi cell, yeast cell, a plant cell, an insect cell or an animal cell such as a mammalian host cell that is either 1) a natural PHA particle producing host cell, or 2) a host cell carrying an expression construct comprising nucleic acid sequences endocing at least a thiolase and a reductase and optionally a phasin. Which genes are required to augment what the host cell lacks for polymer particle formation will be dependent on the genetic makeup of the host cell and which substrates are provided in the culture medium.

The terms "IgG-binding Protein A fragment", or "ZZ domain"as used herein, refer to a portion of the Protein A molecule that is able to bind IgG, including but not limited to the 132 amino acid ZZ domain having the sequence set forth in amino acids 48 to 179 of SEQ ID NO:11 (the pBHR80-ZZ domain including the leader peptide) or amino acids 2 to 133 of SEQ ID NO:12 (the pBHR80-ZZ domain without the leader peptide).

The term "inclusion bodies" as used herein refers to insoluble aggregates of recombinantly expressed polypeptides that comprise primarily of unfolded or partially-folded full or partial length polypeptides rather than correctly folded, native protein. The terms "inclusion bodies" and "insoluble aggregates" are used herein interchangeably.

The term "label" as used herein refers to a molecule that by its presence allows a particle to be distinguished from a particle that does not contain the label. Preferably the label includes any substance that allows identification of a desired event or state. For example, identification of the "desired event or state" allows particles to be distinguished based on whether or not they display the desired event or state. Preferably the label is a coloured or fluorescent molecule or a radioisotope. Alternatively the label comprises one or more metal ions to allow the particle to be separated by magnetism or distinguished from other sets ofpolymer particles by MRI or X-Ray. The label may be a fusion partner or may be bound to or absorbed into or incorporated within a polymer particle.

An example of dye incorporation is given in WO 2004020623 (Bernd Rehm). A number of fluorescent labels are also known in the art, comprising but not limited to fluorescein isothiocyanate (FITC) which fluoresces at about 530nm, phycoerythrin (PE) (575nm), texas red (620 nm), phycoerythrin-texas red (615 nm), allophycocyanin (APC) (660 nm), propidium iodide (PI) (660 nm), phycoerythrin-cyanine dye (BD Cy-Chrome) (670 nm), peridinin chlorophyll protein (perCP) (675 nm), peridinin chlorophyll protein- cyanine dye (perCP-Cy5.5) (694 nm) and), allophycocyanin-cyanine dye (APC-Cy7) (767 nm), for example.

The term "library peptide", as used herein, refers to an individual member of a peptide library. A library peptide preferably comprises any polypeptide of any length including intact proteins (e.g., peptides encoded by cDNA or cDNA fragments (either in-frame, out-of-frame, sense or antisense orientation), random peptides, or biased peptides comprising random amino acids). A peptide library is a collection of distinct polypeptides as defined above or a fragment of interest from one of these entities. Fragments of interest may include functional domains or epitopes, for example. Each peptide is encoded by a nucleic acid molecule which is expressed in the course of displaying the peptide library. A mixture of nucleic acid molecules encoding a peptide library is often referred to in the art as an expression library. The terms "peptide library" and "expression library" may be used interchangeably herein depending on the context. Techniques for preparing peptide libraries for expression according to a method of the invention are discussed below.

The term "linker or spacer" as used herein relates to an amino acid or nucleotide sequence that indirectly fuses two or more polypeptides or two or more nucleic acid sequences encoding two or more polypeptides. Preferably the linker or spacer is about 1, 5, 10, 15, 20, 25, 30, 35, 40, 45, 50, 55, 60, 65, 70, 75, 80, 85, 90, 95 or about 100 amino acids or nucleotides in length.

In one embodiment the linker or spacer may comprise a restriction enzyme recognition rite. In another embodiment the linker or spacer may comprise a protease cleavage recognition suequence such as enterokinase, thrombin or Factor Xa recognition sequence, or a self-splicing element such as an intein. In another embodiment the linker or spacer facilitates independent folding of the fusion polypeptides.

The term "mixed population", as used herein, refers to a population of entities, each entity within the population differing in some respect from another entity within the population. For example, when used in reference to a mixed population of expression constructs, this refers to a population of expression constructs where each expression construct differs in respect of the fusion polypeptide it encodes. Alternatively, when used in reference to a mixed population of fusion polypeptides, this refers to a population of fusion polypeptides where each fusion polypeptide differs in respect of the polymer particle binding domain, the protein that comprises a polymer particle binding domain or the particle forming protein, or the at least one fusion partner it contains. Still further, when used in reference to a mixed population of polymer particles, this refers to a population of polymer particles where each polymer particle differs in respect of the fusion polypeptide or fusion polypeptides it carries.

The term "nucleic acid" as used herein refers to a single- or double- stranded polymer of deoxyribonucleotide, ribonucleotide bases or known analogues of natural nucleotides, or mixtures thereof. The term includes reference to a specified sequence as well as to a sequence complimentary thereto, unless otherwise indicated. The terms "nucleic acid" and "polynucleotide" are used herein interchangeably.

"Operably-linked" means that the sequenced to be expressed is placed under the control of regulatory elements that include promoters, tissue-specific regulatory elements, temporal regulatory elements, enhancers, repressors and terminators.

The term "over-expression" generally refers to the production of a gene product in a host cell that exceeds levels of production in normal or non-transformed host cells. The term "overexpression" when used in relation to levels of messenger RNA preferably indicates a level of expression at least about 3-fold higher than that typically observed in a host cell in a control or non-transformed cell. More preferably the level of expression is at least about 5-fold higher, about 10-fold higher, about 15-fold higher, about 20-fold higher, about 25-fold higher, about 30-fold higher, about 35-fold higher, about 40-fold higher, about 45-fold higher, about 50-fold higher, about 55-fold higher, about 60-fold higher, about 65-fold higher, about 70-fold higher, about 75-fold higher, about 80-fold higher, about 85-fold higher, about 90-fold higher, about 95-fold higher, or about 100-fold higher or above, than typically observed in a control host cell or non-transformed cell.

Levels of mRNA are measured using any of a number of techniques known to those skilled in the art including, but not limited to Northern blot analysis.

The term "particle binding domain" as used herein refers to a polypeptide sequence that forms or comprises a domain capable of binding to the polymer core or to the phospholipid membrane surrounding the polymer core or both. The particle binding domain may, for example, be selected from a particle binding N-terminal fragment of a phasin or depolymerase protein or a C-terminal particle binding fragment of a synthase or a repressor protein. Examples of polypeptide sequences that comprise a polymer particle binding domain include a polymer depolymerase, a polymer regulator, a polymer synthase and a particle size-determining protein.

The C-terminal fragment of the surface protein phasin (PhaP) from *R. eutropha* (amino acid residues from >Ala141) is hydrophilic and may be replaced by fusion partners without preventing binding of the phasin via the polymer particle binding domain (i.e. the N-terminal fragment) to the surface of the polymer particles. This.binding is based on hydrophobic interactions and is reversible (Hanley, S.Z. et al, FEBS Letters 1999, Vol. 447, pp. 99-105). Similarly, the N-terminal fragment (BioF) of the PhaF phasin from P. putida can provide a polymer particle binding domain to attach fusion partners to the particle surface (Moldes C. et al, Appl Environ Microbiol. 2004 Jun;70(6):3205-12).

Similarly, the N-terminal fragment of PHA synthase protein (amino acids 1 to 100) is highly variable and may be deleted or replaced by fusion partners without inactivating the enzyme or preventing covalent attachment of the synthase via the polymer particle binding domain (i.e. the C-terminal fragment) to the polymer core. The polymer particle binding domain of the synthase comprises at least the catalytic domain of the synthase protein that mediates polymerisation of the polymer core and formation of the polymer particles.

The C-terminus (amino acid residue from > 180) of the intracellular polymer depolymerase of *R. eutropha* binds the enzyme to the core of the polymer particles (Saegusa, H. et al., J. Bacteriol. 2001, Vol. 183(1), pp. 94-100).

The N-terminus (amino acid residue from <140) of the expression products, embedded in or associated with the polymer, of the genes *phaI* and *phaF* from *Pseudomonas oleovorans* bind the proteins to the polyester core of the polymer particles (Prieto, M.A. et al., J. Bacteriol. 1999, Vol. 181(3), pp. 858-868).

The term "particle forming protein", as used herein, refers to proteins involved in the formation of the particle. It may, for example, be selected from the group of proteins which comprises a polymer depolymerase, a polymer regulator, a polymer synthase and a particle size-determining protein. Preferably the particle forming protein is selected from the group comprising a thiolase, a reductase, a polymer synthase and a phasin. A particle forming protein such as a synthase may catalyse the formation of a polymer particle by polymerising a substrate or a derivative of a substrate to form a polymer particle.

Alternatively, a particle forming protein such as a thiolase, a reductase or a phasin may facilitate the formation of a polymer particle by facilitating polymerisation. For example, a thiolase or reductase may catalyse production of suitable substrates for a polymerase. A phasin may control the size of the polymer particle formed. Preferably the particle forming protein comprises a particle binding domain and a particle forming domain.

As used herein, the term "particle-forming reaction mixture" refers to at least a polymer synthase substrate if the host cell or expression construct comprises a synthase catalytic domain or a polymer synthase and its substrate if the host cell or expression construct comprises another particle forming protein or a particle binding domain that is not a polymer synthase catalytic domain.

A "particle size-determining protein" refers to a protein that controls the size of the polymer particles. It may for example be derived from the family of phasin-like proteins, preferably selected from the those from the genera *Ralstonia, Alcaligenes* and *Pseudomonas ,* more preferably the phasin gene phaP from *Ralstonia eutropha* and the phasin gene phaF from *Pseudomonas oleovorans.* Phasins are amphiphilic proteins with a molecular weight of 14 to 28 kDa which bind tightly to the hydrophobic surface of the polymer particles. It may also comprise other host cell proteins that bind particles and influence particle size.

The term "peptide to enable affinity purification" or "affinity purification peptide", as used herein, refers to a peptide which binds to a known ligand. This peptide facilitates separation of formed particles from the host cell in which they were produced and collection of particles eluted from a chromatography matrix after screening, for example. Examples include an avidin or a biotin binding fragment thereof, a streptavidin or a biotin binding fragment thereof, biotin, Protein A or an IgG-binding fragment thereof (a ZZ domain for example), an epitope, a polyhistidine or a cellulose binding domain.

A "polymer regulator" as used herein refers to a protein which regulates the transcription of the genes phaA, phaB and phaC involved in the formation of the polymer particles. It is withdrawn from transcription regulation by binding to the particle surface. One example of such a regulator is the phasin repressor (phaR) from *R. eutropha,* which binds to the promoter of a phasin-like gene, the expression product of which regulates the size of polymer particles formed, and prevents the gene from being read. Because the phasin repressor is bound on the surface of the polymer particles formed, this site on the promoter is released and transcription of the underlying gene can begin.

A "polymer synthase" as used herein refers to a protein which is capable of catalysing the the formation of a polymer particle by polymerising a substrate or a derivative of a substrate to form a polymer particle. The nucleotide sequences of 59 PHA synthase genes from 45 different bacteria have been obtained, differing in primary structure, substrate specificity and subunit composition. A polymer synthase comprises at least the synthase catalytic domain at the C-terminus of the synthase protein that mediates polymerisation of the polymer and attachment of the synthase protein to the particle core. Polymer synthases for use in the present invention are described in detail in Rehm B. H. A., Biochem J., (2003), 376(1):15-33, which is herein incorporated by reference. For example, the polymer synthase may comprise a PHA polymer synthase from C. necator, P. aeruginosa, A. vinosum, B. megaterium, *H. marismortui, P. aureofaciens,* or *P. putida,* which have Accession No.s AY836680, AE004091, AB205104, AF109909, YP137339, AB049413 and AF150670, respectively.

The term "polypeptide", as used herein, encompasses amino acid chains of any length but preferably at least 5 amino acids, including full-length proteins, in which amino acid residues are linked by covalent peptide bonds. Polypeptides of the present invention may be purified natural products, or may be produced partially or wholly using recombinant or synthetic techniques. The term may refer to a polypeptide, an aggregate of a polypeptide such as a dimer or other multimer, a fusion polypeptide, a polypeptide variant, or derivative thereof.

The term "promoter" refers to non transcribed cis-regulatory elements upstream of the coding region that regulate gene transcription. Promoters comprise cis-initiator elements which specify the transcription initiation site and conserved boxes such as the TATA box, and motifs that are bound by transcription factors.

The term "protein that comprises a polymer particle binding domain" comprises any proteins other than the particle forming proteins defined above that binds to the polymer core or the phospholipids mayer of the polymer particle.

Examples of protein that comprises a polymer particle binding domain include other particle-associated proteins such as heat shock protein A/B (IbpA/B) of *E. coli* (Accession No.s P29209/P29210) that are thought to stabilize the interface between the hydrophobic particles and the hydrophilic cytoplasm, affecting the particle morphology and reducing the amount of cytosolic proteins bound to the particles. Other examples of particle-associated proteins include tufB and ybeD of *E. coli* (Accession No.s P02990 and P30977), and PhaI, PhaD and PhaS of pseudomonads, which are known to be particle-associated but which function has yet to be clarified (Rehm, B. Biotechnol Lett. 2006 Feb;28(4):207-13). It may also be any fusion polypeptide comprising a polymer particle binding domain.

The term "Protein A polypeptide", as used herein, refers to a polypeptide coding for the *Staphylococcus aureus* cell wall component known as Protein A. Protein A exhibits high affinity for the Fc portion of subclasses of IgG from many species. Such a polypeptide is known as an "antibody binding domain".

The term "reporter peptide", as used herein, refers to a peptide that is itself detectable or that catalyses production of a detectable product. Reporter peptides useful herein include lacZ, luciferase, alkaline phosphatases, peroxidases, or green fluorescent protein (GFP).

The term "target component" as used herein refers to a protein, a protein fragment, a peptide, a polypeptide, a polypeptide fragment, an antibody, an antibody fragment, an antibody binding domain, an antigen, an antigen fragment, an antigenic determinant, an epitope, a hapten, an immunogen, an immunogen fragment, a metal ion, a metal ion-coated molecule, biotin, a biotin derivative, avidin, streptavidin, an inhibitor, a co-factor, a substrate, an enzyme, an abzyme, a receptor, a receptor fragment, a receptor subunit, a receptor subunit fragment, a ligand, a receptor ligand, a receptor agonist, a receptor antagonist, a signalling molecule, a signalling protein, a signalling protein fragment, a growth factor, a growth factor fragment, a transcription factor, a transcription factor fragment, an inhibitor, a cytokine, a chemokine, an inflammatory mediator, a monosaccharide, an oligosaccharide, a polysaccharide, a glycoprotein, a lipid, a cell, a cell-surface protein, a cell-surface lipid, a cell-surface carbohydrate, a cell-surface glycoprotein, a cell extract, a virus, a virus coat protein, a hormone, a serum protein, a milk protein, a macromolecule, a drug of abuse, a coupling reagent, a polyhistidine, a pharmaceutically active agent, a biologically active agent, a label, a coupling reagent, a library peptide, an expression construct, a nucleic acid or a combination thereof.

The term "terminator" refers to sequences that terminate transcription, which are found in the 3' untranslated ends of genes downstream of the translated sequence. Terminators are important determinants of mRNA stability and in some cases have been found to have spatial regulatory functions.

The term "substance" when referred to in relation to being bound to or absorbed into or incorporated within a polymer particle is intended to mean a substance that is bound by a fusion partner or a substance that is able to be absorbed into or incorporated within a polymer particle.

Examples of substances that may be bound by a fusion partner binding domain are described above. Examples of substances able to be absorbed into or incorporated within a polymer particle include dyes and pharmaceutical agents, preferably lipophilic dyes and lipophilic pharmaceutical agents.

The term "variant" as used herein refers to polynucleotide or polypeptide sequences different from the specifically identified sequences, wherein one or more nucleotides or amino acid residues is deleted, substituted, or added. Variants may be naturally occurring allelic variants, or non-naturally occurring variants. Variants may be from the same or from other species and may encompass homologues, paralogues and orthologues. In certain embodiments, variants of the inventive polypeptides and polypeptides possess biological activities that are the same or similar to those of the inventive polypeptides or polypeptides. The term "variant" with reference to polypeptides and polypeptides encompasses all forms of polypeptides and polypeptides as defined herein.

*Polynucleotide variants*

Variant polynucleotide sequences preferably exhibit at least 50%, more preferably at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least %, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to a specified polynucleotide sequence. Identity is found over a comparison window of at least 20 nucleotide positions, preferably at least 50 nucleotide positions, at least 100 nucleotide positions, or over the entire length of the specified polynucleotide sequence.

Polynucleotide sequence identity can be determined in the following manner. The subject polynucleotide sequence is compared to a candidate polynucleotide sequence using BLASTN (from the BLAST suite of programs, version 2.2.10 [Oct 2004]) in bl2seq (Tatiana A. Tatusova, Thomas L. Madden (1999), "Blast 2 sequences - a new tool for comparing protein and nucleotide sequences", FEMS Microbiol Lett. 174:247-250), which is publicly available from NCBI (ftp://ftp.ncbi.nih.gov/blast/). The default parameters of bl2seq are utilized except that filtering of low complexity parts should be turned off.

The identity of polynucleotide sequences may be examined using the following unix command line parameters:

bl2seq -i nucleotideseq 1 -j nucleotideseq2 -F F -p blastn

The parameter -F F turns off filtering of low complexity sections. The parameter-p selects the appropriate algorithm for the pair of sequences. The bl2seq program reports sequence identity as both the number and percentage of identical nucleotides in a line "Identities = ".

Polynucleotide sequence identity may also be calculated over the entire length of the overlap between a candidate and subject polynucleotide sequences using global sequence alignment programs (e.g. Needleman, S. B. and Wunsch, C. D. (1970) J. Mol. Biol. 48, 443-453). A full implementation of the Needleman-Wunsch global alignment algorithm is found in the needle program in the EMBOSS package (Rice,P. Longden,I. and Bleasby,A. EMBOSS: The European Molecular Biology Open Software Suite, Trends in Genetics June 2000, vol 16, No 6. pp.276-277) which can be obtained from http://www.hgmp.mrc.ac.uk/Software/EMBOSS/. The European Bioinformatics Institute server also provides the facility to perform EMBOSS-needle global alignments between two sequences on line at http:/www.ebi.ac.uk/emboss/align/.

Alternatively the GAP program may be used which computes an optimal global alignment of two sequences without penalizing terminal gaps. GAP is described in the following paper: Huang, X. (1994) On Global Sequence Alignment. Computer Applications in the Biosciences 10, 227-235.

Polynucleotide variants of the present invention also encompass those which exhibit a similarity to one or more of the specifically identified sequences that is likely to preserve the functional equivalence of those sequences and which could not reasonably be expected to have occurred by random chance. Such sequence similarity with respect to polypeptides may be determined using the publicly available bl2seq program from the BLAST suite of programs (version 2.2.10 [Oct 2004]) from NCBI (ftp://ftp.ncbi.nih.gov/blast/).

The similarity of polynucleotide sequences may be examined using the following unix command line parameters:

bl2seq -i nucleotideseq 1 -j nucleotideseq2 -F F -p tblastx

The parameter -F F turns off filtering of low complexity sections. The parameter -p selects the appropriate algorithm for the pair of sequences. This program finds regions of similarity between the sequences and for each such region reports an "E value" which is the expected number of times one could expect to see such a match by chance in a database of a fixed reference size containing random sequences. The size of this database is set by default in the bl2seq program. For small E values, much less than one, the E value is approximately the probability of such a random match.

Variant polynucleotide sequences preferably exhibit an E value of less than $1 \times 10^{-10}$, more preferably less than $1 \times 10^{-20}$, less than $1 \times 10^{-30}$, less than $1 \times 10^{-40}$, less than $1 \times 10^{-50}$, less than $l \times 10^{-60}$, less than $1 \times 10^{-70}$, less than $1 \times 10^{-80}$, less than $1 \times 10^{-90}$, less than $1 \times 10^{-100}$, less than $1 \times 10^{-110}$, less than $1 \times 10^{-120}$ or less than $1 \times 10^{-123}$ when compared with any one of the specifically identified sequences.

Alternatively, variant polynucleotides of the present disclosure hybridize to a specified polynucleotide sequence, or complements thereof under stringent conditions.

The term "hybridize under stringent conditions", and grammatical equivalents thereof, refers to the ability of a polynucleotide molecule to hybridize to a target polynucleotide molecule (such as a target polynucleotide molecule immobilized on a DNA or RNA blot, such as a Southern blot or Northern blot) under defined conditions of temperature and salt concentration. The ability to hybridize under stringent hybridization conditions can be determined by initially hybridizing under less stringent conditions then increasing the stringency to the desired stringency.

With respect to polynucleotide molecules greater than about 100 bases in length, typical stringent hybridization conditions are no more than 25 to 30° C (for example, 10° C) below the melting temperature (Tm) of the native duplex (see generally, Sambrook et al., Eds, 1987, Molecular Cloning, A Laboratory Manual, 2nd Ed. Cold Spring Harbor Press; Ausubel et al., 1987, Current Protocols in Molecular Biology, Greene Publishing,). Tm for polynucleotide molecules greater than about 100 bases can be calculated by the formula Tm = 81. 5 + 0.41% (G + C-log (Na+). (Sambrook et al., Eds, 1987, Molecular Cloning, A Laboratory Manual, 2nd Ed. Cold Spring Harbor Press; Bolton and McCarthy, 1962, PNAS 84:1390). Typical stringent conditions for polynucleotide of greater than 100 bases in length would be hybridization conditions such as prewashing in a solution of 6X SSC, 0.2% SDS; hybridizing at 65°C, 6X SSC, 0.2% SDS overnight; followed by two washes of 30 minutes each in 1X SSC, 0.1% SDS at 65°C and two washes of 30 minutes each in 0.2X SSC, 0.1% SDS at 65°C.

With respect to polynucleotide molecules having a length less than 100 bases, exemplary stringent hybridization conditions are 5 to 10°C below Tm. On average, the Tm of a polynucleotide molecule of length less than 100 bp is reduced by approximately (500/oligonucleotide length)°C.

With respect to the DNA mimics known as peptide nucleic acids (PNAs) (Nielsen et al., Science. 1991 Dec 6; 254(5037):1497-500) Tm values are higher than those for DNA-DNA or DNA-RNA hybrids, and can be calculated using the formula described in Giesen et al., Nucleic Acids Res. 1998 Nov 1;26(21):5004-6. Exemplary stringent hybridization conditions for a DNA-PNA hybrid having a length less than 100 bases are 5 to 10°C below the Tm.

Variant polynucleotides of the present disclosure also encompasses polynucleotides that differ from the sequences of the invention but that, as a consequence of the degeneracy of the genetic code, encode a polypeptide having similar activity to a polypeptide encoded by a polynucleotide of the present invention. A sequence alteration that does not change the amino acid sequence of the polypeptide is a "silent variation". Except for ATG (methionine) and TGG (tryptophan), other codons for the same amino acid may be changed by art recognized techniques, e.g., to optimize codon expression in a particular host organism.

Polynucleotide sequence alterations resulting in conservative substitutions of one or several amino acids in the encoded polypeptide sequence without significantly altering its biological activity are also included in the invention. A skilled artisan will be aware of methods for making phenotypically silent amino acid substitutions (see, e.g., Bowie et al., 1990, Science 247, 1306).

Variant polynucleotides due to silent variations and conservative substitutions in the encoded polypeptide sequence may be determined using the publicly available bl2seq program from the BLAST suite of programs (version 2.2.10 [Oct 2004]) from NCBI (ftp://ftp.ncbi.nih.gov/blast/) via the tblastx algorithm as previously described.

***Polypeptide Variants***

The term "variant" with reference to polypeptides encompasses naturally occurring, recombinantly and synthetically produced polypeptides. Variant polypeptide sequences preferably exhibit at least 50%, more preferably at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71 %, at least 72%, at least 73%, at least 74%, at least 75%, at least 76%, at least %, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to a sequences of the present invention. Identity is found over a comparison window of at least 20 amino acid positions, preferably at least 50 amino acid positions, at least 100 amino acid positions, or over the entire length of a polypeptide of the invention.

Polypeptide sequence identity can be determined in the following manner. The subject polypeptide sequence is compared to a candidate polypeptide sequence using BLASTP (from the BLAST suite of programs, version 2.2.10 [Oct 2004]) in bl2seq, which is publicly available from NCBI (ftp://ftp.ncbi.nih.gov/blast/). The default parameters of bl2seq are utilized except that filtering of low complexity regions should be turned off.

Polypeptide sequence identity may also be calculated over the entire length of the overlap between a candidate and subject polynucleotide sequences using global sequence alignment programs. EMBOSS-needle (available at http: /www.ebi.ac.uk/emboss/align/) and GAP (Huang, X. (1994) On Global Sequence Alignment. Computer Applications in the Biosciences 10, 227-235.) as discussed above are also suitable global sequence alignment programs for calculating polypeptide sequence identity.

Polypeptide variants of the present invention also encompass those which exhibit a similarity to one or more of the specifically identified sequences that is likely to preserve the functional equivalence of those sequences and which could not reasonably be expected to have occurred by random chance. Such sequence similarity with respect to polypeptides may be determined using the publicly available bl2seq program from the BLAST suite of programs (version 2.2.10 [Oct 2004]) from NCBI (ftp://ftp,ncbi.nih.gov/blast/). The similarity of polypeptide sequences may be examined using the following unix command line parameters:

```
bl2seq -i peptideseq1 -j peptideseq2 -F F -p blastp
```

Variant polypeptide sequences preferably exhibit an E value of less than 1 x $10^{-10}$, more preferably less than 1 x 10 $^{-20}$, less than 1 x 10 $^{-30}$, less than 1 x 10 $^{-40}$, less than 1 x 10 $^{-50}$, less than 1 x 10 $^{-60}$, less than 1 x 10 $^{-70}$, less than 1 x 10 $^{-80}$, less than 1 x10 $^{-90}$, less than 1 x 10 $^{-100}$, less than 1 x 10 $^{-110}$, less than 1 x $10^{-120}$ or less than 1 x 10 $^{-123}$ when compared with any one of the specifically identified sequences.

The parameter -F F turns off filtering of low complexity sections. The parameter -p selects the appropriate algorithm for the pair of sequences. This program finds regions of similarity between the sequences and for each such region reports an "E value" which is the expected number of times one could expect to see such a match by chance in a database of a fixed reference size containing random sequences. For small E values, much less than one, this is approximately the probability of such a random match.

Conservative substitutions of one or several amino acids of a described polypeptide sequence without significantly altering its biological activity are also included in the invention. A skilled artisan will be aware of methods for making phenotypically silent amino acid substitutions (see, e.g., Bowie et al., 1990, Science 247,1306).

A polypeptide variant of the present disclosure also encompasses that which is produced from the nucleic acid encoding a polypeptide, but differs from the wild type polypeptide in that it is processed differently such that it has an altered amino acid sequence. For example a variant may be produced by an alternative splicing pattern of the primary RNA transcript to that which produces a wild type polypeptide.

The term "vector" refers to a polynucleotide molecule, usually double stranded DNA, which is used to transport the genetic construct into a host cell. The vector may be capable of replication in at least one additional host system, such as *E. coli.*

**2. Expression Construct Preparation**

Processes for producing and using expression constructs for expression of fusion polypeptides in microorganisms, plant cells or animal cells (cellular expression systems) or in cell free expression systems, and host cells comprising expression constructs useful for forming polymer particles for use in the invention are well known in the art (e.g. Sambrook et al., Molecular Cloning: A Laboratory Manual, 2nd Ed. Cold Spring Harbor Press, 1987; and Ausubel et al., Current Protocols in Molecular Biology, Greene Publishing, 1987).

Expression constructs for use in methods of the invention may be inserted into a replicable vector for cloning or for expression, or may be incorporated into the host genome. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more selectable marker genes, an enhancer element, a promoter, and a transcription termination sequence. Construction of suitable vectors containing one or more of these components employs standard ligation techniques known in the art.

Both expression and cloning vectors contain a nucleic acid sequence that enables the vector to replicate in one or more selected host cells. Such sequences are well known for a variety of bacteria, yeast, and viruses.

In one embodiment the expression construct is present on a high copy number vector.

In one embodiment the high copy number vector is selected from those that may be present at 20 to 3000 copies per host cell.

In one embodiment the high copy number vector contain a high copy number origin of replication (ori), such as ColE1 or a a ColE1-derived origin of replication. For example, the ColE-1 derived origin of replication may comprise the pUC 19 origin of replication.

Numerous high copy number origins of replication suitable for use in the vectors of the present invention are known to those skilled in the art. These include the ColE1-derived origin of replication from pBR322 and its derivatives as well as other high copy number origins of replication, such as M13 FR ori or p15A ori. The 2μ plasmid origin is suitable for yeast, and various viral origins (SV40, polyoma, adenovirus, VSV or BPV) are useful for cloning vectors in mammalian cells.

Preferably, the high copy number origin of replication comprises the ColE1-derived pUC 19 origin of replication.

The restriction site is positioned in the origin of replication such that cloning of an insert into the restriction site will inactivate the origin, rendering it incapable of directing replication of the vector. Alternatively, the at least one restriction site may be positioned within the origin such that cloning of an insert into the restriction site will render it capable of supporting only low or single copy number replication of the vector.

Expression and cloning vectors will typically contain a selection gene, also termed a selectable marker to detect the presence of the vector in the transformed host cell. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

Selectable markers commonly used in plant transformation include the neomycin phophotransferase II gene (NPT II) which confers kanamycin resistance, the aadA gene, which confers spectinomycin and streptomycin resistance, the phosphinothricin acetyl transferase (*bar* gene) for Ignite (AgrEvo) and Basta (Hoechst) resistance, and the hygromycin phosphotransferase gene ( hpt) for hygromycin resistance.

Examples of suitable selectable markers for mammalian cells are those that enable the identification of cells competent to take up expression constructs, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

An expression construct useful for forming polymer particles preferably includes a promoter which controls expression of at least one nucleic acid encoding a polymer synthase, particle forming protein or fusion polypeptide.

Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the nucleic acid encoding a polymer synthase, particle forming protein or fusion polypeptide..

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization.

Examples of suitable promoters for use in plant host cells, including tissue or organ of a monocot or dicot plant include cell-, tissue- and organ-specific promoters, cell cycle specific promoters, temporal promoters, inducible promoters, constitutive promoters that are active in most plant tissues, and recombinant promoters. Choice of promoter will depend upon the temporal and spatial expression of the cloned polynucleotide, so desired. The promoters may be those from the host cell, or promoters which are derived from genes of other plants, viruses, and plant pathogenic bacteria and fungi. Those skilled in the art will, without undue experimentation, be able to select promoters that are suitable for use in modifying and modulating expression constructs using genetic constructs comprising the polynucleotide sequences of the invention. Examples of constitutive plant promoters include the CaMV 35S promoter, the nopaline synthase promoter and the octopine synthase promoter, and the Ubi 1 promoter from maize. Plant promoters which are active in specific tissues, respond to internal developmental signals or external abiotic or biotic stresses are described in the scientific literature. Exemplary promoters are described, e.g., in WO 02/00894, which is herein incorporated by reference.

Examples of suitable promoters for use in mammalian host cells comprise those obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of an expression construct by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the polymer synthase, particle forming protein or fusion polypeptide coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding the polymer synthase, particle forming protein or fusion polypeptide.

In one embodiment the expression construct comprises an upstream inducible promoter, such as a BAD promoter, which is induced by arabinose.

In one embodiment the expression construct comprises a constitutive or regulatable promoter system.

In one embodiment the regulatable promoter system is an inducible or repressible promoter system.

While it is desirable to use strong promoters in the production of recombinant proteins, regulation of these promoters is essential since constitutive overproduction of heterologous proteins leads to decreases in growth rate, plasmid stability and culture viability.

A number of promoters are regulated by the interaction of a repressor protein with the operator (a region downstream from the promoter). The most well known operators are those from the lac operon and from bacteriophage A. An overview of regulated promoters in *E. coli* is provided in Table 1 of "Parameters Influencing the Productivity of Recombinant E. coli Cultivations" Friehs & Reardon. Advances in Biochemical Engineering Technology Vol 48 -Springer Verlag (1991).

A major difference between standard bacterial cultivations and those involving recombinant *E. coli* is the separation of the growth and production or induction phases. Recombinant protein production often takes advantage of regulated promoters to achieve high cell densities in the growth phase (when the promoter is "off" and the metabolic burden on the host cell is slight) and then high rates of heterologous protein production in the induction phase (following induction to turn the promoter "on").

In one embodiment the regulatable promoter system is selected from LacI, Trp, phage $\gamma$ and phage RNA polymerase.

In one embodiment the promoter system is selected from the lac or $P_{tac}$ promoter and the lacI repressor, or the trp promoter and the TrpR repressor.

In one embodiment the LacI repressor is inactivated by addition of isopropyl-ß-D-thiogalactopyranoside (IPTG) which binds to the active repressor causes dissociation from the operator, allowing expression.

In one embodiment the trp promoter system uses a synthetic media with a defined tryptophan concentration, such that when the concentration falls below a threshold level the system becomes self-inducible. In one embodiment 3-ß-indole-acrylic acid may be added to inactivate the TrpR repressor.

In one embodiment the promoter system may make use of the bacteriophage $\gamma$ repressor cI. This repressor makes use of the $\gamma$ prophage and prevent expression of all the lytic genes by interacting with two operators termed $O_L$ and $O_R$. These operators overlap with two strong promoters $P_L$ and $P_R$ respectively. In the presence of the cI repressor, binding of RNA polymerase is prevented. The cI repressor can be inactivated by UV-irradiation or treatment of the cells with mitomycin C. A more convenient way to allow expression of the recombinant polypeptide is the application of a temperature-sensitive version of the cI repressor *cI857.* Host cells carrying a y-based expression system can be grown to mid-exponential phase at low temperature and then transferred to high temperature to induce expression of the recombinant polypeptide.

A widely used expression system makes use of the phage T7 RNA polymerase which recognises only promoters found on the T7 DNA, and not promoters present on the host cell chromosome. Therefore, the expression construct may contain one of the T7 promoters (normally the promoter present in front of gene 10) to which the recombinant gene will be fused. The gene coding for the T7 RNA polymerase is either present on the expression construct, on a second compatible expression construct or integrated into the host cell chromosome. In all three cases, the gene is fused to an inducible promoter allowing its transcription and translation during the expression phase.

The *E. coli* strains BL21 (DE3) and BL21 (DE3) pLysS (Invitrogen, CA) are examples of host cells carrying the T7 RNA polymerase gene. Other cell strains carrying the T7 RNA polymerase gene are known in the art, such as *Pseudomonas aeruginosa* ADD1976 harboring the T7 RNA polymerase gene integrated into the genome (Brunschwig & Darzins, 1992, Gene 111, 35-41) and *Cupriavidus necator* (formerly *Ralstonia eutropha)* harboring the T7 RNA polymerase gene integrated into the genome under phaP promoter control (Barnard et al. Protein Expr Purif. 2004 Dec;38(2): 264-71).

The T7 RNA polymerase offers three advantages over the host cell enzymes: First, it consists of only one subunit, second it exerts a higher processivity, and third it is insensitive towards rifampicin. The latter characteristic can be used especially to enhance the amount of fusion polypeptide by adding this antibiotic about 10 min after induction of the gene coding for the T7 RNA polymerase. During that time, enough polymerase has been synthesised to allow high-level expression of the fusion polypeptide, and inhibition of the host cell enzymes prevents further expression of all the other genes present on both the plasmid and the chromosome. Other antibiotics which inhibit the bacterial RNA polymerase but not the T7 RNA polymerase are known in the art, such as streptolydigin and streptovaricin.

Since all promoter systems are leaky, low-level expression of the gene coding for T7 RNA polymerase may be deleterious to the cell in those cases where the recombinant polypeptide encodes a toxic protein. These polymerase molecules present during the growth phase can be inhibited by expressing the T7-encoded gene for lysozyme. This enzyme is a bifunctional protein that cuts a bond in the cell wall of the host cell and selectively inhibits the T7 RNA polymerase by binding to it, a feed-back mechanism that ensures a controlled burst of transcription during T7 infection. The *E. coli* strain BL21 (DE3) pLysS is an example of a host cell that carries the plasmid pLysS that constitutively expresses T7 lysozyme.

In one embodiment the promoter system makes use of promoters such as API or APR which may be induced or "switched on" to initiate the induction cycle by a temperature shift, such as by elevating the temperature from about 30-37°C to 42°C to initiate the induction cycle.

A strong promoter may enhance fusion polypeptide density at the surface of the particle during in-vivo production.

Preferred fusion polypeptides comprise:

(1) a polymer particle binding domain, a protein that comprises a polymer particle binding domain, or a particle forming protein, or any combination of any two or more thereof, and

(2) a fusion partner comprising

(i) at least one polypeptide, or

(ii) at least one binding domain, or

(iii) at least one reporter peptide, or

(iv at least one affinity purification peptide, or

(v) any combination of any two or more of (i) to (iv).

For example, in one embodiment the fusion partner may comprise

(i) at least one binding domain and at least one reporter peptide, or

(ii) at least one binding domain and at least one affinity purification peptide, or

(iii) at least one binding domain and at least one reporter peptide and at least one affinity purification peptide.

For example, in another embodiment the fusion partner may comprise

(i) at least one polypeptide and at least one binding domain, or

(ii) at least one polypeptide and at least one reporter peptide, or

(iii) at least one polypeptide and at least one affinity purification peptide, or

(iv) at least one polypeptide and at least one binding domain and at least one reporter peptide, or

(v) at least one polypeptide and at least one binding domain and at least one affinity purification peptide, or

(vi) at least one polypeptide and at least one binding domain and at least one reporter peptide and at least one affinity purification peptide.

A nucleic acid sequence encoding a fusion polypeptide for use herein comprises a nucleic acid encoding a particle binding domain, a protein that comprises comprising a particle binding domain or a particle forming protein and a nucleic acid sequence encoding at least one fusion partner. Once expressed, the fusion polypeptide is able to form or facilitate formation of a polymer particle or simply bind to a formed or forming polymer particle, as discussed herein.

In one embodiment the nucleic acid sequence encoding at least one fusion partner is indirectly fused with the nucleic acid sequence encoding a particle binding domain, a particle that comprises comprising a particle binding domain or a particle forming protein through a polynucleotide linker or spacer sequence of a desired length.

In one embodiment the polynucleotide linker or spacer sequence encodes a protease cleavage recognition sequence.

In one embodiment the amino acid sequence of the fusion polypeptide encoding at least one fusion partner is contiguous with the C-terminus of the amino acid sequence encoding a phasin, preferably a phaP phasin or a N-terminal phasin fragment.

In one embodiment the amino acid sequence of the fusion protein encoding at least one fusion partner is indirectly fused with the N-terminus of the amino acid sequence encoding a phasin or a C-terminal phasin fragment through a peptide linker or spacer of a desired length that facilitates independent folding of the fusion polypeptides.

In one embodiment the amino acid sequence of the fusion polypeptide encoding at least one fusion partner is contiguous with the N-terminus of the amino acid sequence encoding a synthase or a C-terminal synthase fragment.

In one embodiment the amino acid sequence of the fusion protein encoding at least one fusion partner is indirectly fused with the C-terminus of the amino acid sequence encoding a synthase or a N-terminal synthase fragment through a peptide linker or spacer of a desired length to facilitate independent folding of the fusion polypeptides.

In one embodiment the amino acid sequence of the fusion polypeptide encoding at least one fusion partner is contiguous with the N-terminus of the amino acid sequence encoding a depolymerase, or a C-terminal depolymerase fragment.

One advantage of the fusion polypeptides according to the present disclosure is that the modification of the proteins binding to the surface of the polymer particles does not affect the functionality of the proteins involved in the formation of the polymer particles. For example, the functionality of the polymer synthase is retained if a recombinant polypeptide is fused with the N-terminal end thereof, resulting in the production of recombinant polypeptide on the surface of the particle. Should the functionality of a protein nevertheless be impaired by the fusion, this shortcoming may be offset by the presence of an additional particle forming protein which performs the same function and is present in an active state.

In this manner, it is possible to ensure a stable bond of the recombinant polypeptide bound to the polymer particles via the particle forming proteins.

In one embodiment the nucleic acid may also comprise any one or more of

(1) at least one nucleic acid sequence that.codes for a particle forming protein, the protein comprising a polymer particle binding domain, or

(2) at least one nucleic acid sequence that codes for an additional fusion polypeptide, the additional fusion polypeptide comprising:

(a) a polymer particle binding domain, a protein that comprises a polymer particle binding domain, a particle forming protein, or a combination thereof, and

(b) a fusion partner comprising at least one polypeptide or at least one binding domain or one or more coupling reagents or a combination thereof, or

(c) a fusion partner comprising at least one polypeptide and at least one binding domain or one or more coupling reagents or a combination thereof, or

(d) at least one reporter peptide or affinity purification peptide, or

(3) any combination of two or more thereof;

In one embodiment the reporter peptide is a fluorescent peptide or a reporter enzyme.

In one embodiment the reporter peptide is selected from lacZ, luciferase, alkaline phosphatase, peroxidase, and green fluorescent protein (GFP).

It should be appreciated that the arrangement of the proteins in the fusion polypeptide may be dependent on the order of gene sequences in the nucleic acid contained in the plasmid. For example, it may be desired to produce a fusion polypeptide wherein the particle binding domain or a particle forming protein comprising a particle binding domain is indirectly fused to the polypeptide. The term "indirectly fused" refers to a fusion polypeptide comprising at least a particle binding domain or a particle forming protein comprising a particle binding domain and a polypeptide that are separated by an additional protein which may be any protein that is desired to be expressed in the fusion polypeptide.

In one embodiment the additional protein is selected from a particle forming protein or a fusion polypeptide, or a linker or spacer to facilitate independent folding of the fusion polypeptides, as discussed above. In this embodiment it would be necessary to order the sequence of genes in the plasmid to reflect the desired arrangement of the fusion polypeptide.

In one embodiment the particle binding domain or a particle forming protein comprising a particle binding domain may be directly fused to the polypeptide. The term "directly fused" is used herein to indicate where two or more peptides are linked via peptide bonds.

It may also be possible to form a particle wherein the particle comprises at least two distinct fusion polypeptides that are bound to the polymer core. For example, a first fusion polypeptide comprising a particle binding domain or a particle forming protein comprising a particle binding domain fused to a peptide library could be bound to the polymer core. In addition to this, at least one additional fusion polypeptide could be bound to the polymer core at a different site to said first protein. The additional fusion polypeptide may include a particle forming protein, a fusion polypeptide, a reporter peptide or an affinity purification peptide as discussed above.

In one embodiment the expression construct is expressed *in vivo.* Preferably the expression construct is a plasmid which is expressed in a microorganism, preferably *Escherichia coli.*

In one embodiment the expression construct is expressed *in vitro.* Preferably the expression construct is expressed *in vitro* using a cell free expression system.

In one embodiment one or more genes can be inserted into a single expression construct, or one or more genes can be integrated into the host cell genome. In all cases expression can be controlled through promoters as described above.

In one embodiment the expression construct further encodes at least one additional fusion polypeptide comprising a particle binding domain or a particle forming protein comprising a particle binding domain and at least one or more of a particle forming protein, a fusion polypeptide, a reporter peptide or an affinity purification peptide as discussed above.

In one embodiment the method of the invention further comprises providing at least one additional expression construct encoding at least one additional fusion polypeptide comprising a particle binding domain or a particle forming protein comprising a particle binding domain and at least one or more of a particle forming protein, a fusion polypeptide, a reporter peptide or an affinity purification peptide as discussed above.

In one embodiment the expression construct comprises an expression construct binding peptide binding domain. Preferably the expression construct binding peptide and the expression construct binding peptide binding domain are unique to a single expression construct or group of expression constructs.

Preferably the peptide to enable affinity purification is unique to a single expression construct or group of expression constructs.

Plasmids useful herein are shown in the Figures and are described in detail in published PCT International Application WO 2004020623 (Bernd Rehm)

3. Peptide Library Formation

One aspect of the invention relates to displaying and screening peptides and peptide libraries. In one embodiment, the invention relates to production of polymer particles for displaying and screening peptides and peptide libraries using polymer particles.

Processes for producing peptide libraries are well known in the art (see for example Crossley R, 2004; Mori T, 2004). Any technique for generating an expressible library of peptides (an expression library) that is known in the art may be employed to generate a peptide library for display according to the present invention.

A peptide library useful herein may be prepared from the entire genetic complement of an organism or a portion thereof or prepared from a single parent sequence that encodes a protein of interest. Alternatively the peptide library may be an engineered library. Diversity in libraries may be introduced using known techniques including mutagenesis, PCR and varying the reaction mixture used to synthesise oligonucleotides.

Peptide libraries useful herein may comprise a collection of distinct peptides where each peptide comprises a protein, a protein fragment, a binding domain, a target-binding domain, a binding protein, a binding protein fragment, an antibody, an antibody fragment, an antibody heavy chain, an antibody light chain, a single chain antibody, a single-domain antibody, a Fab antibody fragment, an Fc antibody fragment, an Fv antibody fragment, a F(ab')2 antibody fragment, a Fab' antibody fragment, a single-chain Fv (scFv) antibody fragment, an antibody binding domain, an antigen, an antigenic determinant, an epitope, a hapten, an immunogen, an immunogen fragment, biotin, a biotin derivative, an avidin, a streptavidin, a substrate, an enzyme, an abzyme, a co-factor, a receptor, a receptor fragment, a receptor subunit, a receptor subunit fragment, an inhibitor, a coupling domain, or a combination thereof. Receptors involved in cellular signalling are of particular interest

Such receptors comprise G-protein-coupled receptors, acetylcholine receptors, thrombopoetin receptors, nuclear receptors, chemokine receptors, steroid hormone receptors, epidermal growth factor receptors, toll receptors, toll-like receptors, mannose receptors, 7TM receptors, neuropeptide receptors, NMDA receptors, T cell receptors, hormone receptors, IgG Fc receptors or cytokinine receptors, and sub types thereof as described above.

*Genomic / cDNA Libraries*

Chromosomal DNA may be fragmented to a desired size by sonication, DNase treatment or shearing with a gauge needle, for example.
Sonication has the advantage that a sample can be sonicated for a few seconds, the fragment size analysed by gel electrophoresis, and if the fragments are regarded too long, the sonication can easily be repeated. Thus, fragments of the desired length can easily be obtained. The size desired depends on the intended use of the library. If the main purpose is to map a binding domain, small fragments could be used, while for identification of genes encoding binding proteins, larger fragments are preferred.
A DNA library can be formed by digesting both a plasmid genome and cellular DNA with the same restriction nuclease. The resulting DNA fragments are then added to the cut plasmids and annealed via their cohesive ends to form recombinant DNA.
A cDNA library can be formed by the same method by extracting mRNA and making a cDNA copy catalysed by a reverse transcriptase enzyme (Johnson et al., 1998). The single-stranded DNA molecules are then converted into double-stranded DNA molecule by DNA polymerase and cut with a restriction nuclease as described above.
It should be apparent that any selection of DNA or RNA that can isolated can then be digested into fragments (using a restriction nuclease for example) to generate an expression library encoding a peptide library (Cho G 2000; Wilson D et al., 2001).

*Randomisation/Mutagenesis*

One or more polypeptides from one or more families of molecules could be advantageously randomised to provide a library of candidate molecules for use in the methods of the invention. Preferably, the regions of the molecule known to be important for a particular function, such as an active site, a protein binding site or a nucleic acid binding site could be randomised. However, it may be desirable to randomize other regions of the molecule, such as those involved with formation of secondary, tertiary or quaternary structure.
Techniques such as site-directed or random mutagenesis may be employed to generate mutated polynucleotides which may then be ligated into plasmids for expression in a method of the invention or fragmented prior to ligation into plasmids.
Mutations may be performed by any method known to those of skill in the art. Preferred, however, is site-directed mutagenesis of a nucleic acid sequence encoding a protein of interest. A number of methods for site-directed mutagenesis are known in the art, from methods employing single-stranded phage such as M13 to PCR-based techniques. These include the following techniques:

> Error-prone PCR is a process for performing PCR under conditions where the copying fidelity of the DNA polymerase is low, such that a high rate of point mutations is obtained along the entire length of the PCR product. Leung, D. W., et al., Technique, 1:11-15 (1989) and Caldwell, R. C. & Joyce G. F., PCR Methods Applic., 2:28-33 (1992).

Oligonucleotide directed mutagenesis is a process which allows for the generation of site-specific mutations in any cloned DNA segment of interest. Reidhaar-Olson, J. F. & Sauer, R. T., et al., Science, 241:53-57 (1988).
Assembly PCR is a process which involves the assembly of a PCR product from a mixture of small DNA fragments. A large number of different PCR reactions occur in parallel in the same vial, with the products of one reaction priming the products of another reaction.
Sexual PCR mutagenesis (also known as "DNA shuffling") refers to forced homologous recombination between DNA molecules of different but highly related DNA sequence in vitro, caused by random fragmentation of the DNA molecule based on sequence homology, followed by fixation of the crossover by primer extension in a PCR reaction. Stemmer, W. P., PNAS, USA, 91:10747-10751 (1994).
*In vivo* mutagenesis is a process of generating random mutations in any cloned DNA of interest which involves the propagation of the DNA in a strain *of E. coli* that carries mutations in one or more of the DNA repair pathways. These "mutator" strains have a higher random mutation rate than that of a wild-type parent. Propogating the DNA in one of these strains will eventually generate random mutations within the DNA.
Cassette mutagenesis is a process for replacing a small region of a double stranded DNA molecule with a synthetic oligonucleotide "cassette" that differs from the native sequence. The oligonucleotide often contains completely and/or partially randomized native sequence.
Recursive ensemble mutagenesis refers to an algorithm for protein engineering (protein mutagenesis) developed to produce diverse populations of phenotypically related mutants whose members differ in amino acid sequence. This method uses a feedback mechanism to control successive rounds of combinatorial cassette mutagenesis. Arkin, A. P. and Youvan, D. C., PNAS, USA, 89:7811-7815 (1992).

Exponential ensemble mutagenesis is a process for generating combinatorial libraries with a high percentage of unique and functional mutants, wherein small groups of residues are randomized in parallel to identify, at each altered position, amino acids which lead to functional proteins, Delegrave, S. and Youvan, D. C., Biotechnology Research, 11: 1548-1552 (1993); and random and site-directed mutagenesis, Arnold, F. H., Current Opinion in Biotechnology, 4: 450-455 (1993).

*Antibody Library*

Large libraries of wholly or partially synthetic antibody combining sites, or paratopes, can be constructed yielding large libraries of monoclonal antibodies having diverse and novel immunospecificities (Feldhaus and Siegel 2004; Tribbick G 2002). Production of antibody libraries is reviewed in Adda et al (2002).

A library may be created by inserting a library of random oligonucleotides or a cDNA library encoding antibody fragment such as $V_L$ and $V_H$ into a plasmid. As a result, peptide libraries that contain diverse peptides can be constructed.

The diversity of a combinatorial antibody library can be increased by shuffling of the heavy and light chain genes, by altering the complementarily determining region 3 (CDR3) of the cloned heavy chain genes of the library and by introducing random mutations into the library by error-prone polymerase chain reactions (PCR).

Mutagenesis can be induced in a CDR of an immunoglobulin light chain gene for the purpose of producing light chain gene libraries for use in combination with heavy chain genes and gene libraries to produce antibody libraries of diverse and novel immunospecificities. The method comprises amplifying a CDR portion of an immunoglobulin light chain gene by polymerase chain reaction (PCR) using a PCR primer oligonucleotide and then isolating the amplified CDR to form a library of mutagenised immunoglobulin light chain genes. This isolated library of mutagenised light chain genes, in combination with one or more heavy chain genes, can be used to form a combinatorial antibody library of expressed heavy and light chain genes.

A semisynthetic antibody library composed of single chain Fv fragments could be constructed by replacing the heavy chain CDR3 region of a single chain Fv region by a random sequence of amino acids using trinucleotide codons.

Alternatively, any PCR method could be used that generates polynucleotides encoding polypeptides. In addition sonication of DNA can also be used to produce DNA fragments.

*Synthetic Peptides*

Peptide libraries may be prepared through chemical synthesis of oligonucleotides according to known techniques. Diversity may be introduced by varying the reaction mixtures used or by employing error prone synthetic methods or enzymes.

Suitable techniques used in microarray formation are reviewed by Seliger H, Hinz M, Happ E., "Arrays of immobilized oligonucleotides--contributions to nucleic acids technology", Curr Pharm Biotechnol., 2003, 4(6):379-95; and Gao X, Gulari E, Zhou X., "In situ synthesis of oligonucleotide microarrays", Biopolymers, 2004, 73(5):579-96.

For any of the methods described above, enrichment processes, such as PCR, could be used to further isolate or amplify nucleic acids encoding library members of interest.

## 4. Expression Construct Expression and Particle Production

In some aspects of the disclosure it is desirable to achieve overexpression of the expression constructs in the host cell. Overexpression can be achieved by i) use of a strong promoter system, for example the T7 RNa polymerase promoter system; ii) use of a high copy number plasmid, for example a plasmid containing the colE1 origin of replication or iii) stabilisation of the messenger RNA, for example through use of fusion sequences. The benefits of overexpression may allow the production of smaller particles where desired and the production of a higher number of polymer particles.

The formation of polyhydroxyalkanoate (PHA) particles and of the proteins involved in their formation are reported in Madison, L. L. et al, "Metabolic Erigineering of Poly(3-hydroxyalkanoates): From DNA to Plastic", Microbiology and Molecular Biology Reviews, (1999), 63(1):21-53; published PCT International Application WO 2004020623 (Bernd Rehm); and Rehm B. H. A., "Polyester synthases: natural catalysts for plastics", Biochem J., (2003), 376(1):15-33, Brockelbank JA. et al., Appl Environ Microbiol. 2006 Aug 25 [Epub ahead of print]; Peters V & Rehm BH., Appl Environ Microbiol. 2006 Mar;72(3):1777-83; B. Thomas Bäckström, Jane A Brockelbank and Bernd H.A. Rehm [*in press*] and Rehm BHA "Biopolyester particles produced by microbes or using polyester synthases: self assembly and potential applications" in Microbial Biotechnology: biological self-assembly systems and biopolymer-based nanostructures (Rehm BHA, Ed) Horizon Bioscience (2006), all of which are herein incorporated by reference.

An expression construct can be expressed by transforming a host cell with the expression construct comprising a nucleic acid sequence encoding a fusion polypeptide comprising a particle binding domain or a particle forming protein comprising a particle binding domain and at least one fusion partner comprising:

(1) at least one binding domain or one or more coupling reagents or a combination thereof, or

(2) at least one polypeptide, or

(3) a combination thereof.

Following transformation, the transformed host cell is cultured under conditions suitable for expression of the fusion polypeptides from the expression constructs and for formation of polymer particles. Such conditions comprise those suitable for expression of the chosen expression construct, such as a plasmid in a suitable organism as are known in the art. Provision of a suitable substrate in the culture media allows the particle forming protein component of a fusion polypeptide to form a polymer particle.

Host cells comprising expression constructs are useful in methods well known in the art (e.g. Sambrook et al., Molecular Cloning: A Laboratory Manual, 2nd Ed. Cold Spring Harbor Press, 1987 ; Ausubel et al., Current Protocols in Molecular Biology, Greene Publishing, 1987) for recombinant production of polymer particles for use in processes of the present invention.

At least one fatty acid with functional side groups is preferably introduced into the culture medium as a substrate for the formation of the polymer particles, with at least one hydroxy fatty acid and/or at least one mercapto fatty acid and/or at least one β-amino fatty acid particularly preferably being introduced. "Fatty acids with functional side groups" should be taken to mean saturated or unsaturated fatty acids. These also include fatty acids containing functional side groups which are selected from the group comprising methyl groups, alkyl groups, hydroxyl groups, phenyl groups, sulfhydryl groups, primary, secondary and tertiary amino groups, aldehyde groups, keto groups, ether groups, carboxyl groups, O-ester groups, thioester groups, carboxylic acid amide groups, hemiacetal groups, acetal groups, phosphate monoester groups and phosphate diester groups. Use of the substrates is determined by the desired composition and the desired properties of the polymer core.

Polymer particles with a different composition of the polymers forming them exhibit different mechanical properties and release biologically active substances, in particular pharmaceutical active ingredients, at different rates. For example, polymer particles composed of C6-C 14 3-hydroxy fatty acids exhibit a higher rate of polymer degradation due to the low crystallinity of the polymer. An increase in the molar ratio of polymer constituents with relatively large side chains on the polymer backbone usually reduces crystallinity and results in more pronounced elastomeric properties. By controlling polymer composition in accordance with the process described in the invention, it is accordingly possible to influence the biodegradability of the polymer particles and thus also the release rate for biologically active substances, in particular pharmaceutically active or skin-care ingredients.

In order to achieve still more accurate control of the size of the polymer core formed, the substrate may be added to the culture medium in a quantity such that it is sufficient to ensure control of the size of the polymer core. This yields an additional possibility for exerting still more effective control over particle size.

The substrate or the substrate mixture may comprise at least one optionally substituted amino acid, lactate, ester or saturated or unsaturated fatty acid, preferably acetyl-CoA.

The polymer particle may comprise a polymer selected from poly-beta-amino acids, polylactates, polythioesters and polyesters. Most preferably the polymer comprises polyhydroxyalkanoate (PHA), preferably poly(3-hydroxybutyrate) (PHB).

In one embodiment a label or a labeled substrate is provided in the substrate mixture so that the label is incorporated into the polymer particle during polymer particle formation, or is allowed to diffuse into the polymer particle. Preferably the label is a coloured or fluorescent molecule, a radioisotope, or one or more metal ions. Preferably the labeled substrate in an amino acid, lactate, ester or saturated or unsaturated fatty acid, preferably acetyl-CoA.

The use of reporter molecules such as tags, dyes or labels to identify components of interest is well known in the art (Mitsopoulos G et al., 2004).

In one embodiment the particle forming protein is able to form polymer particles by catalysing the formation of a polymer particle directly by polymerising a substrate or a derivative of a substrate. Examples of such particle forming proteins include polymer synthases, particularly PHA polymer synthases. Alternatively, the particle forming protein is able to form polymer particles by facilitating the formation of a polymer particle by facilitating polymerisation, for example. Particle forming proteins that are able to facilitate polymerisation include phasins.

The action of a fusion polypeptide comprising a particle forming protein useful herein results in the formation of a polymer particle such as that shown in Figure 1. Figure 1 shows a schematic overview of an *in vivo* produced polymer particle comprising a polyester core encapsulated by a phospholipid monolayer, and the proteins and lipids which are associated with the particle, bound either to the core or the phospholipid monolayer, or both. Polymer particles produced for use in the invention may have one or more of the features identified in Figure 1.

The additional particle forming protein can be any protein that is capable of influencing the metabolism leading to the formation of the polymer particle. Figure 2 shows an example of synthesis of a polymer particle in *R. eutropha* (recently renamed to *Cupriavidus necator).* The polyhydroxy alkanoate polyhydroxybutyric acid (PHB) is produced in a three-stage process starting from the substrate acetyl CoA. The C4 repeat unit in PHB is β-hydroxybutyric acid. The final step in the synthesis results in the formation of a polymer particle with the particle forming proteins bound to the surface thereof, preferably the outer surface of the polymer core or the phospholipid monolayer.

A nucleic acid sequence encoding a particle forming protein can be selected such that it codes for a thiolase, a reductase or a polymer synthase. A polymer synthase is taken to be any protein which is capable of catalysing the final step for formation of a polymer. Apart from the polymer synthases described in the present invention, formation of the polymer may, for example, also be undertaken by a lipase.

In one embodiment the additional particle forming protein is derived from the family of phasin-like proteins and is preferably selected from the group comprising the phasin gene phaP from *Ralstonia eutropha* and the phasin gene phaF from *Pseudomonas oleovorans.* Phasins are amphiphilic proteins with a molecular weight of 14 to 28 kDa which bind tightly to the hydrophobic surface of the polymer particles.

In one embodiment the particle forming protein is a PHA polymer synthase, selected from the polymer synthase is selected from the group comprising a polymer synthase from *R. eutropha, P. oleovorans, P. putida, P. aeruginosa, Aeromonas punctata* or *Thiocapsa pfennigii.*

In one embodiment the particle binding domain is a particle binding domain selected from the particle binding domains of the particle forming proteins described above.

The expression constructs can be transfected into a host cell.

Preferably the host cell is a bacterial cell, a fungi cell, yeast cell, a plant cell, an insect cell or an animal cell, preferably an isolated or non-human host cell.

Suitable prokaryote host cells comprise eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia,* e.g., *E. coli, Enterobacter, Erwinia, Klebsiella, Proteus, Salmonella, e.g., Salmonella typhiniurium, Serratia, e.g., Serratia marcescans,* and *Shigella,* as well as *Bacilli* such as *B. subtilis* and *B. licheniformis, Pseudomonas* such as *P. aeruginosa,* and Actinomycetes such as *Streptomyces, Rhodococcus, Corynebacterium* and *Mycobaterium.*

In some embodiments *E. coli* strain W3110 may be used because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA ; E. coli* W3110 strain 9E4, which has the complete genotype *tonA ptr3; E. coli* W3110 strain 27C7 (ATCC 55,244), which has the complete genotype *tonA ptr3 phoA E15 (argF-lac)169 degP ompT kan*$^r$; *E. coli* W3110 strain 37D6, which has the complete genotype *tonA ptr3 phoA E15 (argF-lac)169 degP ompT rbs7 ilvG kan*$^r$; *E. coli* W3110 strain 40B4, which is strain 37D6 with a non-kanamycin resistant *degP* deletion mutation.

In addition to prokaryotes, eukaryotic microbes such as filamentous fungi or yeast are suitable cloning or expression hosts for use in the methods of the invention. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9: 968-975 (1991)) such as, e.g., *K. lactis* (MW98-8C, CBS683, CBS4574; Louvencourt et al., J. Bacteriol., 154(2):737-742 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans,* and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces* such as *Schwanniomyces occidentalis* (EP 394,538 published 31 October 1990); and filamentous fungi such as, e.g., *Neurospora, Penicillium, Tolypocladium* (WO 91/00357 published 10 January 1991), and *Aspergillus* hosts such as *A. nidulans* (Ballance et al., Biochem. Biophys. Res. Commun., 112:284-289 [1983]; Tilburn et al., Gene, 26:205-221 [1983]; Yelton et al., Proc. Natl. Acad. Sci. USA, 81: 1470-1474 [1984]) and *A. niger* (Kelly and Hynes, EMBO J., 4:475-479 [1985]). Methylotropic yeasts are suitable herein and comprise yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Examples of invertebrate host cells include insect cells such as Drosophila S2 and Spodoptera Sf9, as well as plant cells, such as cell cultures of cotton, corn, potato, soybean, petunia, tomato, and tobacco. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* have been identified. A variety of viral strains for transfection are publicly available, e.g., the L-1 variant of *Autographa californica* NPV and the Bm-5 strain *of Bombyx mori* NPV, and such viruses may be used as the virus herein according to the present invention, particularly for transfection of *Spodoptera frugiperda* cells.

Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:69 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

In one embodiment the host cell is a cell with an oxidising cytosol, for example the *E. coli* Origami strain (Novagen).

In another embodiment the host cell is a cell with a reducing cytosol, preferably *E. coli.*

The host cell is preferably selected from the genera comprising *Ralstonia, Acaligenes, Pseudomonas* and *Halobiforma.* Preferably the microorganism used is selected from the group comprising *Ralstonia eutropha, Alcaligenes latus, Escherichia coli, Pseudomonas fragi, Pseudomonas putida, Pseudomonas oleovorans, Pseudomonas aeruginosa, Pseudomonas fluorescens,* and *Halobiforma haloterrestris.* This group comprises both microorganisms which are naturally capable of producing biocompatible, biodegradable particles and microorganisms, such as for example *E. coli,* which, due to their genetic makeup, are not capable of so doing. The genes required to enable the latter-stated microorganisms to produce the particles are introduced as described above.

Extremely halophilic archaea produce polymer particles with lower levels of unspecific binding of protein, allowing easier isolation and purification of the particles from the cells.

Purposeful selection of the at least one further nucleic acid sequence which codes for a particle forming protein also makes it possible to influence the subsequent composition of the polymer particles. Genes that code for proteins involved in the metabolic pathway towards formation of the polymer particles may have different substrate specificities, form different reaction products or block branches in the metabolic pathway in order to exert a purposeful influence on the substrates and molecules involved in the formation of the polymer particles.

In principle, any culturable host cell may be used for the production of polymer particles by means of the above-described process, even if the host cell cannot produce the substrates required to form the polymer particles due to a different metabolism. In such cases, the necessary substrates are added to the culture medium and are then converted into polymer core by the proteins which have been expressed by the genes which have been introduced into the cell.

The genes required to enable the latter-stated host cells to produce the polymer particles include a thiolase, a reductase or a polymer synthase, such as phaA thiolase, phaB ketoacyl reductase or phaC synthase from *Ralstonia eutropha.* Figure 2 shows an example of synthesis of a polymer particle in *R. eutropha* and the genes required to form the substrates necessary for polymer particle formation. Which genes are required to augment what the host cell lacks for polymer particle formation will be dependent on the genetic makeup of the host cell and which substrates are provided in the culture medium.

At a minimum, a synthase alone can be used in any host cell with (R)-Hydroxyacyl-CoA or other CoA thioester or derivatives thereof as a substrate.

The polymer particle can also be formed *in vitro.* Preferably a cell free expression system is used. In order to produce an environment to allow particle formation *in vitro* the necessary substrates for polymer particle formation should be included in the media.

The particle forming protein can be used for the *in vitro* production of functionalised polymer particles using (R)-Hydroxyacyl-CoA or other CoA thioester as a substrate.

The fusion polypeptides can be purified from lysed cells using a cell sorter, centrifugation, filtration or affinity chromatography prior to use in *in vitro* polymer particle production.

*In-vitro* polymer particle formation enables optimum control of surface composition, including the level of fusion polypeptide coverage, phospholipid composition and so forth.

In one embodiment, a desirable characteristic of the polymer core is that it is persistent. The term "persistent" refers to the ability of the polymer core to resist degradation in a selected environment. An additional desirable characteristic of the polymer core is that it is formed from the particle forming protein and binds to the C- or N-terminal of the particle forming protein during particle assembly.

The polymer particle preferably comprises a phospholipid monolayer that encapsulates the polymer core. Preferably said particle forming protein spans said lipid monolayer.

The particle forming protein is preferably bound to the polymer core or to the phospholipid monolayer or is bound to both.

The particle forming protein is preferably covalently or non-covalently bound to the polymer particle it forms.

Preferably at least about 1%, 2%, 3%, 4%, 5%, 10%, 15%, 20%, 23%, 30%, 35%, 40%, 45%, 50%, 55%, 60%,

65%, 70%, 75%, 80%, 85%, 90%, 95%, 99% or 100% of the surface area of the polymer core is covered by fusion polypeptides.

In natural systems particle forming proteins have been formed to cover less than 1% of the polymer particle surface. In the present invention fusion polypeptides with at least recombinant polypeptide bound to the N- or C-terminus of the particle forming protein cover at least 10%, preferably about 25% or more of the polymer particle surface.

The polymer particles may have a diameter between about 10 nm to about 3 $\mu$m, between about 10 nm to about 900 nm, or between about 10 nm to about 110 nm.

In some embodiments it is desirable to produce many small polymer particles.

Accordingly, one aspect of the present disclosure relates to a process for producing polymer particles, the process comprising:

A) providing a cell comprising at least one expression construct under the control of a strong promoter, the expression construct comprising:

(1) at least one nucleic acid sequence that codes for a polymer synthase, the polymer synthase comprising a polymer particle binding domain; or

(2) at least one nucleic acid sequence that codes for a fusion polypeptide, the fusion polypeptide comprising a polymer synthase and at least one fusion partner the polymer synthase comprising a polymer particle binding domain; and

(3) optionally

(a) at least one nucleic acid sequence that codes for a particle forming protein, the protein comprising a polymer particle binding domain, or

(b) at least one nucleic acid sequence that codes for an additional fusion polypeptide, the additional fusion polypeptide comprising:

(i) a polymer particle binding domain, a protein that comprises a polymer particle binding domain, a particle forming protein, or a combination thereof, and

(ii) a fusion partner comprising at least one polypeptide or at least one binding domain or one or more coupling reagents or a combination thereof, or

(iii) a fusion partner comprising at least one polypeptide and at least one binding domain or one or more coupling reagents or a combination thereof, or

(iv) at least one reporter peptide or affinity purification peptide, or

(c) any combination of two or more thereof;

B) cultivating the cell under conditions suitable for expression of the expression construct and for formation of polymer particle by the polymer synthase, wherein the polymer synthase remains associated with the particle it forms; and

C) separating the polymer particles from the cultivated cells to produce a composition comprising polymer particles.

In preferred embodiments the promoter is the T7 promoter.

In some preferred embodiments the polymer particles have a diameter below about 300 nm, below about 200 nm, below about 150 nm, or below about 105 nm.

The methods of production of the invention allow production of compositions comprising a greater proportion of smaller particles than are produced by wild type organisms or by known particle producing host cells. For example, the methods of the invention allow production of a composition of particles wherein 90% of the particles in the composition have a diameter of between about 10 nm to about 200 nm, preferably:

(a) 80 % of the particles in the composition have a diameter of between about 10 nm to about 150 nm;

(b) 60 % of the particles in the composition have a diameter of between about 10 nm to about 100 nm;

(c) 45 % of the particles in the composition have a diameter of between about 10 nm to about 80 nm;

(d) 40 % of the particles in the composition have a diameter of between about 10 nm to about 60 nm;

(e) 25 % of the particles in the composition have a diameter of between about 10 nm to about 50 nm; or

(f) 5 % of the particles in the composition have a diameter of between about 10 nm to about 35 nm.

In one embodiment the host cell comprises at least about 20, 25, 30, 35, 40, 45, 50, 55, 60, 65, 70, 75, or 80 polymer particles per cell.

In one embodiment the fusion partner may comprise a polypeptide such as a protein, a protein fragment, a binding domain, a target-binding domain, a binding protein, a binding protein fragment, an antibody, an antibody fragment, an antibody heavy chain, an antibody light chain, a single chain antibody, a single-domain antibody (a VHH for example), a Fab antibody fragment, an Fc antibody fragment, an Fv antibody fragment, a F(ab')2 antibody fragment, a Fab' antibody fragment, a single-chain Fv (scFv) antibody fragment, an antibody binding domain (a ZZ domain for example), an antigen, an antigenic determinant, an epitope, a hapten, an immunogen, an immunogen fragment, biotin, a biotin derivative, an avidin, a streptavidin, a substrate, an enzyme, an abzyme, a co-factor, a receptor, a receptor fragment, a receptor subunit, a receptor subunit fragment, a ligand, an inhibitor, a hormone, a lectin, a polyhistidine, a coupling domain, a DNA binding domain, a FLAG epitope, a cysteine residue, a library peptide, a reporter peptide, an affinity purification peptide, or any combination of any two or more thereof.

Single chain antibody fragments (scFv) have previously been used as ligands in a number of bioseparation applications. In addition to their specificity and ability to be reproducibly produced, the smaller size of such fragments as compared to whole antibodies may reduce non-specific binding of contaminants. ScFvs have been used to separate a large number of target components including fractionating enantiomers in racemic mixtures and removal of micro-organisms from food and water samples.

The reducing environment of the cytoplasm of cells impairs the formation of the intradomain disulphide bonds essential for the correct folding and functionality of single-chain antibody (ScFv) fragments. Accordingly, most ScFvs expressed in the cytoplasm (intrabodies) are mostly inactive. However some intrabodies (e.g. scFv(F8)) have been isolated that can be functionally produced in the cytosol and that have been successfully used as scaffold to graft certain binding affinities such as anti-hen egg lysozyme (Donini et al., 2003, Journal of Molecular Biology, 330:323-332) Furthermore, cysteine-free intrabodies have been engineered to overcome this difficulty (Woern and Plueckthun, 1998, FEBS Letters, 427:357-361).

An additional approach to stabilising pre-screened intrabodies is to express them as fusion polypeptides. Disulphide-bond containing ScFvs have been shown to be expressed in a soluble and functional form with the bacterial cytoplasm when fused to Maltose-binding Protein, a cytoplasmic protein of *E. coli* (Bach *et al* (2001), Shaki-Loewenstein et al., 2005, Journal of Immunological Methods, 2005, in press). Additionally, host cell strains with an oxidising cytosol may be used to express the fusion polypeptides.

It is also conceivable to engineer functional VHH intrabodies, which can be fused to the particle forming protein.

Intracellular binding of intrabodies to a LacZ (beta-galactosidase) dimer mediated the formation of the active tetrameric LacZ which activity can be easily detected. This was used as a screening tool for functional intrabodies after random mutagenesis (Martineau et al., 1998, Journal of Molecular Biology 280:117-127). Thus similar screening strategies can be envisaged leading to any functional intrabodies which can ultimately be fused to the particle forming protein enabling attachment of the respective intrabody to the particle.

Fusion polypeptides comprising amino acid sequences of a wide number of intrabodies can be used in the present invention.

During modification of the genes which code for proteins which, once expressed, bind to the particle surface, it is also possible to introduce constructs with different modifications into the cell. Once these fusion polypeptides with their different polypeptides of fragments thereof have been expressed and the polymer particles have been formed, it is possible in this manner to use the different fusion polypeptides to multifunctionalise the particle surface. This process enables straightforward and efficient mass production of functionalised polymer particles.

In one embodiment the process further comprises:

(1) binding a coupling reagent to the fusion partner binding domain.

In another embodiment the process further comprises

(1) binding a coupling reagent to the fusion partner binding domain and

(2) binding at least one substance to the coupling reagent.

Coupling reagents can be used for the subsequent functionalisation of fusion polypeptides bound on the surface of the polymer particles, these coupling reagents preferably being selected from the group comprising bis(2-oxo-3-oxazolydinyl)phosphonic chloride (BOP-Cl), bromotrispyrrolidinophosphonium hexafluorophosphate (PyBroP), benzotriazol-1-yl-oxy-trispyrrolidinophosphonium hexafluorophosphate (PyBOP), n-hydroxysuccinimide biotin, 2-(1H-benzotriazol-1-yl)-1,1,3,3-tetramethyluronium hexafluorophosphate (HBTU), dicyclohexylcarbodiimide, disuccinimidyl carbonate, 1-(3-dimethylaminopropyl)-3-ethylcarbodiimide (EDC), bis(2-oxo-3-oxazolydinyl)phosphine, diisopropylcarbodiimide (DIPC), 2-(1H-benzotrioxazolyl)-1,1,3,3-tetramethyluronium tetrafluoroborate (TBTU), 2-(5-norbornene-2,3-dicarboxyimido)-1,1,3,3-tetramethyluronium tetrafluoroborate (TNTU), para-nitrophenylchloroformate, and O-(n-succinimidyl)-1,1,3,3-tetramethyluronium tetrafluoroborate (TSTU).

Once formed, the polymer particles can be separated from the host cell by disrupting the cell and recovering the particles, preferably by physical disruption of the cell followed by separation using a cell sorter, centrifugation, filtration or affinity chromatography.

Purification of particles might employ the addition (chemically or by fusion technology) of molecules to the surface enabling affinity purification and/or adsorption to surfaces also for screening purposes.

## 5. Functionalised Polymer Particles

It has been discovered that polyhydroxyalkyl polymer particles can be stably maintained as particles outside the host cell that produced them, and that these particles can be designed to suit a number of applications.

Functionalised polymer particles may comprise one or more surface-bound fusion polypeptides, one or more substances incorporated or adsorbed into the polymer particle core, one or more substances bound to surface bound fusion polypeptides, or a combination thereof.

In one embodiment a substance may be immobilised on the particle surface during particle formation, bound to a fusion partner or coupling reagent, or integrated into the particle by loading, diffusion or incorporation.

In one embodiment the substance is selected from the list comprising a protein or protein fragment, a peptide, a polypeptide, an antibody or antibody fragment, an antibody binding domain, an antigen, an antigenic determinant, an epitope, an immunogen or fragment thereof, a metal ion, a metal ion-coated molecule, biotin, avidin, streptavidin or derivatives thereof, an inhibitor, a co-factor, a substrate, an enzyme, a co-factor, a receptor, receptor subunit or fragment thereof, a ligand, an inhibitor, a monosaccharide, an oligosaccharide, a polysaccharide, a glycoprotein, a lipid, a cell or fragment thereof, a cell extract, a virus, a hormone, a serum protein, a milk protein, a macromolecule, a drug of abuse, or any combination of any two or more thereof.

In one embodiment at least one antibody antigen, antigenic determinant, epitope, or immunogen of fragment thereof may be immobilised on the surface of the polymer particles or integrated into the polymer particle.

In one embodiment the fusion partner comprises a metal ion binding domain that binds a metal ion or a metal ion-coated molecule and the resulting particles used as medical imaging reagents.

In one embodiment DNA from an identified infectious agent can be fragmented and inserted into expression constructs encoding fusion polypeptides that comprise a polymer particle binding domain. In this way, polymer particles displaying antigenic determinants can be produced and screened using serum from infected patients and antigen-presenting particles identified, isolated and reproduced using well-known and scalable bacterial production systems.

In one embodiment multiple antigens may be immobilised on the surface of the polymer particles.

In other embodiments the substance may be a pharmaceutical agent selected from the list comprising alpha-galactoceramide, dideoxyinosine, floxuridine, 6-mercaptopurine, doxorubicin, daunorubicin, 1-darubicin, cisplatin, methotrexate, taxol, antibiotics, anticoagulants, germicides, antiarrhythmic agents and active ingredient precursors or derivatives thereof, or proteins selected from the list comprising insulin, calcitonin, ACTH, glucagons, somatostatin, somatotropin, somatomedin, parathyroid hormone, erythropoietin, hypothalamic release factors, prolactin, thyroid-stimulating hormone, endophins, enkephalins, vasopressins, non-naturally occurring opiates, superoxide dismutase, antibodies, interferons, asparaginase, arginase, arginine deaminase, adenosine deaminase, ribonuclease, trypsin, chymotrypsin or pepsin, the particles having application in drug delivery.

In other embodiments the polymer particles can be used to deliver substances useful in cleaning applications, by expressing one or more functional enzymes at high density on the particle surface or by loading the particles with substances.

In one embodiment one or more enzymes may be immobilised to the particle to provide stability in environments of harsh pH or temperature and in storage, extending the functional life and enhancing robustness in complex 'dirty' environments.

In one embodiment particles displaying one or more enzymes have potential in laundry detergents.

In one embodiment the substance may be at least one enzyme selected from the list comprising enzyme selected from the list comprising cellulases, peroxidases, proteases, glucoamylases, amylases, lipases, cutinases, pectinases, reductases, oxidases, phenoloxidases, lipoxygenases, ligninases, pullulanases, tannases, pentosanases, malanases, β-glucanases, arabinosidases, racemases, hydrolases, dehydrogenases, polymerases, dioxygenases, monoxgenases, lyases, synthetases, epimerases, hydroxylases, transferases, transacylases and synthases.

In one embodiment particles there may be multiple different enzymes and surfactants on the particle surface.

Expressing one or more enzymes as fusion polypeptides may enable one-step production of functional immobilised enzymes without the need for complex extraction or refolding steps.

Such embodiments may also reduce the bulk of detergent. It is also envisaged such particles may result in improved performance of laundry detergents, for example by reducing bulk of the laundry detergent, due to the proximity of enzymes and surfactants on the particle surface, improved presentation and stability of enzymes and surfactants, and through the inclusion of targeting molecules to direct the particles to specific types of clothing or to specific types of stain or substance.

In one embodiment the ability to stably immobilise on or more enzymes on the particle surface may also have application in biocatalysis and bioremediation. Multiple enzymes can be immobilised on a single particle, facilitating multiple or multi-step enzymic conversions.

In another embodiment the substance may be an anti-redeposition agent selected from the list comprising methylcellulose, carboxymethylcellulose, hydroxyethylcellulose, polyacrylate polymers, copolymers of maleic anhydride and acrylic acid, copolymers of maleic anhydride and ethylene, copolymers of maleic anhydride and methylvinyl ether, copolymers of maleic anhydride and methacrylic acid, or any combination of any two or more thereof

In other embodiments the polymer particles can be used to deliver substances useful in skin care products.

In one embodiment the particles can carry a range of substances, simplying formulation and reducing manufacturing cost. Particles can also be designed to degrade at specific rates by altering the polymer particle composition, to provide controlled release of active molecules to the skin. In other embodiments, the particle size may enhance function, for example by improving penetration into the skin surface, or controlling skin contact surface area.

In one embodiment the substance may be a skin care active, selected from the list comprising sunscreen agents, particulate materials, conditioning agents, thickening agents, water-soluble vitamins, water-dispersible vitamins, oil-dispersible vitamins, emulsifying elastomers comprising dimethicone copolyol crosspolymers, non-emulsifying elastomers comprising dimethicone/vinyl dimethicone crosspolymers, oil-soluble skin care actives comprising oil-soluble terpene alcohols, phytosterols, anti-acne actives, beta-hydroxy acids, vitamin $B_3$ compounds, retinoids, anti-oxidants/radical scavengers, chelators, flavonoids, anti-inflammatory agents, anti-cellulite agents, topical anesthetics, antiperspirants and fragrances, or any combination of any two or more thereof.

In other embodiments the particles can be used to encapsulate substances such as flavours, vitamins, nutrients or bioactives, to improve shelf-life, flavour, and nutrient availability/content.

Potential applications include:

1) delivery of nutrients or bioactives to specific parts of the digestive system, for example drugs or bioactives to lower parts of the intensive to treat Crohn disease or ulcerative colitis;

2) biocatalytic uses in food processing, for example cheese making and enzyme stabilisation and/or recycling;

3) downregulation of food or other allergies by stimulating immunity at intestinal or other mucosal surfaces;

4) food packaging or coatings, for example edible coatings to improve shelflife, flavour or visual appearance;

4) emulsification of food ingredients for prolonged periods of time for storage;

5) keeping food ingredients from mixing until shaken or otherwise activated, for example to prevent an enzymatic reaction prevented until desired;

6) viscoactive modification of foods, to provide active delivery mechanism for drugs or nutrients; and

7) improvement of mouth-feel or acceptability of foodstuffs, for example the encapsulation of fish oils to enable addition of omega-3 fatty acids to foods without aftertaste.

In further embodiments the polymer particles may be used in further diagnostic applications for detecting and optionally isolating target components, in protein production and combinatorial screening.

**6. Detection and Optional Isolation of Target components**

In one aspect the invention relates to processes for the detection and optional isolation of at least one target component in a sample. In one embodiment the process comprises detecting and optionally isolating at least one target component in a sample comprising:

A) providing a polymer particle comprising at least one fusion polypeptide comprising a polymer particle binding domain and at least one fusion partner, the fusion partner comprising:

(1) at least one binding domain capable of binding one or more target components or one or more coupling reagents or a combination thereof, or

(2) at least one polypeptide, or

(3) a combination thereof, and
optionally

(a) at least one particle forming protein, the protein comprising a polymer particle binding domain, or

(b) at least one additional fusion polypeptide, the additional fusion polypeptide comprising:

(i) a polymer particle binding domain, a protein that comprises a polymer particle binding domain, a particle forming protein, or a combination thereof, and

(ii) a fusion partner comprising at least one polypeptide or at least one binding domain capable of binding one or more target components or one or more coupling reagents or a combination thereof, or

(iii) a fusion partner comprising at least one polypeptide and at least one binding domain capable of binding one or more target components or one or more coupling reagents or a combination thereof, or

(iv) at least one reporter peptide or affinity purification peptide, or

(c) any combination of two or more thereof;

(B) contacting the polymer particle with a sample comprising a target component such that the fusion partner binds the target component to form a complex,

(C) detecting the presence or absence of the target component, and

(D) optionally separating the polymer particles containing a bound target component from the sample

The use of reporter molecules such as tags, dyes or labels to identify components of interest is well known in the art (Mitsopoulos G et al., 2004).

The presence of the detectable label allows a polymer particle to be distinguished from a polymer particle that does not contain the label. The label may be incorporated into the polymer particle core, be coupled to a molecule that will bind directly or indirectly to the complex, or may be attached to the polymer particle as a reporter peptide.

In one embodiment the label is coupled to a molecule that binds to the complex. The labeled molecule may bind to any part of the complex, including the polymer core, an element of the phospholipid monolayer, an element of a fusion polypeptide, or to a target component bound to a fusion partner.

Detecting the presence of a label preferably includes measuring the intensity of the label, allowing a quantitative measure of the level of the target component in a sample to be calculated.

In one embodiment two or more labels may be used, allowing the simultaneous detection and quantification of multiple parameters.

Other aspects relate to systems for the detection and optional isolation of at least one target component in a sample. The systems comprise a polymer particle and at least one label, wherein the polymer particle comprises at least one fusion polypeptide and wherein the fusion polypeptide comprises at least one binding domain that will bind a target component, such that when the polymer particle is contacted with a sample comprising a target component the binding domain binds to the target component to form a complex, wherein the presence or absence of the label allows the detection of the presence or absence of the target component; and optional separation of the complex.

In one embodiment the target component may comprise a protein, a protein fragment, a peptide, a polypeptide, a polypeptide fragment, an antibody, an antibody fragment, an antibody binding domain, an antigen, an antigen fragment, an antigenic determinant, an epitope, a hapten, an immunogen, an immunogen fragment, a metal ion, a metal ion-coated molecule, biotin, a biotin derivative, avidin, streptavidin, an inhibitor, a co-factor, a substrate, an enzyme, an abzyme, a receptor, a receptor fragment, a receptor subunit, a receptor subunit fragment, a ligand, a receptor ligand, a receptor agonist, a receptor antagonist, a signalling molecule, a signalling protein, a signalling protein fragment, a growth factor, a growth factor fragment, a transcription factor, a transcription factor fragment, an inhibitor, a cytokine, a chemokine, an inflammatory mediator, a monosaccharide, an oligosaccharide, a polysaccharide, a glycoprotein, a lipid, a cell, a cell-surface protein, a cell-surface lipid, a cell-surface carbohydrate, a cell-surface glycoprotein, a cell extract, a virus, a virus coat protein, a hormone, a serum protein, a milk protein, a macromolecule, a drug of abuse, a coupling reagent, a polyhistidine, a pharmaceutically active agent, a biologically active agent, a label, a coupling reagent, a library peptide, an expression construct, a nucleic acid or a combination thereof.

In one embodiment the fusion partner encodes Myelin Oligodendrocyte Glycoprotein (MOG) or fragments thereof and the target component is an anti-Myelin Oligodendrocyte Glycoprotein (MOG) antibody or fragment thereof.

In one embodiment the fusion partner encodes an antibody or antibody fragment that will bind a target component related to Type 1 and Type 2 immune responses, apoptosis, and/or angiogenesis and the target component is selected from the group comprising Interleukin-3 (IL-3), Interleukin-4 (IL-4), Interleukin-5 (IL-5), Interleukin-10 (IL-10), Interleukin-7 (IL-7), Interleukin-1$\beta$ (IL-1$\beta$), Interleukin-6 (IL-6), Interleukin-12p70 (IL-12p70), Granulocyte Macrophage-Colony Stimulating Factor (GM-CSF), cleaved PARP, Bcl-2, and active Caspase-3 protein levels, Interleukin-8 (IL-8), basic Fibroblast Growth Factor (bFGF), Angiogenin (ANG), Vascular Endothelial Growth Factor (VEGF), and Tumor Necrosis Factor (TNF), Interleukin-8 (CXCL8/IL-8), RANTES (CCL5/RANTES), Monokine-induced by Interferon-$\gamma$ (CXCL9/MIG), Monocyte Chemoattractant Protein-1 (CCL2/MCP-1), and Interferon-$\gamma$-induced Protein-10 (CXCL10/IP-10).

Antibody binding domains can be used to bind antibodies to the surface of the polymer particles, creating functionalised particles that can be used in a variety of immnoseparation and immunodetection applications.

In one embodiment the fusion partner comprises is an antibody binding domain, preferably an IgG-binding domain such as Protein A comprising the 132 amino acid ZZ domain having the sequence set forth in amino acids 48 to 179 of SEQ ID NO:6 or amino acids 2 to 133 of SEQ ID NO:7.

In one embodiment other IgG binding proteins such as Protein G and Protein L may also be used. Protein A and Protein G exhibit high affinity for the Fc portion of subclasses of IgG from many species. Unlike Protein A and Protein G, Protein L binds to immunoglobulins through the kappa light chain and as a result binds to all subclasses of Human, Mouse and Rat IgG, but not Bovine IgG. Accordingly, Protein L is useful for affinity purification of antibodies from culture supernatants containing bovine serum and from the milk of transgenic animals. Protein L also does not interfere with the binding of an antigen to the antigen binding site and thus is suitable for immunoprecipitation and immunodetection.

In one embodiment the fusion partner encodes a receptor protein, a subtype of a receptor or a subunit of a receptor complex, or a fragment thereof and the target component is a receptor ligand.

In one embodiment the label is a detectable label such as a coloured dye, a fluorescent molecule such as a fluorophore or fluorochrome, a radioisotope; or one or more metal ions such that the incorporation or non-incorporation of such a label by a particle can be determined.

In one embodiment the labeled molecule is labeled by coupling the molecule to a fluorescent molecule known in the art, comprising but not limited to fluorescein isothiocyanate (FITC) which fluoresces at about 530nm, phycoerythrin (PE) (575nm), texas red (620 nm), phycoerythrin-texas red (615 nm), allophycocyanin (APC) (660 nm), propidium iodide (PI) (660 nm), phycoerythrin-cyanine dye (BD Cy-Chrome) (670 nm), peridinin chlorophyll protein (perCP) (675 nm), peridinin chlorophyll protein- cyanine dye (perCP-Cy5.5) (694 nm) and), allophycocyanin-cyanine dye (APC-Cy7) (767 nm).

In one embodiment the label is coupled to monoclonal antibodies that will bind directly to the complex.

In one embodiment a primary antibody is used that binds to the complex, followed by a label-coupled secondary antibody that will bind to the primary antibody.

In one embodiment a primary antibody is used that binds to the polymer particle or the target component, followed by a biotinylated secondary antibody. A streptavidin-coupled label is then used that will bind to the secondary antibody.

Figure 21 shows a schematic view of the microbial production of antigen displaying PHA granules, their use in binding antigen-specific antibodies followed by detection using labeled secondary antibodies.

In one embodiment a label or a labeled substrate is provided in the media so that the label is incorporated into the polymer particle during polymer particle formation, or is allowed to diffuse into a polymer particle. An example of dye incorporation is given in WO 2004020623 (Bernd Rehm) which is incorporated by reference.

In one embodiment the polymer particle is labeled with a discrete level of fluorescent dye to allow it to be distinguished from other sets of polymer particles by its mean fluorescence intensity (MFI) upon flow cytometric analysis.

In one embodiment polymer particles may be immobilised on s solid substrate, preferably on a plate such as an ELISA plate or microarray, on a bead such as a polystyrene bead, in immunotubes or in a suitable chromatography matrix.

In other embodiments the polymer particles can be contacted with the sample in solution, and the bound target component detected using chromatography. For example, a labeled complex can be used as part of a diagnostic test strip, the particle complexes travelling along the strip until they contact a test area and provide a visible signal. Diagnostic immunostrips are well known in the art.

**Immunosorbent Assay Detection**

Enzyme-linked Immunosorbent Assays (ELISA) can be used for the detection and quantification of the labeled particle complex. ELISAs have well-established protocols in the art for the measurement of target components in solutions.

ELISA techniques used for the detection and quantification of the labeled particle complex include any of a number of well known enzyme-linked immunoassays. Examples of such systems are well known in the art. The assay techniques are based upon the formation of a complex between a complementary binding pair, followed by detection with a detection system comprising an enzyme-conjugate label and a chromogenic or fluorogenic substrate.

In one embodiment the ELISA is in the "sandwich" assay format. In this format the target analyte to be measured is bound between two antibodies - the capture antibody and the detection antibody. The binding domain may form the capture antibody or a capture antibody may be bound to the binding domain.

In another embodiment the ELISA is a competitive assay, where a labelled antigen is used instead of a labelled antibody. Unlabelled antigen and the labelled antigen compete for binding to the capture antibody and the amount of target component bound can be determined by the proportion of labelled antigen detected.

Either monoclonal or polyclonal antibodies may be used as the capture and detection antibodies in sandwich ELISA systems. Monoclonal antibodies have an inherent monospecificity toward a single epitope that allows fine detection and quantitation of small differences in antigen. A polyclonal antibody can also be used as the capture antibody to bind as much of the antigen as possible, followed by the use of a monoclonal antibody as the detecting antibody in the sandwich assay to provide improved specificity.

It should be understood that such immunoassay techniques are not restricted to the use of antibodies but are equally applicable to any colourimetric or fluorometric assay.

**FACS detection**

In one embodiment the detection system utilises cell sorting (for example via FACS) to detect and quantify labeled polymer particle complexes and thus the bound target components.

Flow cytometry can be used to separate and simultaneously characterise polymer particle complexes on the basis of any of a number of pre-selected properties. Measurable properties include size, volume, viscosity, light scatter characteristics, content of DNA or RNA and surface antigens. The polymer particles useful and complexes formed in the methods of the invention are separable on the basis of any one or more of these measurable properties. Flow cytometry techniques are well known in the art.

In one embodiment a mixed population of polymer particles and/or polymer particles displaying a mixed population of fusion polypeptides can be used to screen a sample for a plurality of different target components. Such polymer particles can be used in multiplex analyses, to analyse networks of biological response modifiers (BRMs) that are co-expressed by cells that mediate immune and inflammatory responses. BRMs such as cytokines, chemokines, inflammatory mediators, receptors and immunoglobulins associated with illness or disease may be targeted. The use of fluorescence-activated cell sorting (FACS) in muliplex analyses using polymer particles of the invention allows detecting and optionally isolating antigen-specific and quantitative levels of target components in a sample.

**Reporter Peptide Detection**

In one embodiment an amino acid sequence encoding a reporter peptide is fused to the particle forming protein in addition to the binding domain. Alternatively, a reporter peptide is provided as part of a separate fusion polypeptide.

The reporter peptide is itself detectable or will catalyse production of a detectable product. Reporter peptides useful herein include lacZ, luciferase, alkaline phosphatases, peroxidases, or green fluorescent protein (GFP).

Reporter peptides are those that allow polymer particles containing the reporter peptides to be distinguished from polymer particles that do not contain the reporter peptide. Reporter gene expression is generally easily monitored, since in many cases, the cellular phenotype is altered; either due to the presence of a detectable alteration, such as the presence of a fluorescent protein (which, as outlined herein, includes both the use of fusions to the detectable gene itself, or the use of detectable gene constructs that rely on the presence of a protein to be activated), by the addition of a substrate altered by the reporter peptide (e.g., chromogenic (including fluorogenic) substrates for reporter enzymes such as luciferase, 3-galactosidase, etc.), or, for example, by conferring a drug resistive phenotype (e.g., using DHFR with methotrexate selection).

Reporter peptides generally fall into one of several classes, including detection genes, indirectly detectable genes, survival genes, etc. That is, by inserting a polypeptide into a gene that is detectable, for example GFP or luciferase, the expression of the peptide may be monitored. Similarly, the insertion of a gene into a survival gene, such as an antibiotic resistance gene, allows detection of the expression of the peptide via survival of the cells.

Reporter peptides fall into several classes, as outlined above, including, but not limited to, detection genes, indirectly detectable genes, and survival genes.

In a preferred embodiment, the reporter peptide is a detectable protein. A "detectable protein" or "detection protein" (encoded by a detectable or detection gene) is a protein that can be used as a direct label; that is, the protein is detectable (and preferably, a particle comprising the detectable protein is detectable) without further manipulations or constructs. Thus, in this embodiment, the protein product of the reporter peptide itself can serve to distinguish particles that are expressing the detectable gene. In this embodiment, suitable detectable genes include those encoding auto-fluorescent proteins.

In a preferred embodiment, the reporter peptide is Aequorea green fluorescent protein or one of its variants; see Cody et al., (1993); and Inouye and Tsuji (1994).

"Green fluorescent protein" or "GFP" herein is meant an auto-fluorescent protein that generally exhibits fluorescence emission at 400 to 700 nm. The wild-type Aequorea GFP is 238 amino acids in length, contains a modified tripeptide fluorophore buried inside a relatively rigid 3-can structure which protects the fluorophore from the solvent, and thus solvent quenching (Cody et al. Biochemistry 1993;32(5):1212-8; Ormo et al., Science 1996;273(5280)1392-6; Prasher et al., Gene 1992;111(2):229-33).

Other suitable detectable proteins include, among others, lacZ (Uppala and Koivunen 2000; Arndt et al., 2000), luciferases (for example, firefly, Kennedy et al. (Journal of Biological Chemistry 1999; 274:13281-91.); Renilla mueller U.S. Pat. No. 6,232,107), 3-galactosidase (Nolan et al. Proceedings of the National Academy of Sciences 1988;85: 2603-7), 3-glucouronidase (Jefferson et al. EMBO Journal 1987;6(13):3901-7), horseradish peroxidase, alkaline phosphatase, and SEAP (e.g., the secreted form of human placental alkaline phosphatase; Cullen et al. (Methods in Enzymology 1992;216:362-8)).

In one embodiment said reporter peptide forms part of the fusion polypeptide in conjunction with the particle forming protein and the binding domain. In another embodiment the reporter peptide may be used in combination with a labeled molecule.

Accordingly, the present invention also relates to the use of at least one polymer particle in the detection and optional isolation of at least one target component in a sample, said polymer particle comprising at least one fusion polypeptide that comprises at least one binding domain that will bind a target component.

An alternative embodiment of the invention provides a process for screening a plurality of different target components in parallel for their ability to interact with a particular binding domain, comprising the following steps: contacting different fluid samples each containing at least one of the different target components with a mixed population of polymer particles, preferably wherein the particular target component is immobilised; and detecting, either directly or indirectly, the interaction of the particular target component with a binding domain using a label or a labeled substrate.

The present invention also relates to kits for use in the detection and optional isolation of at least one target component in a sample, wherein the kits facilitate the employment of the processes and systems of the present invention.

Preferably, kits for carrying out a method of the invention contain all the necessary reagents to carry out the process. The kits preferably comprise one or more containers, containing for example, wash reagents, and/or other reagents capable of detecting the presence or absence of a detectable label.

In the context of the present invention, a compartmentalised kit includes any kit in which reagents are contained in separate containers, and may include small glass containers, plastic containers or strips of plastic or paper. Such containers may allow the efficient transfer of reagents from one compartment to another compartment whilst avoiding cross-contamination of the samples and reagents, and the addition of agents or solutions of each container from one compartment to another in a quantitative fashion. Such kits may also include a container which will accept a test sample, a container which contains the polymers used in the assay and containers which contain wash reagents (such as phosphate buffered saline, Tris-buffers, and like).

Preferably, a kit of the present invention will also include instructions for using the kit components to conduct the appropriate processes.

In one embodiment the present invention comprises optionally isolating at least one polymer particle that binds a target component from those polymer particles that do not. The polymer particle that is isolated comprises a binding domain that binds the target component.

In one embodiment polymer particles comprising at least one binding domain bound to at least one target component are isolated using a number of separation techniques known in the art.

In one embodiment the system is used to isolate at least one desired target component from a mixture. However, in alternative embodiments the system may be used to remove at least one undesired target component from a sample.

**7. Production of recombinant polypeptides**

As discussed above, the present invention relates to processes of producing recombinant polypeptides that form inclusion bodies when expressed in cellular expression systems.

In one embodiment the invention relates to a process for producing recombinant polypeptides, the process comprising:

A) providing a cell comprising at least one expression construct comprising:

(1) at least one nucleic acid sequence that codes for a fusion polypeptide, the fusion polypeptide comprising a polymer particle binding domain and at least one polypeptide that forms inclusion bodies when expressed in a cellular expression system; and

(2) optionally

(a) at least one nucleic acid sequence that codes for a particle forming protein, the protein comprising a polymer particle binding domain, or

(b) at least one nucleic acid sequence that codes for an additional fusion polypeptide, the additional fusion polypeptide comprising:

(i) a polymer particle binding domain, a protein that comprises a polymer particle binding domain, a particle forming protein, or a combination thereof, and

(ii) a fusion partner comprising at least one polypeptide or at least one binding domain or one or more coupling reagents or a combination thereof, or

(iii) a fusion partner comprising at least one polypeptide and at least one binding domain or one or more coupling reagents or a combination thereof, or

(iv) at least one reporter peptide or affinity purification peptide, or

(c) a combination thereof;

B) cultivating the cell under conditions suitable for expression of the fusion polypeptide and for formation of polymer particle by the host cell; and

C) optionally separating the recombinant polypeptides from the cultivated cells.

The formation of inclusion bodies is a frequent consequence of high expression recombinant protein production in cellular expression systems, where quantitative production of the recombinant proteins is strongly impaired by the aggregation of unfolded or partially-folded full or partial length polypeptides rather than correctly folded, native protein.

A number of factors are thought to promote the formation of inclusion bodies during recombinant polypeptide production.

Wilkinson & Harrison (Nature Bio/Technology 9, 443 - 448 (1991)) studied the cause of inclusion body formation in *E. coli* grown at 37°C using statistical analysis of the composition of 81 proteins that do and do not form inclusion bodies. Six composition derived parameters were investigated. In declining order of their correlation with inclusion body formation, the parameters include charge average, turn forming residue fraction, cysteine fraction, proline fraction, hydrophilicity, and total number of residues. Recombinant polypeptides produced in a method of the invention may comprise one or more of these parameters.

Accordingly, many hydrophobic membrane proteins, multidomain proteins and proteins harbouring disulphide bridges are likely to form inclusion bodies in cellular expression systems. Overproduction by itself (the increase in the concentration of the nascent polypeptide chains) can also be sufficient to induce the formation of inclusion bodies.

Solubilization tags (for example NusA, MBP etc.), co-production of chaperones (for example DnaK, GroEL etc.) and/or co-chaperones (for example DnaJ, GrpE, ClpB etc.) and/or disulphide isomerases, secretion to the periplasm or outside the cell, adjustment of production temperature, gene expression control, in vitro gene expression as well as *E.*

*coli* mutants (Origami) providing an oxidative cytosolic background have been employed to overcome inclusion body formation in cellular expression systems.

For example, vector plasmids are tentatively divided into four classes based on their copy number (the copy number is defined as the number of plasmid copies per chromosome): very high-copy-number vectors are present in more than 100 copies per chromosome (pUC vectors), high-copy-number vectors (15-60 copies; pBR322), medium-copy-number vectors (about 10 copies; pACYC177, pACYC184 and pSC101) and low-copy-number vectors (1-2 copies; mini-F). In some cases the use of medium-copy-number vectors can reduce the amount of recombinant protein sufficiently to prevent their aggregation. Alternatively, high-copy-number vectors can be used in combination with a weak promoter such as the wild-type lac promoter.

If these conditions and factors do not enable significant production of functional protein, proteins are obtained from inclusion bodies via refolding and can be clearly categorised as "difficult folders". If refolding is not successful, the protein has then to be isolated from its original host by classical protein purification protocols, which do usually not lead to high yield.

In contrast, the processes of the present invention do not limit recombinant protein production to low levels of expression or the refolding of proteins from inclusion bodies.

In one embodiment the polypeptide is selected by conducting a literature search to identify a polypeptide that has previously been determined to form inclusion bodies when expressed in a cellular expression system. A number of literature databases are publicly accessible, such as the NCBI Entrez PubMed database (http://www.ncbi.nlm.nih.gov/) which includes over 15 million citations from MEDLINE and other life science journals for biomedical articles dating from the 1950s. Such databases can be searched by keyword, topic, author or journal to identify potential polypeptides and include links to full text articles and other related resources.

In one embodiment the polypeptide is selected by expressing a candidate polypeptide in a host cell that can not form polymer particles and examining the cell microscopically to determine whether or not the expressed polypeptide forms inclusion bodies.

Expressing candidate polypeptides can be carried out as described above by transforming a host cell with an expression construct comprising a a promoter functional in the host cell into which the construct will be transformed, the nucleic acid sequence of the candidate polypeptide, and a terminator functional in the host cell into which the construct will be transformed. Following transformation, the transformed host cell is cultured under conditions suitable for expression of the candidate polypeptide from the expression construct and the host cells examined microscopically to determine the presence or absence of refractile inclusion bodies. Flow cytometry can also be used to provide information on inclusion body accumulation at the single-cell level that is not available from other methods. Wittrup et al (Nature Bio/Technology 6, 423 - 426 (1988)) describe the use of single-cell light scatter measurements from flow cytometry as a probe of refractile body formation in recombinant *E. coli.*

In one embodiment a statistical model for prediction of solubility on expression in *E. coli,* as defined by Wilkinson and Harrison [Nature Bio/Technology 9, 443 - 448 (1991)], can be used to select a recombinant polypeptide. This model has been primed on 81 proteins for which expression results are available. Discriminant analysis was used to compare these proteins according to six composition related parameters viz. - charge average, turn forming residue fraction, cysteine fraction, proline fraction, hydrophilicity and total number of amino acid residues. The relative number of turn forming residues (asparagine, glycine, proline and serine) and absolute charge per residue (fraction of positively and negatively charged amino acids), were found to correlate strongly with inclusion body formation. A composite parameter (CV-canonical variable) dependent on the contribution of each of the individual amino acid was derived and is as follows:

$$CV = 15.43 \{(N + G + P + S)/n\} - 29.56 \{[(R + K) - (D + E)/n] - 0.03\}$$

where N, G, P, S, R, K, D, E are the absolute numbers of asparagine, glycine, proline, serine, arginine, lysine, aspartic acid and glutamic acid residues, respectively, and n is the total number of residues in the whole sequence. A threshold discriminate CV' =1.71 [Koschorreck M. et al., BMC Genomics. 2005;6:49-59] was introduced to distinguish soluble proteins from insoluble ones. A protein is predicted to be soluble, if the difference between CV and CV' is negative. On the contrary, a CV-CV' difference larger than zero, predicts the protein to be insoluble. Further a probability of solubility was calculated from the following equation:

$$P = 0.4934 + 0.276 (CV\text{-}CV') - 0.0392(CV\text{-}CV')^2$$

[Koschorreck M. et al., BMC Genomics. 2005;6:49-59].

Using the percentage probabilities to classify proteins as soluble or insoluble, discriminant analysis successfully classifies proteins as being soluble or insoluble with an overall accuracy of 88% [Nature Bio/Technology 9, 443 - 448 (1991)].

For the PfEMP1 domain dataset, the CV-CV' values, probabilities for soluble expression in percentage, relative number of turn forming residues, charge per residue and length of protein sequence were compared. Additionally mean solubility propensities along with the lower and upper quartiles for each domain group were compared. A webserver for the calculation of this index is found at http://www.biotech.ou.edu.

The recombinant polypeptide may comprise various heterologous proteins expressed in cellular expression systems, for example, HTLV-III/LAV virus antigen, HTLV-II virus antigen, HTLV-I virus antigen, and Feline Leukemia Virus antigen, which appear as inclusion bodies under commonly-found culture conditions. Other such examples include human and porcine growth hormones, the foot and mouth disease viral capsid protein, fibroblast interferon, human insulin, somatostatin, alpha, beta, and gamma interferons, and hepatitis B antigen. In short, the method of the invention is applicable to any heterologous proteins expressed in cellular expression systems, wherein said proteins accumulate in the host cells as insoluble inclusion bodies.

Single chain antibody fragments (scFv) have previously been used as ligands in a number of bioseparation applications. In addition to their specificity and ability to be reproducibly produced, the smaller size of such fragments as compared to whole antibodies may reduce non-specific binding of contaminants. ScFvs have been used to separate a large number of target components including fractionating enantiomers in racemic mixtures and removal of microorganisms from food and water samples.

The reducing environment of the cytoplasm of cells impairs the formation of the intradomain disulphide bonds essential for the correct folding and functionality of single-chain antibody (ScFv) fragments and promotes the formation of inclusion bodies. Accordingly, most ScFvs expressed in the cytoplasm (intrabodies) are mostly inactive. Producing ScFvs as fusion polypeptides bound to polymer particle surprising allows the production of soluble, active forms, in both reducing and oxidising cellular environments.

It is also conceivable to engineer functional VHH intrabodies, which can be fused to the particle forming protein.

Intracellular binding of intrabodies to a LacZ (beta-galactosidase) dimer mediated the formation of the active tetrameric LacZ which activity can be easily detected. This was used as a screening tool for functional intrabodies after random mutagenesis (Martineau et al., 1998, Journal of Molecular Biology 280:117-127). Thus similar screening strategies can be envisaged leading to any functional intrabodies which can ultimately be fused to the particle forming protein enabling attachment of the respective intrabody to the particle.

Fusion polypeptides comprising amino acid sequences of a wide number of intrabodies that do not fold properly in the cytoplasm of cells can be produced by a process of the present invention.

During modification of the genes which code for proteins which, once expressed, bind to the particle surface, it is also possible to introduce constructs with different modifications into the cell. Once these fusion polypeptides with their different polypeptides of fragments thereof have been expressed and the polymer particles have been formed, it is possible in this manner to use the different fusion polypeptides to multifunctionalise the particle surface. This process enables straightforward and efficient mass production and separation of functionalised polymer particles.

High-level expression of recombinant proteins in *E. coli* often results in the formation of inclusion bodies.

The inventors have found that expression the recombinant polypeptides as a fusion polypeptide that binds to the polymer particle it forms either directly or indirectly allow the formation of recombinant polypeptide in soluble, active forms, which can subsequently be cleaved from the polymer particle and isolated.

Expressing the recombinant polypeptides in a soluble and active form substantially reduces the cost of recombinant protein expression. Furthermore, the methods of the invention allow the production of many proteins which to date have not be able to be readily expressed recombinantly, due to their tendency to form inclusion bodies.

In one embodiment at least one nucleic acid sequence encoding a folding chaperone or chaperonin is present in the cell and assist the folding of the recombinant polypeptides into their native, functional state. A number of different families of folding chaperones are known in the art, each acting to assist protein folding in a different manner. Folding chaperones that may find use in the present invention comprise Hsp60, Hsp70, Hsp90 and Hsp100, known as the GroEL/GroES complex, DnaK, HtpG and ClpB family in *E. coli,* respectively. Group I and Group II chaperonins are a subset of chaperones that may also be used to assist recombinant polypeptide folding in the present invention.

In one embodiment the nucleic acid sequence the recombinant polypeptide may be directly fused or indirectly fused to the particle binding domain through a peptide linker or spacer of a desired length to facilitate independent folding of the fusion polypeptides, preferably about 1, 5, 10, 15, 20, 25, 30, 35, 40, 45, 50, 55, 60, 65, 70, 75, 80, 85, 90, 95 or about 100 amino acids in length.

In one embodiment the polynucleotide sequence encodes a protease cleavage recognition sequence spaced between nucleic acid sequence encoding a particle forming protein and the nucleic acid sequence encoding the recombinant polypeptide. A protease cleavage site allows the recombinant polypeptide to be readily isolated by isolating and purifying the polymer particles, then contacting the particles with a protease to cleave the recombinant polypeptide from the particles. Suitable protease cleavage recognition sequences comprise an enterokinase recognition sequence (DDDDK), a thrombin recognition sequence (F/GPR) and a Factor Xa recognition sequence (IE/DGR).

In one embodiment the polynucleotide encodes a self-splicing element such as an intein. Inteins are self-splicing host proteins adapted for use in recombinant protein expression and purification schemes. Intein cleavage can be mediated by pH changes or the addition of thiols. A comprehensive list of inteins can be found on the InBase intein database and registry at http://tools.neb.com/inbase/index.php [Perler, F. B. (2002). Nucleic Acids Res. 30, 383-384].

Once formed, the polymer particles can be separated from the host cell by disrupting the cell and recovering the particles, preferably by physical disruption of the cell followed by separation using a cell sorter, centrifugation, filtration or affinity chromatography. Such separation protocols have the advantage of quickly and easily separating the polymer particles, and thus the recombinant polypeptides, from the protein content of the cytoplasm.

Purification of particles might employ the addition (chemically or by fusion technology) of molecules to the surface enabling affinity purification and/or adsorption to surfaces also for screening purposes.

One the polymer particle have been isolated, the recombinant polypeptides can be cleaved from the polymer particles and isolated in a substantially pure, soluble and active form. Such processes obviate the unfolding and refolding steps required to isolate soluble and active proteins from inclusion bodies.

### 8. Screening

One aspect of the invention relates to displaying and screening peptides and peptide libraries on the surface of polymer particles.

The many applications of display can be broken down into three general categories depending on the nature of the peptide being displayed. These are display of (1) proteins or protein fragments; (2) antibody fragments; and (3) random peptides.

Display of proteins or protein fragments can be used to identify catalytic and non-catalytic proteins or fragments thereof that bind other proteins, nucleic acids (DNA and RNA), carbohydrates, lipids or small chemical compounds (organic or inorganic) including compounds that are agonists, antagonists or substrates of the protein of interest. This type of display has been used to identify enzymes that catalyze particular reactions, to study the interaction between protein domains and DNA and to explore protein-protein interactions, especially interactions with multifunctional proteins, antibodies, receptors and proteins in signalling cascades. Display of random peptides can be used in similar ways, particularly to identify novel peptides that bind to target molecules of interest.

Display of antibody or antibody fragments (particularly variable region fragments such as Fab and scFv) can be used to identify antibodies that bind to an epitope of interest.

Processes of screening target molecules against polypeptides are well known in the art (Campbell and Choy 2002; Golebiowski A et al., 2003; Jager et al., 2003; Pini A et al., 2002).

In one embodiment the method of the invention comprises contacting the particles with a combinatorial library of target molecules. Preferably the combinatorial library of target molecules comprises a combinatorial library of protein domains or fragments, antibodies or antibody fragments, or organic or inorganic chemical compounds. In one embodiment the combinatorial library is displayed in a microarray.

In preferred embodiments polymer particles displaying a receptor polypeptide (such as a receptor protein, a subunit of a receptor complex or a fragment thereof) can be used to screen combinatorial libraries (including chemical or peptide combinatorial libraries) and identify binding interactions with candidate ligands that are agonists, antagonists or substrates of the receptor of interest.

Particles displaying a mixed population of library peptides can also be contacted with a target molecule to identify those particles which have a library peptide that interacts with the target molecule. Preferably the interaction is defined by the library peptide binding to the target molecule.

In one embodiment the target molecule is a receptor ligand, a protein or protein fragment, antibody or antibody fragment, carbohydrate, lipid, nucleic acid (DNA or RNA) or an organic or inorganic chemical compound.

In one embodiment the receptor polypeptide is selected from G-protein-coupled receptors, acetylcholine receptors, thrombopoietin receptors, nuclear receptors, chemokine receptors, steroid hormone receptors, epidermal growth factor receptors, toll receptors, toll-like receptors, mannose receptors, 7TM receptors, neuropeptide receptors, NMDA receptors, T cell receptors, hormone receptors, IgG Fc receptors and cytokinine receptors.

In one embodiment the receptor polypeptide may be a subtype of a receptor, comprising subtypes A1, A2a, A2b or A3 of the Adenosine receptor; subtypes alpha2A, alpha2B, alpha2C, beta1, beta2 or beta3 of the adrenergic receptor; subtype CRLR/RAMP3 of the Adrenomedullin receptor; subtypes AT1 or AT2 of the Angiotensin receptor; subtype APJ of the Apelin receptor; subtypes BRS-3, BB1 or BB2 of the Bombesin receptor; B1 or B2 of the Bradykinin receptor; CB1 or CB2 of the Cannabinoid receptor; subtype CRLR/RAMP1 of the CGRP receptor; subtypes CCR1, CCR2b, CCR3, CCR4, CCR5, CCR6, CCR7, CCR8, CCR9a, CCR10, CXCR2, CXCR3, CXCR6, CX3CR1 or XCR1 of the Chemokine receptor; subtype FPRL1 of the Chemotactic Formyl Peptide receptor; C3a or C5a of the Chemotactic Peptide receptor; CCK1 or CCK2 of the Cholecystokinin receptor; D1, D3 or D4 of the Dopamine receptor; subtype HM74-like Eicosanoid receptor; subtypes ETA or ETB of the Endothelin receptor; subtype GABAB of the GABA receptor; subtypes GalR1 or GalR2 of the Galanin receptor, subtype GHS-R of the Ghrelin receptor; mGluR5 of the Glutamate receptor; subtypes H1, H2, H3, or H4 of the Histamine receptor; subtype GPR40 of the LCFAR receptor; subtypes LTB4R1, CysLT1 or CysLT2 of the Leukotriene receptor; subtype EDG8 of the Lysophospholipid receptor; subtypes MCH1 and MCH2 of the MCH receptor, MC3, MC4 and MC5 of the Melanocortin receptor; MT1 and MT2 of the Melatonin receptor; subtype GPR54 of the Metastatine receptor; GPR38 of the Motilin receptor; subtypes M1, M2, M3, M4 and M5 of the Muscarinic receptor; NK and NK3 of the Neurokinin receptor; FM3 and FM4 of the Neuromedin U receptor; subtype NPFF2s of the Neuropeptide FF receptor; NTS1 of the Neurotensin receptor; HM7a of the Nicotinic receptor; ORL1 of the Nociceptin receptor; Kappa of the Opioid receptor; subtypes OX1, OX2 and OX2 like of the Orexin receptor; PKR1 and PKR2 of the Prokineticin receptor; subtype GPR10 of the Prolactin Rel. Peptide; subtypes CRTH2, DP, EP2, EP4 and FP of the Prostanoid receptor; P2Y2, P2Y6 and P2Y11 of the Purinergic receptor; 5HT1A, 5HT2A, 5HT2B, 5HT2Ce, 5HT2Cne, 5HT5A and 5HT6 of the Serotonin receptor; sst1, sst2a, sst3, sst4 and sst5 of the Somatostatin receptor; subtype TRHR1 of the TRH receptor; GPR14 of the Urotensin II receptor; subtypes VPAC1, VPAC2 and PAC1 of the Vasointest Peptide receptor and V1a, V1b and V2 of the Vasopressin receptor.

In one embodiment the method of the invention further comprises contacting the polymer particle or the mixed population of polymer particles with at least one target molecule.

In one embodiment the target molecule is immobilised, preferably on a plate such as an ELISA plate or microarray, on a bead such as a polystyrene bead, in immunotubes or in a suitable chromatography matrix such as an affinity matrix, for example.

In one embodiment the peptide library is incubated on a plate to allow a particle displaying a complementary library peptide to the target to bind the target.

One embodiment of the invention provides a process for screening a plurality of different target molecules in parallel for their ability to interact with a particular polypeptide, comprising the following steps: contacting different fluid samples each containing at least one of the different target molecules with a mixed population of polymer particles prepared according to a method of the invention, preferably wherein the particular target molecule is immobilized; and detecting, either directly or indirectly, the interaction of the particular target molecule with a polypeptide.

In one embodiment polymer particles comprising a polypeptide binding a target molecule are isolated using a cell sorter, affinity chromatography, centrifugation or filtration.

In one embodiment the method of the invention further comprises isolating at least one polymer particle that binds the target molecule from those that do not. The polymer particle that binds comprises a library peptide that binds the target molecule.

In one embodiment the method of the invention further comprises determining the amino acid sequence of a fusion polypeptide of at least one isolated polymer particle or determining the sequence of the nucleic acid sequence encoding the fusion polypeptide. Successive rounds may be used to enrich the pool of particles that specifically bind the target. The amino acid or nucleic acid sequence of a fusion polypeptide which comprises a library peptide that binds a target molecule may be determined by techniques known in the art.

Another aspect of the invention provides a nucleic acid sequence encoding a fusion polypeptide of the invention.

Another aspect of the invention provides an expression construct comprising a nucleic acid sequence of the invention.

Another aspect of the invention provides a cell transformed with an expression construct of the invention.

Another aspect of the invention provides a polymer particle comprising a polymer core and a fusion polypeptide associated with the polymer core wherein the fusion polypeptide comprises a particle forming protein and at least one polypeptide, as defined above.

Another aspect of the disclosure provides a cell comprising polymer particles of the invention.

Another aspect of the disclosure provides a culture comprising cells of the invention.

Another aspect of the disclosure provides a kit comprising at least one particle of the invention and instructions for use in a method of the invention.

Various aspects of the invention will now be illustrated in non-limiting ways by reference to the following examples.

## EXAMPLES

## I PARTICLE PRODUCTION

### Example 1- Preparation of polymer particles with fusion polypeptides comprising - MOG or -IL2 polypeptides.

*Construction of plasmids.*

Plasmids used for construction of plasmid mediating fusion polypeptide particle formation are shown in Figures 3 and 4.

pUC57: Amp (used to clone IL2 and MOG); pHAS (T7): Amp (pET-14b derivative (Yuan et al., 2001, Arch Biochem Biophys. 2001 Oct 1;394(1):87-98.) and pBHR68: Amp (Spiekermann et al. 1999, Arch Microbiol. 1999 Jan; 171(2):73-80) were used to generate PHA particles having fusion polypeptides comprising the phaP phasin, and MOG or IL-2.

The DNA fragments encoding either the full length mature IL2 protein (amino acids 60-169, accession no. AAN38301) or the extracellular part of the MOG protein (amino acids 1-117, accession no. Q61885) from mouse were synthesized by GenScript Corp. (USA). The codon usage was optimized for expression in *E. coli.* Sequence data for the IL2 and MOG encoding DNA fragments are shown in SEQ ID No.s 1 and 2 respectively. Each DNA fragment contained an *Nde*I site at the 5' end and a *Bam*HI site at the 3' end. These DNA fragments were inserted into the *Sma*I site of pUC57 directly after synthesis by GenScript Corp. (USA) resulting in plasmids pUC57-MOG or pUC57-IL2, respectively.

The coding region of *phaP1* gene was amplified by PCR from chromosomal DNA of *Ralstoreia eutropha* (recently renamed to *Cupriavidus necator*) using the thermostable PCR enzyme *Pfx* and oligonucleotides phaP-XbaI-NdeI and phaP-NdeI, introducing an *Xba*I site at the 5' end and an *Nde*I site including an enterokinase site at the 3' end.

The oligonucleotides used for the PCR were as follows:

(1) 5' primer for the phaP coding region containing a XbaI site for cloning purposes (5'-XbaI-transl-ATG-phaP):

AAAAATCTAG AAAAAGGAGA TATACGTATG ATCCTCACCC
CGGAACAAG (SEQ ID NO:3).

(2) 3' primer for the phaP coding region containing a NdeI site, site for cloning purposes, the coding region for the expression of a 6xHis-Tag, for purification purposes, and the enterokinase recognition sequence DDDDK for separation of the MOG and phap chimeric protein (3'-phaP-(stop)-DDDDK-6xHis-NdeI):

AAAAACATAT GGTGGTGATG GTGATGCGAG CCGCGTTTAT
CATCATCATC GGCAGCCGTC GTCTTC (SEQ ID NO:4).

The phaP PCR product containing the 5' XbaI and the 3' enterokinase and NdeI sites is as follows:

AAAAATCTAGAAAAAGGAGATATACGTATGATCCTCACCCCGGAACA
AGTTGCAGCAGCGCAAAAGGCCAACCTCGAAACGCTGTTCGGCCTGA
CCACCAAGGCGTTTGAAGGCGTCGAAAAGCTCGTCGAGCTGAACCTGC
AGGTCGTCAAGACTTCGTTCGCAGAAGGCGTTGACAACGCCAAGAAG
GCGCTGTCGGCCAAGGACGCACAGGAACTGCTGGCCATCCAGGCCGC

AGCCGTGCAGCCGGTTGCCGAAAAGACCCTGGCCTACACCCGCCACCT
GTATGAAATCGCTTCGGAAACCCAGAGCGAGTTCACCAAGGTAGCCG
AGGCTCAACTGGCCGAAGGCTCGAAGAACGTGCAAGCGCTGGTCGAG
AACCTCGCCAAGAACGCCCCGGCCGGTTCGGAATCGACCGTGGCCATC
GTGAAGTCGGCGATCTCCGCTGCCAACAACGCCTACGAGTCGGTGCAG
AAGGCGACCAAGCAAGCGGTCGAAATCGCTGAAACCAACTTCCAGGC
TGCGGCTACGGCTGCCACCAAGGCTGCCCAGCAAGCCAGCGCCACGG
CCCGTACGGCCACGGCAAAGAAGACGACGGCTGCCGATGATGATGAT
AAACGCGGCTCGCATCACCATCACCACCATATGTTTTT (SEQ ID NO:5).

The PCR product was subcloned into TA cloning plasmid pCRII. The *phaP* coding region was again amplified from plasmid pCR-phaP using oligonucleotides phaP-XbaI including an *E. coli* ribosomal binding site and phaP-NdeI. The resulting PCR product was subcloned into the *Xba*I and *NdeI* sites of pHAS (a pET-14b derivative).

Either pUC57-MOG or pUC57-IL2 was hydrolyzed with *NdeI* and *Bam*HI and the corresponding DNA fragments were subcloned into pHAS-phaP resulting in plasmids pHAS-phaP-MOG and pHAS-phaP-IL2, respectively. The respective fusion protein encoding region was then subcloned into pBHR68 using *Xba*I and *Bam*HI sites downstream of the *lac* promoter and upstream of the PHB biosynthesis operon, to yield pBHR68-phaP-IL2 (Figure 3) and pBHR68-phaP-MOG (Figure 4). A schematic view of the plasmids constructs mediating production of antigen displaying PHA granules in *E. coli* in shown in Figure 5.

Clones containing the phaP-IL2 or phaP-MOG inserts were sequenced and one clone containing the correct sequences was selected.

pHAS (pET-14b), pBHR68 and pUC57 plasmids used Amp (75 ug/ml) selection.

*Production of phasin fusion proteins at the PHA granule surface.*

Cells of *E. coli* XL1 Blue were transformed with plasmids pBHR68-PhaP-IL2 and pBHR68-PhaP-MOG, respectively. Transformants were grown at 37°C in LB medium and induced by adding isopropyl-β-D-thiogalactopyranoside (IPTG) to a final concentration of 1 mM. After growth for 48 h, cells were harvested by centrifugation and subjected to PHA granule isolation.

*Isolation of PHA granules.*

Cells were harvested by centrifugation for 15 min at 5,000 x g and 4°C. The sediment was washed and suspended in 3 volumes of 50 mM phosphate buffer (pH 7.5). Cells were passed through French Press four times at 8000 psi. The cell lysate (0.75 ml) was loaded onto a glycerol gradient (88 % and 44 % (v/v) glycerol in phosphate buffer). After ultracentrifugation for 2.5 h at 100,000 x g and 10°C, granules could be isolated from a white layer above the 88 % glycerol layer. The PHA granules were washed with 10 volumes phosphate buffer (50 mM, pH 7.5) and centrifuged at 100,000 x g for 30 min at 4°C. The sediment containing the PHA granules was suspended in phosphate buffer and stored at 4°C.

**Example 2 - Preparation of polymer particles with fusion polypeptides comprising the antibody binding ZZ domain of Protein A.**

*Construction of plasmids.*

Antibody binding domains such as Protein A from *Staphylococcus aureus* can be used to bind antibodies to the surface of the polymer particles, creating functionalised particles that can be used in a variety of immnoseparation and immunodetection applications.

The ZZ-domain of protein A was chosen as an example of a binding domain to be covalently attached to the particle surface.

Plasmids used for construction of plasmid mediating fusion protein particle formation are shown in Figures 6 and 7.

The plasmids pCWE (Peters, V. and Rehm, B.H.A. 2005, FEMS Microbiol. Lett. 248, 93-100), a pBluesrcriptSK- derivative containing the PHA synthase gene from *C. necator* (Fig. 6), pBHR80 (Qi Q., Steinbüchel A., Rehm B.H.A. 2000, Appl. Microbiol. Biotechnol. 54: 37-43), mediating the formation of a polyhydroxyalkanoate core (comprising medium chain length 3-hydroxy fatty acids) (Fig. 7) and pEZZ18, sourced from GE Healthcare (providing the ZZ domain and leader peptide encoding sequences, with Genbank Accession No. M74186 (Loewenadler et al (1987)), were used to generate a recombinant polymer synthase displaying the immunoglobulin binding site of Protein A on the surface of polymer core.

The thermostable PCR enzyme *Pfx* polymerase was used to amplify the ZZ domain and leader peptide from pEZZ 18 and to introduce *NdeI* sites at each end of the amplicon. The oligonucleotides used for the PCR were as follows:

(1) 3' primer for ZZ domain coding region:

GTAATCATATGGGGTACCGAGCTCGAATTCGCGTCTAC (SEQ ID NO:6).

(2) 5' leader peptide (+):

GCGCGCATATGACTTTACAAATACATACAGGGGGTATTAATTTG (SEQ ID NO:7).

(3) 5' leader peptide (-):

GTACACATATGGCGCAACACGATGAAGCCGTAGACAAC (SEQ ID NO:8).

The nucleotide sequences of the PCR product lacking the leader domain is shown in SEQ ID NO:9.

GTACACATAT GGCGCAACAC GATGAAGCCG TAGACAACAA ATTCAACAAA
GAACAACAAA ACGCGTTCTA TGAGATCTTA CATTTACCTA ACTTAAACGA
AGAACAACGA AACGCCTTCA TCCAAAGTTT AAAAGATGAC CCAAGCCAAA
GCGCTAACCT TTTAGCAGAA GCTAAAAAGC TAAATGATGC TCAGGCGCCG

AAAGTAGACA ACAAATTCAA CAAAGAACAA CAAAACGCGT TCTATGAGAT
CTTACATTTA CCTAACTTAA ACGAAGAACA ACGAAACGCC TTCATCCAAA
GTTTAAAAGA TGACCCAAGC CAAAGCGCTA ACCTTTTAGC AGAAGCTAAA
AAGCTAAATG ATGCTCAGGC GCCGAAAGTA GACGCGAATT CGAGCTCGGT
ACCCCATATG ATTAC

The PCR product including the leader domain is shown in SEQ ID NO: 10.

GCGCGCATAT GATGACTTTA CAAATACATA CAGGGGGTAT TAATTTGAAA
AAGAAAAACA TTTATTCAAT TCGTAAACTA GGTGTAGGTA TTGCATCTGT
AACTTTAGGT ACATTACTTA TATCTGGTGG CGTAACACCT GCTGCAAATG
CTGCGCAACA CGATGAAGCC GTAGACAACA AATTCAACAA AGAACAACAA
AACGCGTTCT ATGAGATCTT ACATTTACCT AACTTAAACG AAGAACAACG
AAACGCCTTC ATCCAAAGTT TAAAAGATGA CCCAAGCCAA AGCGCTAACC
TTTTAGCAGA AGCTAAAAAG CTAAATGATG CTCAGGCGCC GAAAGTAGAC
AACAAATTCA ACAAAGAACA ACAAAACGCG TTCTATGAGA TCTTACATTT
ACCTAACTTA AACGAAGAAC AACGAAACGC CTTCATCCAA AGTTTAAAAG
ATGACCCAAG CCAAAGCGCT AACCTTTTAG CAGAAGCTAA AAAGCTAAAT
GATGCTCAGG CGCCGAAAGT AGACGCGAAT TCGAGCTCGG TACCCCATAT
GATTAC

PCR products were then inserted into the *NdeI* sites of each of the plasmids pCWE and pBHR80, respectively. pCWE derivatives were transformed into E. coli harboring plasmid pMCS69 (Hoffmann, N., Amara, A.A., Br. Beermann, Qi, Q., B., Hinz, H.-J., Rehm, B.H.A. (2002) J. Biol. Chem, 277:42926-42936) a pBBR1MCS derivative containing genes phaA and phaB from C. necator colinear to *lac*-promotor that mediates provision of the activated precursors of polyhydroxybutyrate in *E. coli.*

Each hybrid gene of plasmids pCWE-ZZ(+)phaC and pCWE-ZZ(-)phaC was also subcloned into XbaI/BamHI sites of plasmid pBHR69 upstream of the genes phbA and phbB. This resulted in plasmids pBHR69-ZZ(+)phaC and pBHR69-ZZ(-)phaC.

To achieve overproduction of the respective fusion polypeptides at the PHA granule surface, the respective hybrid genes were also subcloned into overexpression vector pET14b downstream of the strong T7 promoter. The resulting plasmids pET14b-ZZ(+)phaC and pET14b-ZZ(-)phaC encoding either ZZ-PhaC plus or minus the signal peptide were transformed into E. coli BL21 (DE3) pLysS harboring pMCS69 (phbA, phbB).

Transformants were grown in LB medium at 30°C to an OD600 of 0.6, and then induced by adding isopropyl-β-D-thiogalactopyranoside (IPTG) to a final concentration of 0.1 mM. After growth for additional 5 h, cells were harvested by centrifugation and stored at - 80°C. When required, antibiotics were used at the following concentrations: ampicillin, 75 μg/ml and chloramphenicol, 50 μg/ml. All chemicals were purchased from Sigma-Aldrich (St. Louis, Mo., USA).

The functionality of the PHA synthase was investigated by analyzing the PHA content of the respective bacterial cells. The amount of accumulated PHA corresponds to the functionality of PHA synthase. The PHA contents were qualitatively and quantitatively determined by gas chromatography/mass spectrometry (GC-MS) after conversion of the PHA into 3-hydroxymethylester by acid-catalyzed methanolysis as perviously described (Peters, V., and B. H. A. Rehm. 2005. FEMS Microbiol. Lett. 248:93-100). No major differences in PHA accumulation could be detected when compared to cells harboring pCWE or pHAS and pMCS69 as control (data not shown). These data suggested that the ZZ-PHA synthase fusion protein mediates PHA biosynthesis and PHA granule formation. The presence and absence of the signal peptide did not impact on PHA synthase function.

Cells were harvested by centrifugation for 15 min at 5,000 x g and 4°C. The sediment was washed and suspended in 3 volumes of 50 mM phosphate buffer (pH 7.5). Cells were passed through French Press four times at 8000 psi. 0.75 ml of the cell lysate was loaded onto a glycerol gradient (88 % and 44 % (v/v) glycerol in phosphate buffer). After ultracentrifugation for 2.5 h at 100,000 x g and 10°C, granules could be isolated from a white layer above the 88 % glycerol layer. The PHA granules were washed with 10 volumes phosphate buffer (50 mM, pH 7.5) and centrifuged at 100,000 x g for 30 min at 4°C. The sediment containing the PHA granules was suspended in phosphate buffer and stored at 4°C.

**II DIAGNOSTICS**

Particles displaying a specific protein function (e.g. antigen) enable antigen or antibody capture analysis to be conducted for a range of diagnostic applications.

**Example 3 - Preparation of polymer particles displaying antigens**

Investigations carried out for the purposes of the invention have revealed that the polymer particles expressing a specific protein antigen can be used to detect antigen-specific and quantitative levels of antibodies from immunised mice using FACS.

For this example polymer particles displaying fusion polypeptides comprising a phaP phasin, and Mouse Oligodendrocyte Glycoprotein (MOG) antigen prepared according to Example 1 were used.

8-12 week C57BL/6J mice were immunized subcutaneously over the left and right flanks with indicated 50μg of recombinant MOG protein emulsified in complete Freund's adjuvant (CFA) (Difco, MI), containing 400 μg of Mycobacterium tuberculosis. Control mice were immunized as above, but with 50μg of ovalbumin (OVA) instead of MOG.

Four weeks later, mice were tail-bled and whether MOG-specific IgG antibodies could be detected and quantified were then investigated. The antisera from 4 different MOG or OVA immunized mice were pooled and diluted in FACS buffer (PBS, 1% FCS, 5mM EDTA, 0.1% sodium azide).

To $5x10^6$ polymer particles expressing the MOG-phaP fusion polypeptide, 50μl of the diluted antisera was added in one well of a 96-well plate, and incubated for 15 minutes at room temperature. Wells were then washed three times with FACS-buffer, and 50μl of a 5000 dilution of biotinylated goat anti-mouse IgG (Southern Biotechnology, Ltd) was then added and incubated for 15 minutes. After another 3 washes, wells were incubated with 50μl of a 1:2000 dilution Streaptavidin-PE (PharMingen) in FACS-buffer for 15 minutes, then washed 3 times and transferred to 200μl of FACS buffer in tubes. The samples were then run through a FACSorter and results analysed using CellQuest software (Figure 8A).

The results show that, whereas antigen-specific IgG antibodies from MOG immunised mice bound to the particles expressing the MOG-phaP fusion polypeptide, there was no or very little binding of antisera from OVA immunised mice. The results also indicate that the level of MOG-specific IgG antibodies can be detected over a broad range of dilutions (>3 orders of magnitude) as shown in Figure 8B. Further assays of antisera specificity are shown in Example 4.

These results show that the polymer particles and FACS technology is a fast, accurate and quantitative method for detecting antigen-specific antibodies. Conventional ELISA technologies are often used for this application, but such methods are more time consuming, are less sensitive and does not have the same dynamic range of detection.

**Example 4 - Determining antigen-specificity of antisera from immunised mice using Enzyme-linked Immunosorbent Assays (ELISA).**

Example 3 showed that antisera from MOG-immunized, but not OVA-immunized, mice contained antigen-specific IgG antibodies that recognized the PhaP-MOG fusion protein. The specificity of the antisera was also tested using ELISA.

Round-bottom 96-well plates (Becton, Dickinson and Company) were coated overnight at 4°C (50 μl/well) with 3μg/ml of recombinant mouse $MOG_{1-117}$ or OVA protein (Sigma-Aldrich) in PBS. Supernatant was discarded and wells blocked by adding 1% BSA in PBS (100 μl/well) for 1 h at ambient temperature. Plates were then washed 3 times with 10mM Tris/HCl, pH 7.5, 0.05% Tween 20 (ELISA buffer).

Diluted sera (50 μl/well) in 0.1% BSA/PBS from $MOG_{1-117}$ or OVA immunized mice of Example 3 were added and after 2 h, plates were washed 3 times with ELISA buffer. Biotin-conjugated anti-mouse IgG antibodies (Southern Biotechnology, Ltd) diluted 1:4,000 in 0.1 % BSA/PBS were added for 1h, and wells then wash 3 times with ELISA buffer. Amdex Streptavidin-HRP (Amersham Biosciences) diluted 1:3,000 in 0.1% BSA/PBS, (50 μl/well) was added to each well and incubated for 30 minutes. Plates were then wash 3 times and 3,3',5,5'-tetramethylbenzidine (TMB) added for 5-30 minutes. Colour development was stopped using 2 M $H_2SO_4$ and the ELISA plates were then read at 450 nm on a Benchmark microplate reader (Bio-Rad Laboratories Inc. Hercules, CA, USA).

MOG-immunised, but not OVA-immunised, anti-sera contained antigen-specific IgG antibodies that recognised the recombinant MOG-coated microtiter wells. OVA immunised mice produced OVA-specific IgG, as microtitre wells coated with OVA, but not with MOG, showed bound IgG which was easily detectable using ELISA (Figure 8C).

**Examples 5 - Use of polymer particles to detect antigen-specific antibodies.**

Investigations carried out for the purposes of the invention have revealed that expressing specific protein antigens as fusion polypeptides on the surface of the polymer particles can be used to detect monoclonal antibodies specific for conformational epitopes.

Polymer particles ($5x10^6$) expressing the MOG-phaP or IL-2-phaP fusion polypeptides produced according to Example I were incubated with anti-MOG (clone 8-18C5) unlabeled or-IL2 PE labeled mAbs (clone JES6-5H4) for 15 minutes at ambient temperature. Particles were then washed 3 times. Particles incubated with anti-MOG (clone 8-18C5) were then either incubated with APC labeled anti-mouse IgG or with biotinylated anti-mouse IgG for 15 minutes, and then washed 3 times. The particles incubated with the biotinylated anti-mouse IgG were further incubated with streptavidin-PE for 15 minutes and then washed 3 times.

All polymer particles were analysed on a FACSorter (Figure 9). Figure 9 shows the fluorescence of MOG (column A) or IL-2 (column B) fusion polypeptides on the surface of polymer particles incubated with (i) particles incubated with directly labeled anti-IL2-phycoerythrin, (ii) particles incubated with anti-MOG + biotinylated anti-mouse IgG + streptavidin-phycoerythrin, (iii) particles incubated with anti-MOG + directly labeled anti-mouse IgG-allophycocyanin.

The filled histograms show the fluorescence of the MOG- or IL-2 polymer particles incubated with the anti-IL-2 or anti-MOG mAbs, respectively, as a negative control. The empty histograms show the fluorescence of the MOG- or IL-2 polymer particles incubated with anti-MOG mAbs or anti-IL-2, respectively.

The results show that, whereas antigen-specific antibodies each bound to their respective fusion polypeptides, there was no or very little non-specific antibody binding.

**Example 6 - Production of ZZ-domain-PhaC fusion polypeptides.**

Antibody binding domains such as Protein A from *Staphylococcus aureus* can be used to bind antibodies to the surface of the polymer particles, creating functionalised particles that can be used in a variety of immnoseparation and immunodetection applications.

In this example, particles having fusion polypeptides comprising the ZZ-domain of protein A produced according to Example 2 were used.

The particles were subjected to SDS-PAGE analysis as previously described (Peters, V., and B. H. A. Rehm. 2006. Appl. Environ. Microbiol. 72:1777-83.).

ZZ-PhaC plus the N terminal signal peptide has a theoretical molecular weight of 83,981 and a protein with an apparent molecular weight of 84 kDa could be detected as predominant protein. Without the signal peptide the fusion protein has a theoretical molecular weight of 79,338 and a protein with an apparent molecular weight of 80 kDa appeared as predominant protein.

The identity of these proteins was confirmed by peptide fingerprinting using MALDI-TOF/MS. Thus both open reading frames could be efficiently and completely expressed in *E. coli.* The plasmids pET14b-ZZ(+)phaC and pET14b-ZZ(-)phaC encoding either ZZ-PhaC plus or minus the signal peptide mediated overproduction of ZZ-PhaC at the PHA granule surface.

**Example 7 - Display of the ZZ-domain at the PHA particle surface.**

To localise the ZZ domain at the particle surface, particles of *E. coli* harboring plasmids pCWE-ZZ(+)phaC, pCWE-ZZ(-)phaC, pET14b-ZZ(+)phaC, pET14b-ZZ(-) phaC of Example 2, as well as particles produced by wild type PHA synthase (pCWE or pHAS) were isolated and used for ELISA.

For ELISA, wells of microtiter plates were coated with 100 $\mu$l of a PHA granules suspension and incubated overnight at 4°C. After blocking with 3 % (w/v) BSA for 1 h, each well was incubated with pooled human serum (Sigma-Aldrich, USA) and then with polyclonal anti-IgG antibody conjugated to Horse Radish Peroxidase (HRP) (Abcam Inc, MA, USA). After each step, wells were washed several times with phosphate buffered saline. As substrate, 100 $\mu$l of an o-phenylenediamine solution (OPD, Abbott Diagnostics, IL) was added to each well and after 15 min, the reaction was stopped by adding 0.5 volumes of 3N $H_2SO_4$. The amount of substrate conversion was measured at a wavelength of 490 nm using a microtiter plate reader.

A specific binding of IgG to PHA granules isolated from *E. coli* harboring any plasmid encoding a ZZ-PHA synthase fusion protein was suggested by at least 2-fold increase in absorption at a wavelength of 490 nm when compared to the wild type PHA granules (Figure 10). The presence and absence of the signal peptide did not impact on IgG binding capacity. However, overproduction of ZZ-PhaC at the PHA granule surface significantly increased the binding capacity (Figure 10).

**Example 8 - Comparison of ZZ-PHA particles with commercially available Protein A particles.**

PHA particles displaying the IgG binding domain ZZ from protein A derived from pET14b-ZZ(-) phaC of Example 2 were used for IgG purification from human serum.

For comparative analysis protein A-Sepharose beads with immobilised, recombinant protein A were also used to purify IgG. IgG purification was conducted according to protein A sepharose 4B bead purification protocols (Sigma, USA). SDS-PAGE analysis of eluted proteins showed that the immunoglobulins (a protein representing heavy chains with an apparent molecular weight of 50 kDa and a protein representing the light chains with an apparent molecular weight of 25 kDa) were purified from human serum using the ZZ-PHA granules displaying the ZZ domain as part of the PHA synthase on the surface of the granules.

The immunoglobulins eluted from PHA granules at pH 2.7 and showed a high degree of purity comparable to the commercially available protein A-Sepharose beads (Figure 11). PHA granules formed by wild type PHA synthase did not show elution of proteins, suggesting that unspecific binding of serum proteins does not interfere with IgG purification and that the ZZ domains mediates IgG purification (Figure 11)

Control PHA granules containing only wildtype PHA synthase showed only low levels of unspecific binding which were temperature independent

**Example 9 - Preparation of polymer particles having fusion polypeptides cromprising streptavidin.**

The plasmid pBluescript (Stratagene) was used as a vector backbone, into which the phaC gene amplified from genomic DNA of *R. eutropha* was ligated without a start codon.

The nucleotide sequence of streptavidin as set forth in SEQ ID NO: 15 (derived from pET15b-NusA-SA published in Sørensen HP, Sperling-Petersen HU, Mortensen KK. Protein Exp Purif. 2003 Dec; 32(2):252-259) was subcloned using SpeI into the phaC containing plasmid at the N-terminus of PhaC.

The 5' (GCACTAGTAT GACCACGGTC TCGATTAC)- and 3'(TAACTAGTCT GCTGAACGGC GTC) primers of the steptavidin sequence are set forth in SEQ ID NO: 13 and SEQ ID NO: 14, respectively.

*PCR reaction (100 μl):*

dNTPs: 200 μM
primers: 0.5 μM each
template: 10 ng pET15b-NusA-SA
$MgCl_2$: 2 mM
Pfu DNA polymerase (Stratagene): 2.5 U
*Reaction buffer (Stratagene): 1x*

Reaction condition:

1. Denaturation: 3 min 94°C
2. Denaturation: 20 s 94°C
3. Annealing: 20 s melting temperature 50°C
4. Elongation: 60 s at 72°C

30 cycles of step 2-4.

The same bacterial strains and cultivation conditions as for Example 5.

Such streptavidin or other biotin binding fusion proteins could be used to bind biotin-labeled substrates including antigens, antibodies and nucleic acids. The strength of the biotin-streptavidin interaction permits captured substrates to be useful as ligands in subsequent separations including mRNA isolation and the capture of primary and secondary antibodies.

**III PARTICLE PRODUCTION**

**Example 10 - Production of recombinant proteins as fusion polypeptides on PHA particles.**

Production of recombinant proteins as fusion polypeptides on polymer particles was demonstrated in Example 1 using fusion polypeptides comprising a phaP phasin, and MOG or IL-2. Moreover, Example 5 demonstrated that the IL-2-phaP fusion polypeptides were produced as conformationally correct epitopes. The ability to produce conformationally correct recombinant polypeptides provides a useful vehicle for recombinant protein production.

Recombinant polypeptides can be enzymatically cleaved from the polymer particles and the phaP polypeptide using the engineered protein digestion sequences located between the recombinant polypeptide and the phaP polypeptide of the fusion polypeptide, as shown in the schematic drawings in Figure 5. In this example, cleavage of the recombinant polypeptide at the sequence DDDDK shows this site is recognised by enterokinase and is available.

Polymer particles ($5x10^6$) expressing the IL-2-phaP fusion polypeptides were produced according to the methods described in Example 1 above, then incubated with bovine enterokinase for (i) 0 hours, (ii) 1 hour, and (iii) 16 hours (Figure 12).

Following incubation, the particles were washed and then incubated with phycoerythrin labeled IL-2 mAbs (clone JES6-5H4) or anti-MOG (clone 8-18C5) unlabeled mAbs for 15 minutes at ambient temperature.

Particles incubated with anti-MOG (clone 8-18C5) were then incubated with biotinylated anti-mouse IgG for 15 minutes, and then washed 3 times, followed by further incubation with streptavidin-coupled phycoerythrin for 15 minutes.

All particles were then washed 3 times and analysed using FACS.

The filled histograms of Figure 12 show the fluorescence of the IL-2 polymer particles incubated with the anti-MOG mAbs, as a negative control. The lack of fluorescence detected shows there was no or very little non-specific antibody binding occurring.

The empty histograms show the fluorescence of the IL-2 polymer particles incubated with anti-IL-2 mAbs. After exposure to enterokinase for 0 hrs (i), the empty histogram shows a clear shift in fluorescence over that the negative control, showing IL-2 is detected and is attached to the polymer particles.

After 1 hr incubation with enterokinase (ii), a smaller shift in fluorescence intensity indicates that a proportion of IL-2 has been cleaved from the particles.

After 16 hours incubation with enterokinase (iii), no IL-2 is detectable on the surface of the polymer particles, indicating the IL-2 has been cleaved from the polymer particles.

Therefore, the IL-2-phaP fusion polypeptides can be cleaved from the polymer particles by overnight incubation with bovine enterokinase. Proteins that typically form inclusion bodies during recombinant protein expression can therefore be expressed and then cleaved off and very easily purified from the polymer particles, producing very pure proteins with high biological activity.

## IV SCREENING

### Example 11- Preparation of recombinant PHA particles containing the alpha subunit of human Acetylcholine Receptor.

The plasmids pBADHisB (Invitrogen) is used as a vector backbone, into which the phaP gene amplified from genomic DNA of *R. eutropha* is ligated.

The soluble domain of human acetylcholine-receptor as set forth in SEQ ID NO:16 ("Reconstitution of conformationally dependent epitopes on the N-terminal extracellular domain of the human muscle acetylcholine receptor alpha subunit expressed in Escherichia coli: implications for myasthenia gravis therapeutic approaches"; 2000; International Immunology, Vol. 12, No. 9, pp. 1255-1265) is subcloned using XhoI into the phaP containing plasmid at the C-terminus of PhaP.

Figure 13 shows the resulting vector pBAD-P-AchR.

Polymer particles having such fusion polypeptides can be used to screen for molecules that bind the soluble domain of human acetylcholine-receptor.

### Example 12 -Preparation of recombinant PHA particles containing the alpha subunit of human Thrombepoietin Receptor Mpl).

Once again the plasmid pBADHisB (Invitrogen) is used as a vector backbone, into which the phaP gene amplified from genomic DNA of *R. eutropha* is ligated.

The alpha subunit of human Thrombopoietin Receptor Mpl as set forth in SEQ ID NO: 17 ("Expression of the Soluble Extracellular Domain of Human Thrombopoietin Receptor Using a Maltose-Binding Protein-Affinity Fusion System"; 2004; Biol. Pharm. Bull. 27(2): 219-221) is subcloned into the phaP containing plasmid at the C-terminus of PhaP.

The 5' (GGCTACTCGA GATGAATTCG AGCTCGAACA ACAACAACAA TAAC) and 3' (CAGCTTCGAA TTAAGTTGGG TCCGACCACG AGCTCCAGGG) primers of the Mpl sequence are set forth in SEQ ID NO: 18 and SEQ ID NO:19, respectively.

Figure 14 shows the resulting vector pBAD-P-Mpl.

Polymer particles having such fusion polypeptides can be used to screen for molecules that bind the alpha subunit of human Thrombopoietin Receptor.

## V ENZYME IMMOBILISATION

### Example 13 - Stability of the bond between the surface proteins and the polymer core of the polymer particles

Investigations carried out for the purposes of the invention have revealed that the polymer synthase cannot be detached from the core of the biodegradable polymer particle either by treatment with denaturing reagents, such as for example sodium dodecyl sulphate (SDS), urea, guanidium hydrochloride or dithiothreitol, nor by the use of acidic conditions.

This is indicative of the presence of a covalent linkage between the polymer particles and the polymer particle binding domain of the polymer synthase. The elevated stability of the bond enables stable transportation of substances bound to or incorporated into the polymer particles to their target site.

The N-terminus fragment of the surface-bound polymer synthase (N-terminus to the beginning of the conserved $\alpha/\beta$-hydrolase domain) is extremely variable and may be replaced by functional proteins using genetic engineering methods. In this manner, polymer synthase activity and synthesis of polymer particles are retained (Rehm, B.H.A. et al, Biochem. Biophys. Acta 2002, Vol. 1594, pp. 178-190). As a consequence, surface functionalisation is obtained which exhibits elevated stability.

Protein analysis has surprisingly showed that with a N-terminal fusion the copy number of the polymer synthase at the particle surface is strongly enhanced compared to the non-fused polymer synthase, which is almost only detectable by immunological analysis.

**Example 14 - Protein A stability.**

ZZ-PHA granules of Example 2 were subjected to repeated purification cycles, demonstrating consistent purification performance and strong stability. Temperature stability was tested by subjecting ZZ-PHA granules to increasing temperatures and then assessing the IgG binding capacity using ELISA. Granules were heated for 15 min at different temperatures and 100 $\mu$l of suspension added to microtiter plate. The plate was left overnight at 4oC. The plate was then washed three times in PBS, human serum dissolved in PBS was added and the plate incubated for 1 hour at room temperature. The wells were washed three times with PBS and anti-IgG-HRP was added and incubated for 30 minutes at room temperature. The wells were then washed ten times with PBS and developed as described in Example 7.

Figure 15 shows that at 60°C, binding capacity was dropping to 60% binding capacity suggesting that the ZZ domain was partially unfolding.

**Example 15-$\beta$-Galactosidase Enzyme Immobilisation**

Investigations carried out for the purposes of the invention have revealed that expressing enzymes as fusion polypeptides on the surface of the polymer particles can be used to stabilise the enzyme for prolonged periods of time under a range of storage conditions.

In this example, PHA synthases were used to covalently immobilize $\beta$-galactosidase at the PHA granule surface.

*Construction of plasmids.*

Plasmids used to produce a LacZ-PhaC1 ($\beta$-galactosidase-PHA synthase) fusions were constructed as follows (restriction recognition sites are underlined). A SpeI site containing adaptor, encoding the linker region, was generated by hybridization of the oligonucleotides adaptor 5'-P-<u>TATG</u>GCTCTG C<u>ACTAGT</u>CAC TGC-<u>CA</u>-3' (SEQ ID NO: 20) and adaptor reverse 5'-P-<u>TATG</u>GCAGTG <u>ACTAGT</u>GCAG AG-<u>CA</u>-3' (SEQ ID NO: 21). The adaptor was inserted into the NdeI site of pBHR80. The SpeI site was used to insert the lacZ gene in frame with the respective PHA synthase gene. The lacZ gene coding region was amplified by PCR from genomic DNA of E. coli S17-1 using oligonucleotides 5'-lacZ-SpeI 5'-GG**ACTAGT**AT GACCATGATT ACGGATTCAC TGG-3' (SEQ ID NO: 22) and 3'-lacZ-SpeI 5'-CA<u>ACTAGT</u>TT TTTGACACCA GACCAACTGG TAATTG-3' (SEQ ID NO: 23) which provided SpeI sites. To investigate the LacZ-PHA synthase in the natural host a broad-host-range construct was generated by subcloning the XbaI/BamHI DNA fragment from pBHR80AlacZ into the respective sites of pBBR1JO-5 (Peters, V., and B. H. A. Rehm. 2005. FEMS Microbiol. Lett. 248:93-100) resulting in plasmid pBBR1J05- lacZphaC1. To achieve overexpression of LacZ-PhaCl and PhaC1, the XbaI/BamHI DNA fragments from pBHR80 and pBHR80AlacZ were subcloned into the respective sites of pET14b and transformed into strain BL21 (DE3) pLysS.

Cells of E. coli BL21 (DE3) pLysS were transformed with plasmids pET14b-phaC1 and pET14b-lacZphaC1. Transformants were grown at 30°C to an $OD_{600}$ of 0.6, and then induced by adding isopropyl-$\beta$-D-thiogalactopyranoside (IPTG) to a final concentration of 0.5 mM. After growth for additional 5 h, cells were harvested by centrifugation and stored at - 80°C.

*Complementation of isogenic marker free P. aeruginosa $\Delta$phaC1-Z-C2 mutant.*

For complementation of the PHA-negative mutant, the lacZ-phaC1 gene of plasmid pBHR80AlacZ was hydrolysed with XbaI and BamHI and inserted into XbaI and BamHI sites of broad-host-range vector pBBR1JO-5, resulting in plasmid pBBR1JO5-lacZphaC1. E. coli S17-1 was used as donor to transfer plasmid pBBR1JO5-lacZphaC1 into P. aeruginosa ΔphaC1-Z-C2, and transconjugants were selected on MSM containing 150 μg/ml gentamycin (6). Cells were then grown under PHA-accumulating conditions and the PHA content was determined by GC/MS analysis.

*In vivo PHA synthase activity.*

In vivo PHA synthase activity was obtained by analyzing PHA content of the respective bacterial cells. The amount of accumulated PHA corresponds to the relative in vivo PHA synthase activity. The PHA contents were qualitatively and quantitatively determined by gas chromatography/mass spectrometry (GC/MS) after conversion of the PHA into 3-hydroxymethylester by acid-catalyzed methanolysis.

*Isolation of PHA granules.*

Cells were harvested by centrifugation for 15 min at 5,000 x g and 4°C. The sediment was washed and suspended in 3 volumes of 50 mM phosphate buffer (pH 7.5). Cells were passed through French Press three times at 8000 Psi. 0.8 ml of the cell lysate was loaded onto a glycerol gradient (88 % and 44 % (v/v) glycerol in phosphate buffer). After ultracentrifugation for 2 h at 100,000 x g and 4°C, granules could be isolated from a white layer above the 88 % glycerol layer. The PHA granules were washed with 10 volumes phosphate buffer (50 mM, pH 7.5) and centrifuged at 100,000 x g for 30 min at 4°C. The sediment containing the PHA granules was suspended in phosphate buffer and stored at 4°C.

To localize LacZ at the PHA granules surface, PHA granules of P. aeruginosa ΔphaC1-Z-C2 harboring plasmid pBBR1JO5-lacZphaC1 and PHA granules produced by the wildtype P. aeruginosa PAO1 were isolated and used for ELISA.

For ELISA, wells of microtiter plates were coated with 200 μl of a PHA granules suspension and incubated overnight at 4°C. After blocking with 3 % (w/v) BSA for 1 h, each well was incubated with polyclonal anti-β-galactosidase antibody conjugated to Horse Radish Peroxidase (HRP) (Abcam Inc, MA, USA). After each step, wells were washed several times with phosphate buffered saline. As substrate, 200 μl of an o-phenylenediamine solution (OPD, Abbott Diagnostics, IL) was added to each well and after 30 min, the reaction was stopped by adding 0.5 volumes of 3N $H_2SO_4$. The amount of substrate conversion was measured at a wavelength of 405 nm using a microtiter plate reader.

A specific binding of anti-LacZ antibodies to PHA granules isolated from P. aeruginosa ΔphaC1-Z-C2 harboring pBBR1JO5-lacZphaC1 was suggested by a two-fold increase in absorption at a wavelength of 405 when compared to the wildtype PHA granules (Figure 16).

*β-galactosidase activity assays.*

The LacZ activity was analyzed in order to determine whether LacZ remains active when fused with its C terminus to the N terminus of the PHA synthase and immobilized at the PHA granule surface. LacZ activity could be detected and showed an average activity of 68,000 MU.

*Determination of kinetic parameters of β-galactosidase immobilized at the PHA granule surface.*

In order to determine enzyme kinetics, β-galactosidase activity of isolated PHA granules was monitored for 10 min (data not shown). The ortho-nitrophenyl-β-(D)-galactopyranosid (ONPG) concentration was ranging from 50 μM to 500 μM. The correlation of $V_o$ with the substrate concentration could be fitted to Michaelis-Menten kinetics with the aid of non-linear regression analysis (Sigma Plot enzyme kinetics, systat soflware, Inc.). A $K_M$ of 630 μM and a $V_{max}$ of 17.6 nmol/min could be derived.

*Enzyme stability.*

The stability of LacZ immobilized at PHA granules under various storage conditions was investigated. LacZ activity was monitored over a period of 12 weeks. The PHA granule suspension was stored at 4°C either with added protease inhibitor cocktail (Roche Diagnostics, IN, USA) or with protease inhibitor cocktail plus 20% (v/v) glycerol. The addition of 20% (v/v) glycerol resulted in the best storage stability with 91% of the initial activity after four weeks. No addition of 20% (v/v) glycerol resulted after four weeks in a reduction of LacZ activity to 84 % of the initially determined LacZ activity (Table 1). Interestingly, after 12 weeks, there was still 87% of the initial activity detectable in the PHA granule suspension containing glycerol, while the LacZ activity of the PHA granule suspension without glycerol showed already an activity reduced to 75% of the initial LacZ activity (Table 1).

TABLE 1. Determination of enzyme stability of β-galactosidase at PHA granule surface.

| Storage time (weeks) | β-galactosidase activity (protease inhibitor)[a] [MU] | β-galactosidase activity (protease inhibitor/ 20% (v/v) glycerol)[a] [MU] |
|---|---|---|
| 0 | 77400 | 77400 |
| 3 | 65000 | 68900 |
| 4.5 | 60900 | 70300 |
| 12 | 58194 | 67287 |
| 27 | 55500 | 65300 |

[a], Protease inhibitor and 20% (v/v) glycerol were added to the PHA granules suspension at time point 0. PHA granules were stored at 4°C. β-galactosidase activity was measured on isolated PHA granules.

**Example 16 - Comparison between lac and T7 promoters.**

In this example the effect of promoter strength was investigated.

PHA particles displaying the IgG binding domain ZZ from protein A of Example 2 derived from pET 14b-ZZ(-) phaC (under the control of the T7 promoter) were compared to particles derived from pBHR69-ZZ(-)phaC (under control of the lac promoter).

Figure 17 shows ZZ domain displaying aged particles derived from pBHR69-ZZ(-)phaC (A), particles freshly derived from pBHR69-ZZ(-)phaC (B) and pET14b-ZZ(-) phaC (C), incubated with 0 (i), 1 (ii) or 10 (iii) μg/ml of a labeled mouse IgG2b mAb (which binds well to protein A and is fluorescently labeled (conjugated to) with PE (phycoerythrin)). At the highest concentration, one can see that T7 particles bind at least 10 x more IgG/particle. At lower, but not saturating, concentrations (1 μg/ml) it still binds more IgG.

**Example 17-Comparison between ZZ-particles and commercially available BioMag beads.**

In this example the IgG binding affinity ofZZ domain displaying particles was compared with commercially available Protein A BioMag beads (Perspective Biosystems).

PHA particles (A) (ca. 150 nm diameter) derived from pBHR69-ZZ(-)phaC of Example 2 displaying the IgG binding domain ZZ from protein A were shown to display at least the same binding affinity as the BioMag beads (B). (Figure 18).

**Example 18 - Particle size BioMag-BNP comparative example.**

In this example the effect of particle size was investigated.

PHA particles displaying the IgG binding domain ZZ from protein A of Example 2 derived from pET14b-ZZ(-) phaC were compared to particles derived from pBHR69-ZZ(-)phaC.

PHA particles derived from pET14b-ZZ(-) phaC with an average diameter of about 100 nm (C) were shown to have an about 10-fold increased binding affinity when compared to aged particles derived from pBHR69-ZZ(-)phaC (A) and particles freshly derived from pBHR69-ZZ(-)phaC (B) both of which had an average particle diameter of about 150 nm. (Figure 19).

**Example 19 -size distribution of polymer particles derived from a strong promoter.**

In this example the effect of promoter strength on particle size was investigated. Cells were grown to cell densities of $16^9$-$10^{10}$/mL in 50 mL culture. Cell images were taken by transmission electron microscopy at an absolute magnification of 16,000 to 75,250 times, allowing resolution down to about 40 nm. Representative images of particle size were selected and polymer particles within 33 cells were measured with a ruler.

The size distribution of polymer particles displaying the IgG binding domain ZZ from protein A of Example 2 derived from pET14b-ZZ(-) phaC is shown in Figure 20.

90% of the particles produced had a diameter of about 200 nm or below, 80 % had a diameter about 150 nm or below, 60 % had a diameter about 100 nm or below, 45 % had a diameter about 80 nm or below, 40 % had a diameter about 60 nm or below, 25 % had a diameter about 50 nm or below, and 5 % had a diameter about 35 nm or below.

**Example 20 - Quantifying Particle fusion polypeptide coverage**

The level of fusion polypeptide coverage can be estimated by separating the particle attached proteins using SDS-PAGE, the fraction of fusion polypeptides estimated based on the intensity of the fusion protein band as outlined in Example 6.

**INDUSTRIAL APPLICATION**

The methods, of the present invention have utility in diagnostics, protein production, biocatalyst immobilisation, and drug delivery.

Those persons skilled in the art will understand that the above description is provided by way of illustration only and that the invention is not limited thereto.

## Claims

1. A method of detecting and optionally isolating at least one target component, the method comprising:

   (a) providing at least one polymer particle comprising at least one fusion polypeptide, wherein the fusion polypeptide Is produced *in vivo* in a host cell from an expression construct operably linked to a strong promoter such that at least 20% of the surface area of the polymer particles is covered by fusion polypeptides, the fusion polypeptide being covalently or non-covalently bound to the polymer particle, the fusion polypeptide comprising

      (i) polymer synthase that comprises a polymer particle binding domain, and
      (ii) at least one fusion partner that will bind a target component,

   (b) contacting the polymer particle with a composition comprising a target component such that the fusion partner binds the target component to form a complex,
   (c) detecting and optionally, isolating the complex.

2. A method as claimed in claim 1 wherein the fusion partner that will bind a target component is Protein A or a ZZ domain.

3. A method as claimed in claim 1 or 2 wherein the target component Is selected from a protein, a protein fragment, a peptide, a polypeptide, a polypeptide fragment, an antibody, an antibody fragment, an antibody binding domain, an antigen, an antigen fragment, an antigenic determinant, an epitope, a hapten, an immunogen, an immunogen fragment, a metal, a metal-coated molecule, biotin, a biotin derivative, avidin, streptavidin, an inhibitor, a co-factor, a substrate, an enzyme, an abzyme, a receptor, a receptor fragment, a receptor subunit, a receptor subunit fragment, a ligand, a receptor ligand, a receptor agonist, a receptor antagonist, a signalling molecule, a signalling protein, a signalling protein fragment, a growth factor, a growth factor fragment, a transcription factor, a transcription factor fragment, an inhibitor, a cytokine, a chemokine, an inflammatory mediator, a monosaccharide, an oligosaccharide, a polysaccharide, a glycoprotein, a lipid, a cell, a cell surface protein, a cell-surface lipid, a cell-surface carbohydrate, a cell-surface glycoprotein, a cell extract, a virus, a virus coat protein, a hormone, a serum protein, a milk protein, a macromolecule, a drug of abuse, a coupling reagent, a polyhistidine, a pharmaceutically active agent, a biologically active agent, a label, a coupling reagent, a library peptide, an expression construct, a nucleic acid or a combination thereof.

4. A method of claim 1, 2 or 3, wherein the at least one polymer particle is produced by a process comprising:

   A) providing a host cell comprising at least one expression construct operably linked to a strong promoter, the expression construct comprising:

      (1) at least one nucleic acid sequence encoding a polymer synthase; or
      (2) at least one nucleic acid sequence encoding a fusion polypeptide that comprises a polymer synthase and at least one fusion partner;

   B) cultivating the host cell under conditions suitable for expression of the expression construct and for formation of polymer particles by the polymer synthase; and
   C) separating the polymer particles from the cultivated host cells to produce a composition comprising polymer particles.

5. A method as claimed in claim 4 wherein the host cell further comprises an additional expression construct comprising at least one nucleic acid sequence encoding an additional fusion polypeptide, the fusion polypeptide comprising

   (a) a polymer particle binding domain, a polypeptide that comprises a polymer particle binding domain or a particle forming protein that comprises a polymer particle binding domain, and
   (b) at least one fusion partner.

6. A method as claimed in any one of claims 1 to 5 wherein the strong promoter is a viral promoter or a phage promoter, preferably a T7 phage promoter

7. A method as claimed in any one of claim 4 to 7, wherein the process of producing the at least one polymer particle further comprises adding at least one polypeptide or at least one substance or a combination thereof while cultivating the host cells so that the at least one polypeptide or at least one substance binds to or is incorporated into the polymer particles or further comprising contacting the polymer particles with adding at least one polypeptide or at least one substance or a combination thereof that binds to or is adsorbed into the polymer particles.

**8.** A method as claimed in claim 7 wherein the substance is a lipophilic substance that is incorporated into or adsorbed into the polymer particles, or a coloured or fluorescent molecule, a radioisotope, one or more metals or a combination thereof.

**9.** A method of any one of claims 1 to 8 wherein the at least one polymer particle comprises a composition comprising a plurality of polymer particles having an average diameter of less than about 200 nm, or less than about 150 or 110 nm.

**10.** A method as claimed In any one of claims 1 to 11 wherein at least about 30, 40 or 50% of the surface area of the polymer particles is covered by surface-bound proteins.

**11.** A method as claimed in claim 9 wherein the composition further comprises at least one substance bound to or incorporated into the polymer particles or a combination thereof,

**12.** A method as claimed claim 11 wherein the substance is a lipophilic substance; or a coloured or fluorescent molecule, a radioisotope, one or more metals, or a combination thereof.

**13.** A method as claimed in any one of claims 1 to 12 wherein the fusion partner is selected from a protein, a protein fragment, a binding domain, a target-binding domain, a binding protein, a binding protein fragment, an antibody, an antibody fragment, an antibody heavy chain, an antibody light chain, a single chain antibody, a single-domain antibody, a Fab antibody fragment, an Fc antibody fragment, an Fv antibody fragment, a F(ab')2 antibody fragment, a Fab' antibody fragment, a single-chain Fv (scFv) antibody fragment, an antibody binding domain, an antigen, an antigenic determinant, an epitope, a hapten, an immunogen, an immunogen fragment, biotin, a biotin derivative, an avidin, a streptavidin, a substrate, an enzyme, an abzyme, a co-factor, a receptor, a receptor fragment, a receptor subunit, a receptor subunit fragment, a ligand, an inhibitor, a hormone, a lectin, a polyhistidine, a coupling domain, a DNA binding domain, a FLAG epitope, a cysteine residue, a library peptide, a reporter peptide, and an affinity purification peptide, or a combination thereof.

**14.** A method as claimed in any one of claims 1 to 13 wherein

(a) a coupling reagent is bound to the fusion partner, or
(b) a coupling reagent Is bound to the fusion partner and a substance is bound to the coupling reagents, or
(c) a substance is bound to the fusion partner, or
(d) the polymer synthase or the particle forming protein has been chemically modified with a coupling reagent to form at least one binding domain, or
(e) a combination thereof.

**15.** A method as claimed in any one of claims 1 to 13 wherein the fusion partner comprises a binding domain and

(a) a coupling reagent is bound to the binding domain, or
(b) a coupling reagent is bound to the binding domain and a substance is bound to the coupling reagent, or
(c) a substance is bound to the binding domain,
(d) or a combination thereof.

**16.** A method as claimed in any one of claims 1 to 15 wherein detecting the complex comprises contacting the polymer particle with a label that will bind to the complex, to the at least one fusion polypeptide or to the polymer particle, and detecting the label,

**17.** A method as claimed in any one of claims 1 to 16 wherein the polymer particle comprises

(a) a label bound to or incorporated Into the polymer particle, or
(b) at least one additional fusion polypeptide comprising

(I) a polymer particle binding domain, a polypeptide that comprises a polymer particle binding domain or a particle forming protein that comprises a polymer particle binding domain, and

(II) a fusion partner that will bind a labelled molecule, or

(c) at least one additional fusion polypeptide comprising a reporter protein, or
(d) any combination of any two or more thereof.

**18.** A method as claimed in any one of claims 1 to 17 wherein the polymer particle comprises two or more different fusion polypeptides.

**19.** A method as claimed in any one of claims 1 to 18 wherein the polymer particle further comprises at least one compound bound to or incorporated into the polymer particle, or a combination thereof, wherein the compound Is a coloured or fluorescent molecule, a radioisotope, or one or more metals, or a combination thereof.

**20.** A method as claimed In any one of claims 1 to 19 wherein the fusion partner that will bind a target component further comprises

(a) an antigen, an antigenic determinant, an epitope or an immunogen, or
(b) an antibody or antibody fragment, or
(c) an antibody binding domain.

**21.** A method as claimed in any one of claims 1 to 20, wherein detection of the complex comprises cell sorting, preferably fluorescence-activated cell sorting.

**22.** A method as claimed in any one of claims 1 to 21, wherein the fusion partner further comprises an antibody binding domain, preferably Protein G or Protein L.

**23.** A method as claimed In any one of claims 1 to 22 for diagnosis of a disease or condition.

**24.** A method of any one of claims 1 to 22 wherein the polymer is selected from poly-betaamino acids, polylactates, polythloesters and polyesters.

**25.** A method of any one of claims 1 to 24 wherein the polymer synthase is a polyhydroxyalkanoate polymer synthase,

**Patentansprüche**

**1.** Verfahren zum Nachweisen und gegebenenfalls Isolieren wenigstens einer Zielkomponente, wobei man bei dem Verfahren:

(a) wenigstens ein Polymerteilchen bereitstellt, das wenigstens ein Fusionspolypeptid umfasst, wobei das Fusionspolypeptid in vivo in einer Wirtszelle von einem Expressionskonstrukt in operativer Verknüpfung mit einem starken Promotor produziert wird, so dass wenigstens 20% der Oberfläche der Polymerteilchen mit Fusionspolypeptiden bedeckt sind, wobei das Fusionspolypeptid kovalent oder nicht kovalent an das Polymerteilchen gebunden ist, wobei das Fusionspolypeptid

(i) Polymersynthase, die eine Polymerteilchen bindende Domäne umfasst, und
(ii) wenigstens einen Fusionspartner, der eine Zielkomponente bindet,

umfasst,
(b) das Polymerteilchen mit einer eine Zielkomponente umfassenden Zusammensetzung in Kontakt bringt, so dass der Fusionspartner die Zielkomponente unter Bildung eines Komplexes bindet,
(c) den Komplex nachweist und gegebenenfalls isoliert.

**2.** Verfahren nach Anspruch 1, wobei es sich bei dem Fusionspartner, der eine Zielkomponente bindet, um Protein A oder eine ZZ-Domäne handelt.

**3.** Verfahren nach Anspruch 1 oder 2, wobei die Zielkomponente aus einem Protein, einem Proteinfragment, einem Peptid, einem Polypeptid, einem Polypeptidfragment, einem Antikörper, einem Antikörperfragment, einer Antikörper bindenden Domäne, einem Antigen, einem Antigenfragment, einer antigenen Determinante, einem Epitop, einem Hapten, einem Immunogen, einem Immunogenfragment, einem Metall, einem metallbeschichteten Molekül, Biotin, einem Biotin-Derivat, Avidin, Streptavidin, einem Inhibitor, einem Cofaktor, einem Substrat, einem Enzym, einem Abzym, einem Rezeptor, einem Rezeptorfragment, einer Rezeptor-Untereinheit, einem Fragment einer Rezeptor-Unter-einheit, einem Liganden, einem Rezeptorliganden, einem Rezeptoragonisten, einem Rezeptorantagonisten, einem Signalgebungsmolekül, einem Signalgebungsprotein, einem Fragment eines Signalgebungsproteins, einem Wachstumsfaktor, einem Wachstumsfaktorfragment, einem Transkriptionsfaktor, einem Fragment eines Transkriptionsfaktor, einem Inhibitor, einem Cytokin, einem Chemokin, einem Entzündungsvermittler, einem Monosaccharid, einem Oligosaccharid, einem Polysaccharid, einem Glykoprotein, einem Lipid, einer Zelle, einem Zelloberflächenprotein, einem Zelloberflächenlipid, einem Zelloberflächenkohlenhydrat, einem Zelloberflächenglykoprotein, einem Zellextrakt, einem Virus, einem Virushüllprotein, einem Hormon, einem Serumprotein, einem Milchprotein, einem Makromolekül, einer Droge, einem Kupplungsreagens, einem Polyhistidin, einem pharmazeutischen Wirkstoff, einem biologischen Wirkstoff, einer Markierung, einem Kupplungsreagens, einem Bibliothekspeptid, einem Expressionskonstrukt, einer Nukleinsäure oder einer Kombination davon ausgewählt ist.

**4.** Verfahren gemäß Anspruch 1, 2 oder 3, wobei das wenigstens eine Polymerteilchen in einem Vorgang produziert wird, bei dem:

A) eine Wirtszelle bereitgestellt wird, die wenigstens ein Expressionskonstrukt in operativer Verknüpfung mit einem starken Promotor umfasst, wobei das Expressionskonstrukt:

(1) wenigstens eine für eine Polymersynthase codierende Nukleinsäuresequenz; oder
(2) wenigstens eine für ein Fusionspolypeptid, das eine Polymersynthase und wenigstens einen Fusionspartner umfasst, codierende Nukleinsäuresequenz

umfasst;
B) die Wirtszelle unter für die Expression des Expressionskonstrukts und für die Bildung von Polymerteilchen durch die Polymersynthase geeigneten Bedingungen kultiviert wird; und
C) die Polymerteilchen von den kultivierten Wirtszellen getrennt werden, so dass eine Polymerteilchen umfassende Zusammensetzung erhalten wird.

**5.** Verfahren nach Anspruch 4, wobei die Wirtszelle ferner ein zusätzliches Expressionskonstrukt umfasst, das wenigstens eine für ein zusätzliches Fusionspolypeptid codierende Nukleinsäuresequenz umfasst, wobei das Fusionspolypeptid

(a) eine Polymerteilchen bindende Domäne, ein Polypeptid, das eine Polymerteilchen bindende Domäne umfasst, oder ein Teilchen bildendes Protein, das eine Polymerteilchen bindende Domäne umfasst, und
(b) wenigstens einen Fusionspartner

umfasst.

**6.** Verfahren nach einem der Ansprüche 1 bis 5, wobei es sich bei dem starken Promotor um einen viralen Promotor oder einen Phagen-Promotor, vorzugsweise einen T7-Phagen-Promotor handelt.

**7.** Verfahren nach einem der Ansprüche 4 bis 7, wobei bei dem Vorgang der Produktion des wenigstens einen Polymerteilchens ferner wenigstens ein Polypeptid oder wenigstens eine Substanz oder eine Kombination davon während der Kultivierung der Wirtszellen zugegeben wird, so dass das wenigstens eine Polypeptid bzw. die wenigstens eine Substanz an die Polymerteilchen bindet oder darin eingebaut wird, oder ferner die Polymerteilchen unter Zugeben wenigstens eines Polypeptids oder wenigstens einer Substanz oder einer Kombination davon, das bzw. die an die Polymerteilchen bindet oder darin adsorbiert wird, in Kontakt bringt.

**8.** Verfahren nach Anspruch 7, wobei es sich bei der Substanz um eine lipophile Substanz, die in die Polymerteilchen eingebaut oder adsorbiert wird; oder ein Farb- oder Fluoreszenzmolekül, ein Radioisotop, ein oder mehrere Metalle oder eine Kombination davon handelt.

**9.** Verfahren gemäß einem der Ansprüche 1 bis 8, wobei das wenigstens eine Polymerteilchen eine mehrere Polymerteilchen mit einem mittleren Durchmesser von weniger als etwa 200 nm oder weniger als etwa 150 oder 110 nm umfassende Zusammensetzung umfasst.

**10.** Verfahren nach einem der Ansprüche 1 bis 11, wobei wenigstens etwa 30, 40 oder 50% der Oberfläche der Polymerteilchen mit oberflächengebundenen Proteinen bedeckt sind.

**11.** Verfahren nach Anspruch 9, wobei die Zusammensetzung ferner wenigstens eine an die Polymerteilchen gebundene oder darin eingebaute Substanz oder eine Kombination davon umfasst.

**12.** Verfahren nach Anspruch 11, wobei es sich bei der Substanz um eine lipophile Substanz; oder ein Farb- oder Fluoreszenzmolekül, ein Radioisotop, ein oder mehrere Metalle oder eine Kombination davon handelt.

**13.** Verfahren nach einem der Ansprüche 1 bis 12, wobei der Fusionspartner aus einem Protein, einem Proteinfragment, einer Bindungsdomäne, einer zielbindenden Domäne, einem Bindungsprotein, einem Bindungsproteinfragment, einem Antikörper, einem Antikörperfragment, einer schweren Antikörperkette, einer leichten Antikörperkette, einem Einzelketten-Antikörper, einem Einzeldomänen-Antikörper, einem Fab-Antikörperfragment, einem Fc-Antikörperfragment, einem Fv-Antikörperfragment, einem F(ab')2-Antikörperfragment, einem Fab'-Antikörperfragment, einem Einzelketten-Fv(scFv)-Antikörperfragment, einer Antikörper bindenden Domäne, einem Antigen, einer antigenen Determinante, einem Epitop, einem Hapten, einem Immunogen, einem Immunogenfragment, Biotin, einem Biotin-Derivat, einem Avidin, einem Streptavidin, einem Substrat, einem Enzym, einem Abzym, einem Cofaktor, einem Rezeptor, einem Rezeptorfragment, einer Rezeptor-Untereinheit, einem Fragment einer Rezeptor-Untereinheit, einem Liganden, einem Inhibitor, einem Hormon, einem Lectin, einem Polyhistidin, einer Kupplungsdomäne, einer DNA bindenden Domäne, einem FLAG-Epitop, einem Cysteinrest, einem Bibliothekspeptid, einem Reporterpeptid und einem Affinitätsreinigungspeptid oder einer Kombination davon ausgewählt ist.

**14.** Verfahren nach einem der Ansprüche 1 bis 13, wobei

(a) ein Kupplungsreagens an den Fusionspartner gebunden ist oder
(b) ein Kupplungsreagens an den Fusionspartner und eine Substanz an das Kupplungsreagens gebunden ist oder
(c) eine Substanz an den Fusionspartner gebunden ist oder
(d) die Polymersynthase oder das Teilchen bildende Protein mit einem Kupplungsreagens chemisch modifiziert wurde, so dass wenigstens eine Bindungsdomäne gebildet wird, oder
(e) eine Kombination davon.

**15.** Verfahren nach einem der Ansprüche 1 bis 13, wobei der Fusionspartner eine Bindungsdomäne umfasst und

(a) ein Kupplungsreagens an die Bindungsdomäne gebunden ist oder
(b) ein Kupplungsreagens an die Bindungsdomäne und eine Substanz an das Kupplungsreagens gebunden ist oder
(c) eine Substanz an die Bindungsdomäne gebunden ist
(d) oder eine Kombination davon.

**16.** Verfahren nach einem der Ansprüche 1 bis 15, wobei man beim Nachweisen des Komplexes das Polymerteilchen mit einer Markierung in Kontakt bringt, die an den Komplex, an das wenigstens eine Fusionspolypeptid oder an das Polymerteilchen bindet, und die Markierung nachweist.

**17.** Verfahren nach einem der Ansprüche 1 bis 16, wobei das Polymerteilchen

(a) eine an das Polymerteilchen gebundene oder darin eingebaute Markierung oder
(b) wenigstens ein zusätzliches Fusionspolypeptid, das

(i) eine Polymerteilchen bindende Domäne, ein Polypeptid, das eine Polymerteilchen bindende Domäne umfasst, oder ein Teilchen bildendes Protein, das eine Polymerteilchen bindende Domäne umfasst, und
(ii) einen Fusionspartner, der ein markiertes Molekül bindet,

umfasst, oder
(c) wenigstens ein zusätzliches Fusionspolypeptid, das ein Reporterprotein umfasst, oder
(d) eine beliebige Kombination von zwei oder mehr davon

umfasst.

**18.** Verfahren nach einem der Ansprüche 1 bis 17, wobei das Polymerteilchen zwei oder mehr unterschiedliche Fusionspolypeptide umfasst.

**19.** Verfahren nach einem der Ansprüche 1 bis 18, wobei das Polymerteilchen ferner wenigstens eine an das Polymerteilchen gebundene oder darin eingebaute Verbindung oder eine Kombination davon umfasst, wobei es sich bei der Verbindung um ein Farb- oder Fluoreszenzmolekül, ein Radioisotop oder ein oder mehrere Metalle oder eine Kombination davon handelt.

**20.** Verfahren nach einem der Ansprüche 1 bis 19, wobei der Fusionspartner, der eine Zielkomponente bindet, ferner

(a) ein Antigen, eine antigene Determinante, ein Epitop oder ein Immunogen oder
(b) einen Antikörper oder ein Antikörperfragment oder
(c) eine Antikörper-Bindungsdomäne

umfasst.

**21.** Verfahren nach einem der Ansprüche 1 bis 20, wobei der Nachweis des Komplexes Zellsortierung, vorzugsweise fluoreszenzaktivierte Zellsortierung umfasst.

**22.** Verfahren nach einem der Ansprüche 1 bis 21, wobei der Fusionspartner ferner eine Antikörper-Bindungsdomäne, vorzugsweise Protein G oder Protein L umfasst.

**23.** Verfahren nach einem der Ansprüche 1 bis 22 zur Diagnose einer Krankheit oder eines Leidens.

**24.** Verfahren gemäß einem der Ansprüche 1 bis 22, wobei das Polymer aus Polybetaaminosäuren, Polylactaten, Polythioestern und Polyestern ausgewählt ist.

**25.** Verfahren gemäß einem der Ansprüche 1 bis 24, wobei es sich bei der Polymersynthase um eine Polyhydroxyalkanoat-Polymersynthase handelt.

## Revendications

**1.** Procédé de détection et éventuellement d'isolement d'au moins un composant cible, le procédé comprenant :

(a) la fourniture d'au moins une particule de polymère comprenant au moins un polypeptide de fusion, le polypeptide de fusion est produit *in vivo* dans une cellule hôte à partir d'une construction d'expression fonctionnellement liée à un promoteur fort de sorte qu'au moins 20 % de la surface des particules de polymère soit couverte par des polypeptides de fusion, le polypeptide de fusion étant lié de façon covalente ou non covalente à la particule de polymère, le polypeptide de fusion comprenant

(i) une polymère synthase qui comprend un domaine de liaison de particule de polymère, et
(ii) au moins un partenaire de fusion qui se lie à un composant cible,

(b) la mise en contact de la particule de polymère avec une composition comprenant un composant cible de sorte que le partenaire de fusion se lie au composant cible pour former un complexe,
(c) la détection et éventuellement l'isolement du complexe.

**2.** Procédé selon la revendication 1 dans lequel le partenaire de fusion qui se lie à un composant cible est la protéine A ou un domaine ZZ.

**3.** Procédé selon la revendication 1 ou 2 dans lequel le composant cible est choisi parmi une protéine, un fragment de protéine, un peptide, un polypeptide, un fragment de polypeptide, un anticorps, un fragment d'anticorps, un domaine de liaison d'anticorps, un antigène, un fragment d'antigène, un déterminant antigénique, un épitope, un haptène, un immunogène, un fragment d'immunogène, un métal, une molécule revêtue de métal, une biotine, un dérivé de biotine, l'avidine, la streptavidine, un inhibiteur, un cofacteur, un substrat, une enzyme, une abzyme, un récepteur, un fragment de récepteur, une sous-unité de récepteur, un fragment de sous-unité de récepteur, un ligand, un ligand de récepteur, un agoniste de récepteur, un antagoniste de récepteur, une molécule de signalisation, une protéine de signalisation, un fragment de protéine de signalisation, un facteur de croissance, un fragment de facteur de croissance, un facteur de transcription, un fragment de facteur de transcription, un inhibiteur, une cytokine, une chimiokine, un médiateur inflammatoire, un monosaccharide, un oligosaccharide, un polysaccharide, une glycoprotéine, un lipide, une cellule, une protéine de surface cellulaire, un lipide de surface cellulaire, un glucide de surface cellulaire, une glycoprotéine de surface cellulaire, un extrait de cellules, un virus, une protéine d'enveloppe virale, une hormone, une protéine sérique, une protéine de lait, une macromolécule, un médicament sujet à abus, un réactif de couplage, une polyhistidine, un agent pharmaceutiquement actif, un agent biologiquement actif, un marqueur, un réactif de couplage, un peptide de banque, une construction d'expression, un acide nucléique ou une combinaison de ceux-ci.

**4.** Procédé de la revendication 1, 2 ou 3, dans lequel l'au moins une particule de polymère est produite par un procédé comprenant :

A) la fourniture d'une cellule hôte comprenant au moins une construction d'expression fonctionnellement liée à un promoteur fort, la construction d'expression comprenant :

(1) au moins une séquence d'acide nucléique codant pour une polymère synthase ; ou
(2) au moins une séquence d'acide nucléique codant pour un polypeptide de fusion qui comprend une polymère synthase et au moins un partenaire de fusion ;

B) la culture de la cellule hôte dans des conditions adaptées pour l'expression de la construction d'expression et pour la formation de particules de polymère par la polymère synthase ; et
C) la séparation des particules de polymère des cellules hôtes cultivées pour produire une composition comprenant des particules de polymère.

**5.** Procédé selon la revendication 4 dans lequel la cellule hôte comprend en outre une construction d'expression additionnelle comprenant au moins une séquence d'acide nucléique codant pour un polypeptide de fusion additionnel, le polypeptide de fusion comprenant

(a) un domaine de liaison de particule de polymère, un polypeptide qui comprend un domaine de liaison de particule de polymère ou une protéine formant une particule qui comprend un domaine de liaison de particule de polymère, et
(b) au moins un partenaire de fusion.

**6.** Procédé selon l'une quelconque des revendications 1 à 5 dans lequel le promoteur fort est un promoteur viral ou un promoteur de phage, de préférence un procédé de phage T7.

**7.** Procédé selon l'une quelconque des revendications 4 à 7, dans lequel le processus de production de l'au moins une particule de polymère comprend en outre l'ajout d'au moins un polypeptide ou au moins une substance ou une combinaison de ceux-ci pendant la culture des cellules hôtes de sorte que l'au moins un polypeptide ou au moins une substance se lie à ou soit incorporé dans les particules de polymère ou comprenant en outre la mise en contact des particules de polymère avec l'ajout d'au moins un polypeptide ou au moins une substance ou une combinaison de ceux-ci qui se lie à ou est adsorbée dans les particules de polymère.

**8.** Procédé selon la revendication 7 dans lequel la substance est une substance lipophile qui est incorporée dans ou adsorbée dans les particules de polymère ; ou une molécule colorée ou fluorescente, un radio-isotope, un ou plusieurs métaux ou une combinaison de ceux-ci.

**9.** Procédé de l'une quelconque des revendications 1 à 8 dans lequel l'au moins une particule de polymère comprend une composition comprenant une pluralité de particules de polymère ayant un diamètre moyen inférieur à environ 200 nm, ou inférieur à environ 150 ou 110 nm.

**10.** Procédé selon l'une quelconque des revendications 1 à 11 dans lequel au moins environ 30, 40 ou 50 % de la surface des particules de polymère est couverte par les protéines liées à la surface.

**11.** Procédé selon la revendication 9 dans lequel la composition comprend en outre au moins une substance liée à ou incorporée dans les particules de polymère ou une combinaison de ceux-ci.

**12.** Procédé selon la revendication 11 dans lequel la substance est une substance lipophile ; ou une molécule colorée ou fluorescente, un radio-isotope, un ou plusieurs métaux, ou une combinaison de ceux-ci.

**13.** Procédé selon l'une quelconque des revendications 1 à 12 dans lequel le partenaire de fusion est choisi parmi une protéine, un fragment de protéine, un domaine de liaison, un domaine de liaison de cible, une protéine de liaison, un fragment de protéine de liaison, un anticorps, un fragment d'anticorps, une chaîne lourde d'anticorps, une chaîne légère d'anticorps, un anticorps monocaténaire, un anticorps à domaine unique, un fragment d'anticorps Fab, un fragment d'anticorps Fc, un fragment d'anticorps Fv, un fragment d'anticorps F(ab')2, un fragment d'anticorps Fab', un fragment d'anticorps Fv monocaténaire (scFv), un domaine de liaison d'anticorps, un antigène, un déterminant antigénique, un épitope, un haptène, un immunogène, un fragment d'immunogène, une biotine, un dérivé de biotine, une avidine, une streptavidine, un substrat, une enzyme, une abzyme, un cofacteur, un récepteur, un fragment de récepteur, une sous-unité de récepteur, un fragment de sous-unité de récepteur, un ligand, un inhibiteur, une hormone, une lectine, une polyhistidine, un domaine de couplage, un domaine de liaison d'ADN, un épitope FLAG, un résidu cystéine, un peptide de banque, un peptide rapporteur, et un peptide de purification d'affinité, ou une combinaison de ceux-ci.

**14.** Procédé selon l'une quelconque des revendications 1 à 13 dans lequel

(a) un réactif de couplage est lié au partenaire de fusion, ou
(b) un réactif de couplage est lié au partenaire de fusion et une substance est liée au réactif de couplage, ou
(c) une substance est liée au partenaire de fusion, ou
(d) la polymère synthase ou la protéine formant une particule a été chimiquement modifiée avec un réactif de couplage pour former au moins un domaine de liaison, ou
(e) une combinaison de ceux-ci.

**15.** Procédé selon l'une quelconque des revendications 1 à 13 dans lequel le partenaire de fusion comprend un domaine de liaison et

(a) un réactif de couplage est lié au domaine de liaison, ou
(b) un réactif de couplage est lié au domaine de liaison et une substance est liée au réactif de couplage, ou
(c) une substance est liée au domaine de liaison,
(d) ou une combinaison de ceux-ci.

**16.** Procédé selon l'une quelconque des revendications 1 à 15 dans lequel la détection du complexe comprend la mise en contact de la particule de polymère avec un marqueur qui se lie au complexe, à l'au moins un polypeptide de fusion ou à la particule de polymère, et la détection du marqueur.

**17.** Procédé selon l'une quelconque des revendications 1 à 16 dans lequel la particule de polymère comprend

(a) un marqueur lié à ou incorporé dans la particule de polymère, ou
(b) au moins un polypeptide de fusion additionnel comprenant

(i) un domaine de liaison de particule de polymère, un polypeptide qui comprend un domaine de liaison de particule de polymère ou une protéine formant une particule qui comprend un domaine de liaison de particule de polymère, et
(ii) un partenaire de fusion qui se lie à une molécule marquée, ou

(c) au moins un polypeptide de fusion additionnel comprenant une protéine rapporteuse, ou
(d) une combinaison quelconque de deux ou plus de ceux-ci.

**18.** Procédé selon l'une quelconque des revendications 1 à 17 dans lequel la particule de polymère comprend deux polypeptides de fusion différents ou plus.

**19.** Procédé selon l'une quelconque des revendications 1 à 18 dans lequel la particule de polymère comprend en outre au moins un composé lié à ou incorporé dans la particule de polymère, ou une combinaison de ceux-ci, le composé étant une molécule colorée ou fluorescente, un radio-isotope, ou un ou plusieurs métaux, ou une combinaison de ceux-ci.

**20.** Procédé selon l'une quelconque des revendications 1 à 19 dans lequel le partenaire de fusion qui se lie à un composant cible comprend en outre

(a) un antigène, un déterminant antigénique, un épitope ou un immunogène, ou
(b) un anticorps ou un fragment d'anticorps, ou
(c) un domaine de liaison d'anticorps.

**21.** Procédé selon l'une quelconque des revendications 1 à 20, dans lequel la détection du complexe comprend le tri de cellules, de préférence le tri de cellules activé par fluorescence.

**22.** Procédé selon l'une quelconque des revendications 1 à 21, dans lequel le partenaire de fusion comprend en outre un domaine de liaison d'anticorps, de préférence la protéine G ou la protéine L.

**23.** Procédé selon l'une quelconque des revendications 1 à 22 pour le diagnostic d'une maladie ou affection.

**24.** Procédé de l'une quelconque des revendications 1 à 22 dans lequel le polymère est choisi parmi des poly(acides $\beta$-aminés), des polylactates, des polythioesters et des polyesters.

**25.** Procédé de l'une quelconque des revendications 1 à 24 dans lequel la polymère synthase est une polyhydroxyalcanoate polymère synthase.

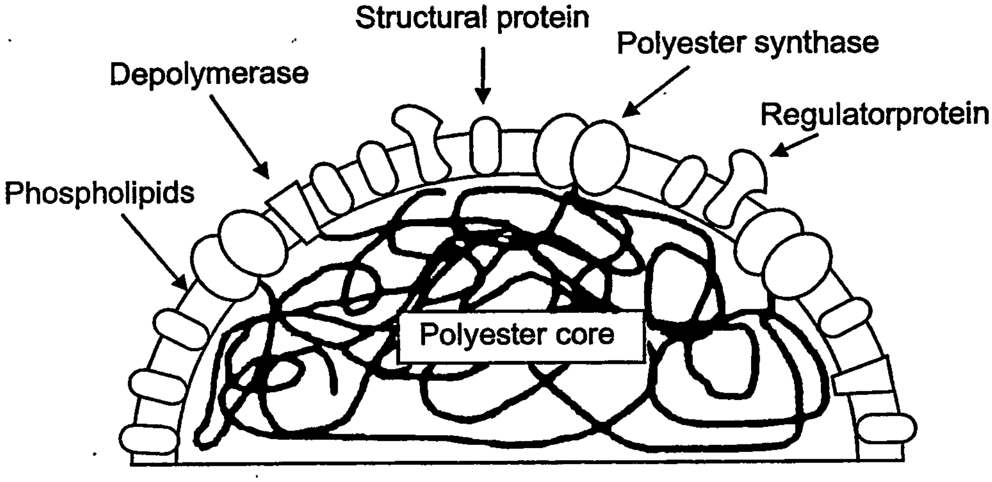


# FIGURE 1

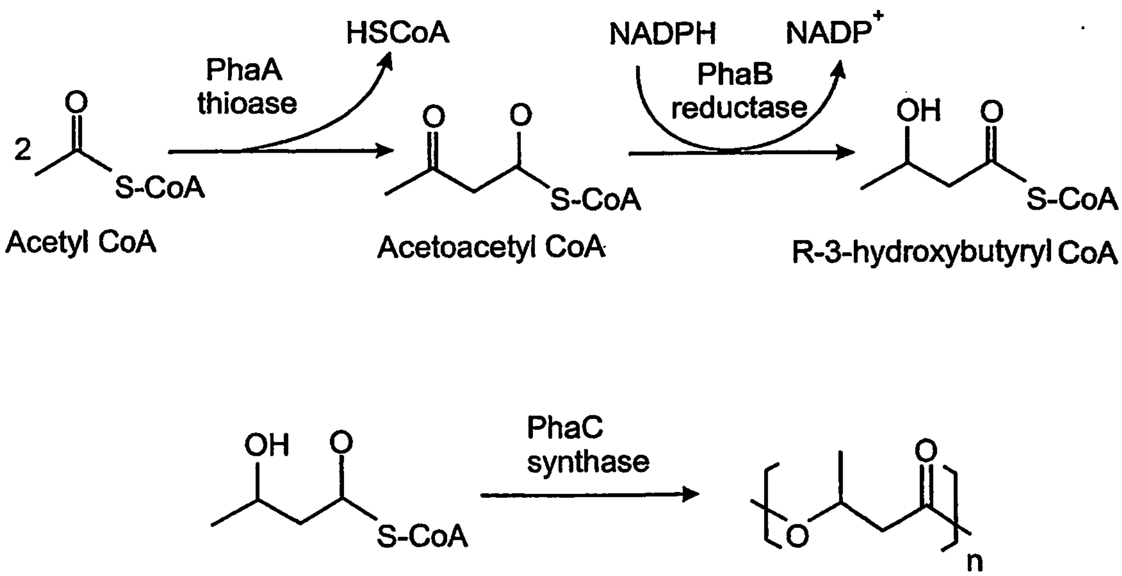


Acetyl CoA

Acetoacetyl CoA

R-3-hydroxybutyryl CoA

# FIGURE 2

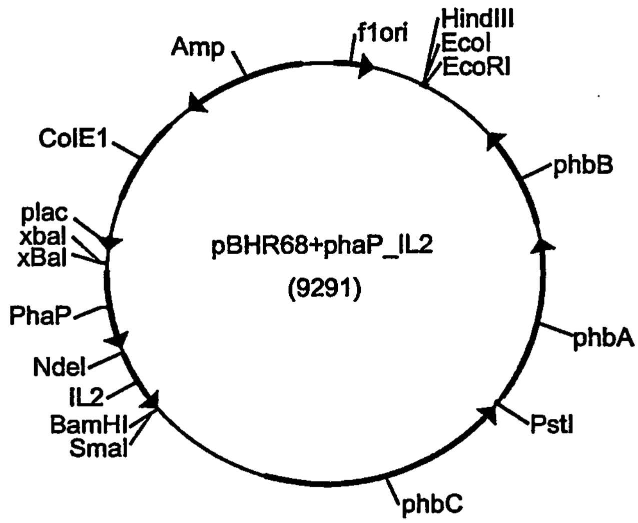


# FIGURE 3

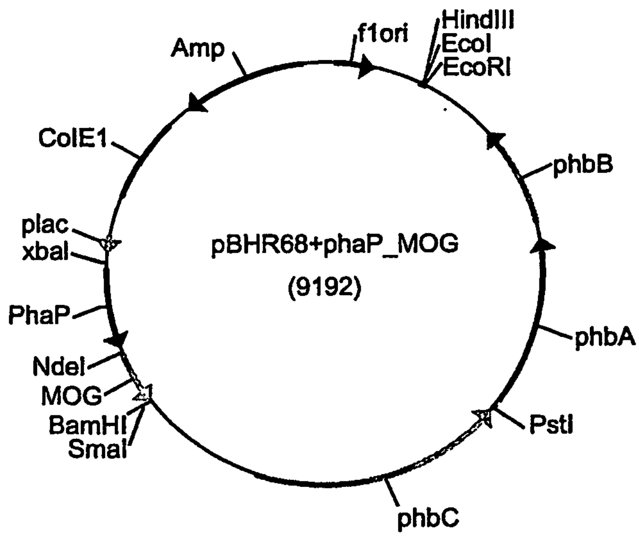


# FIGURE 4

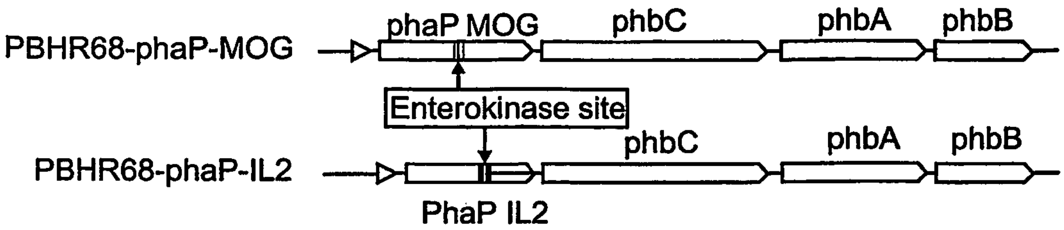


# FIGURE 5

# FIGURE 6

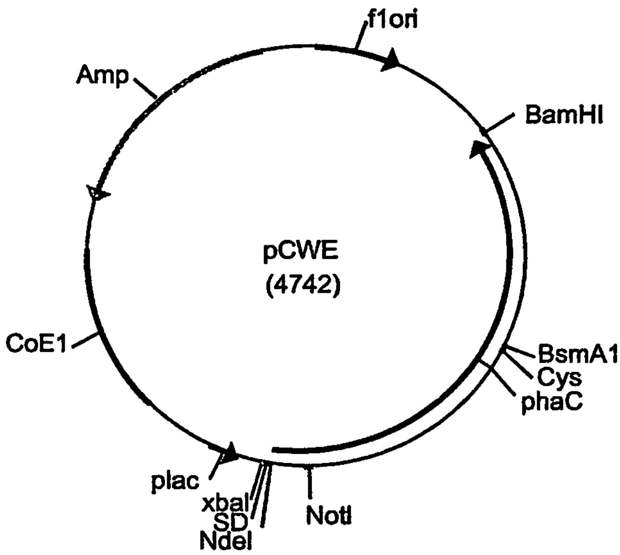


# FIGURE 7

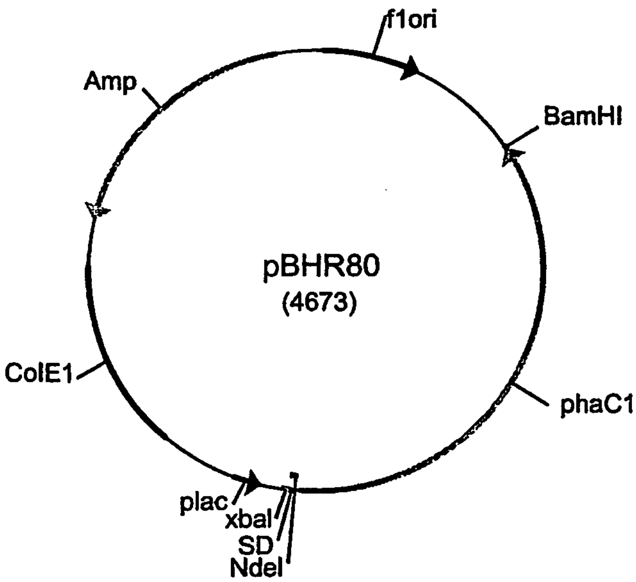

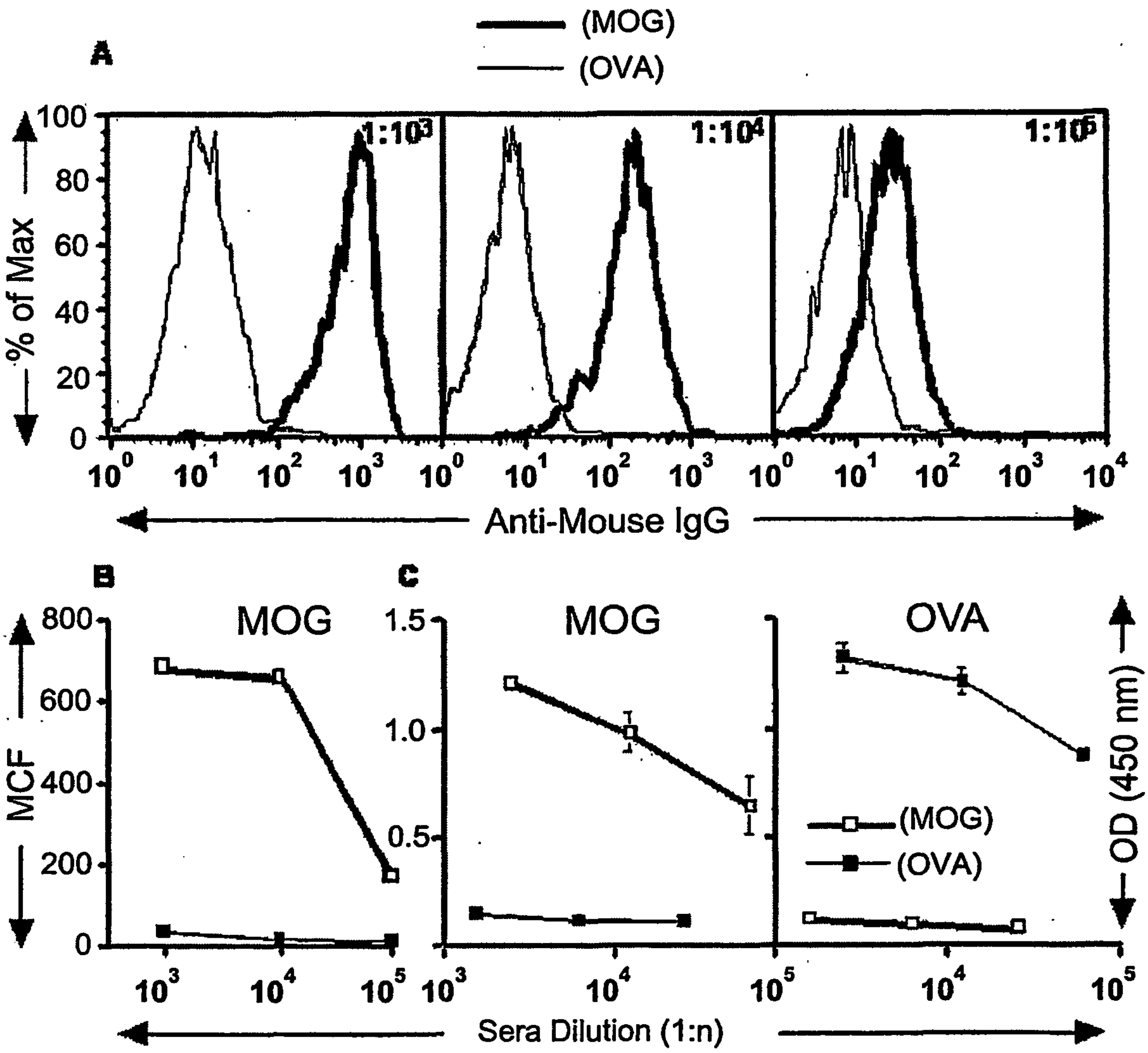
A
(MOG)
(OVA)
1:10³
1:10⁴
1:10⁵
% of Max
100
80
60
40
20
0
10⁰ 10¹ 10² 10³ 10⁰ 10¹ 10² 10³ 10⁰ 10¹ 10² 10³ 10⁴
Anti-Mouse IgG
B
C
MOG
MOG
OVA
800
600
400
200
0
MCF
1.5
1.0
0.5
(MOG)
(OVA)
OD (450 nm)
10³ 10⁴ 10⁵ 10³ 10⁴ 10⁵ 10⁴ 10⁵
Sera Dilution (1:n)

FIGURE 8

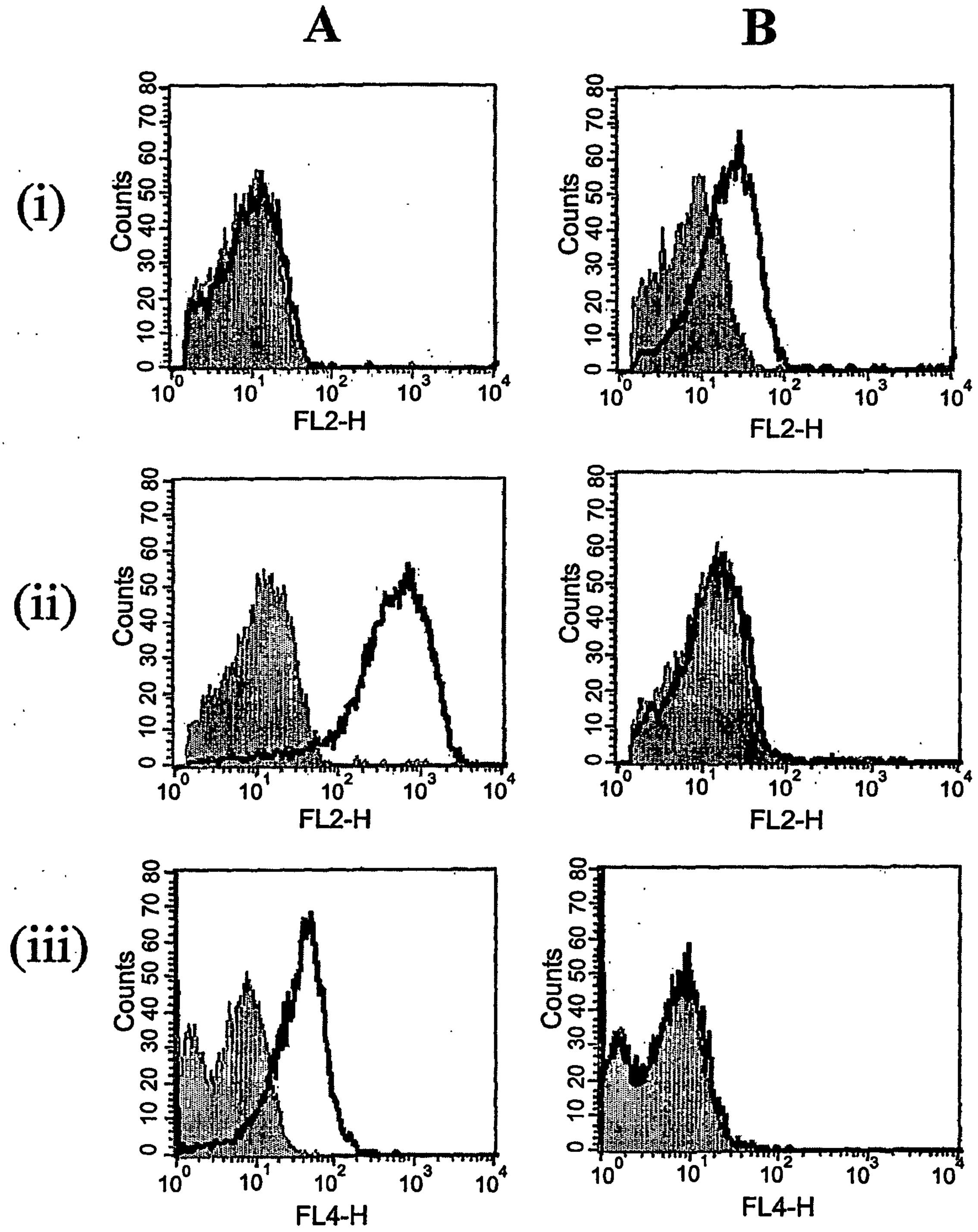
A
B
(i)
(ii)
(iii)
Counts
FL2-H
FL4-H
0
10
20
30
40
50
60
70
80
$10^0$
$10^1$
$10^2$
$10^3$
$10^4$

# FIGURE 9

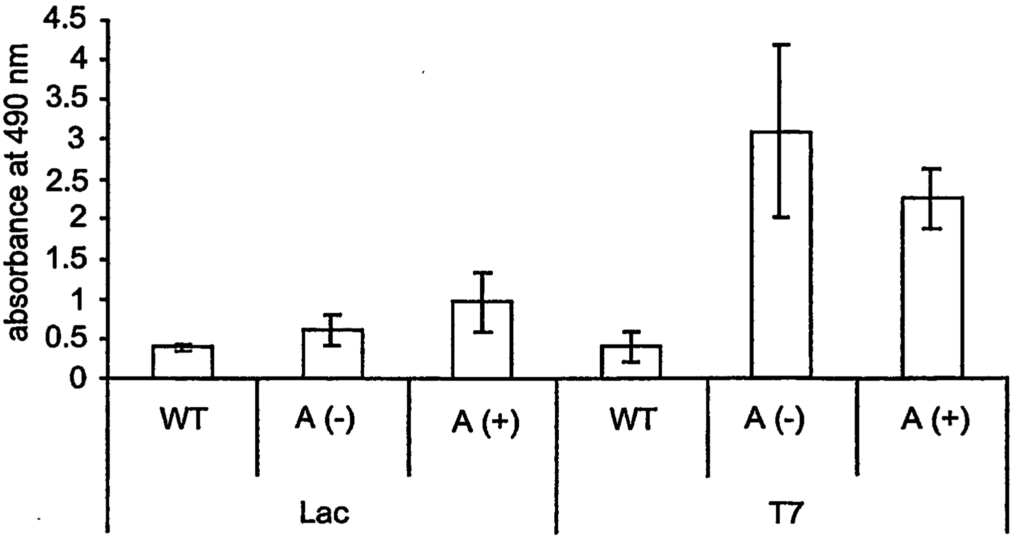


FIGURE 10

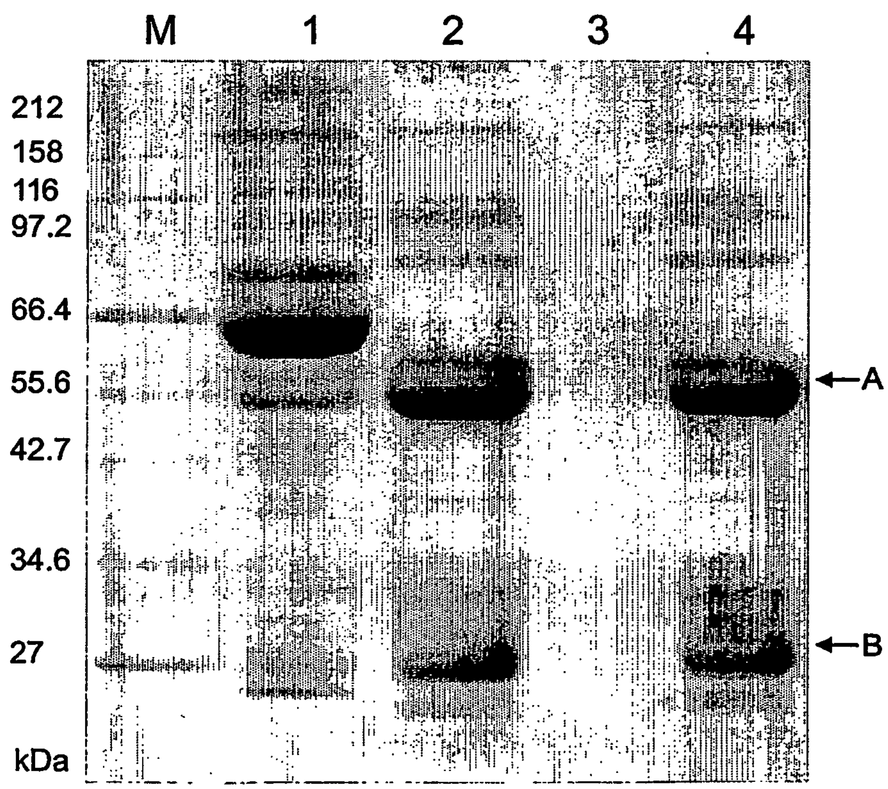


FIGURE 11

(i)

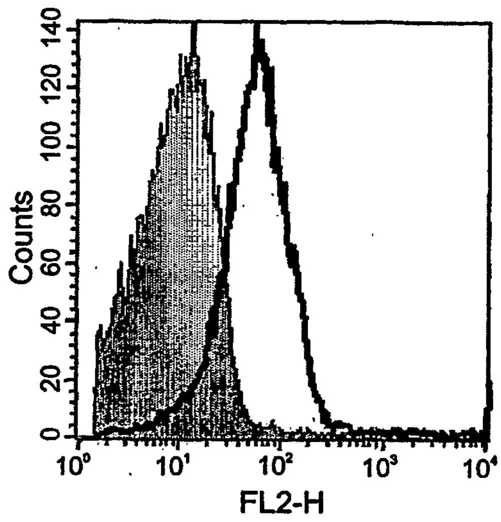


(ii)

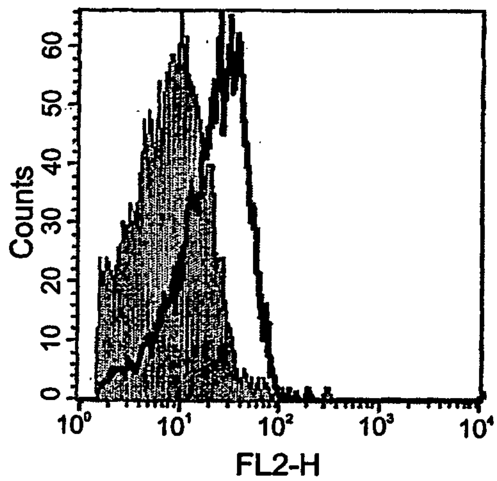


(iii)

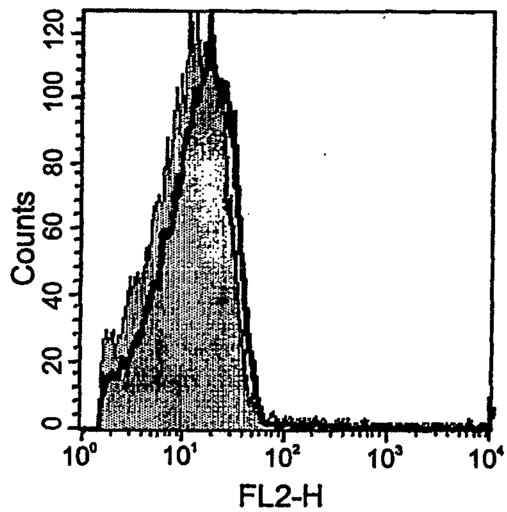


**FIGURE 12**

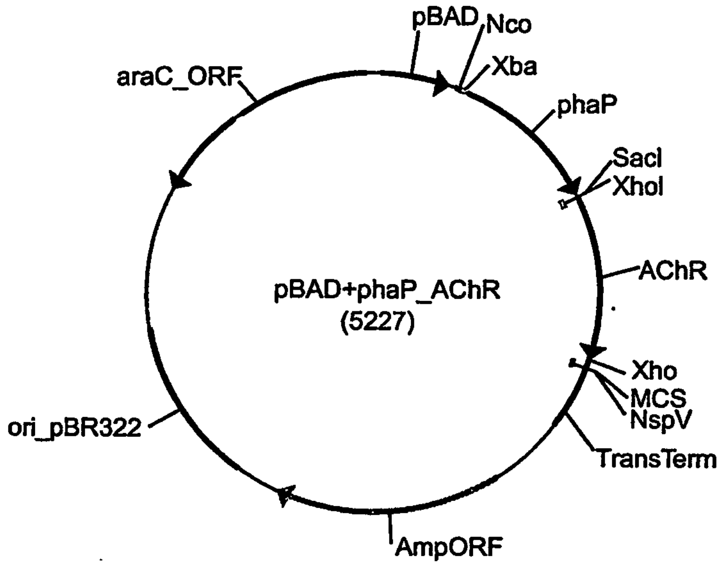


# FIGURE 13

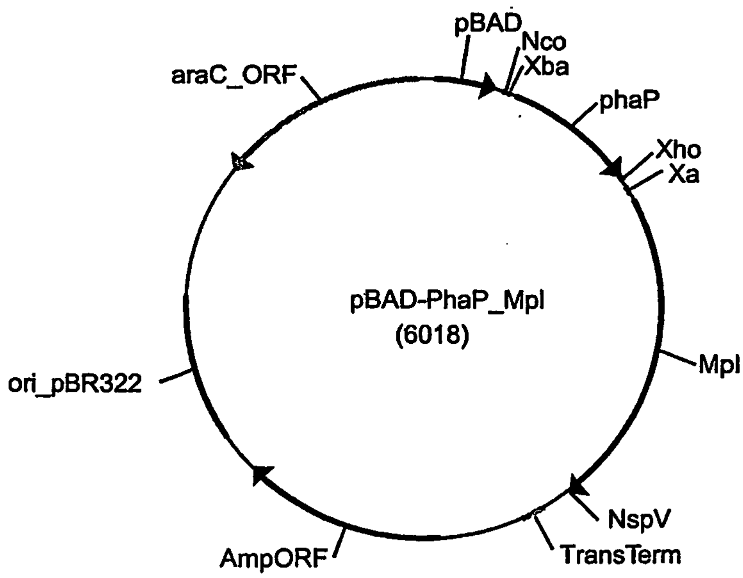


# FIGURE 14

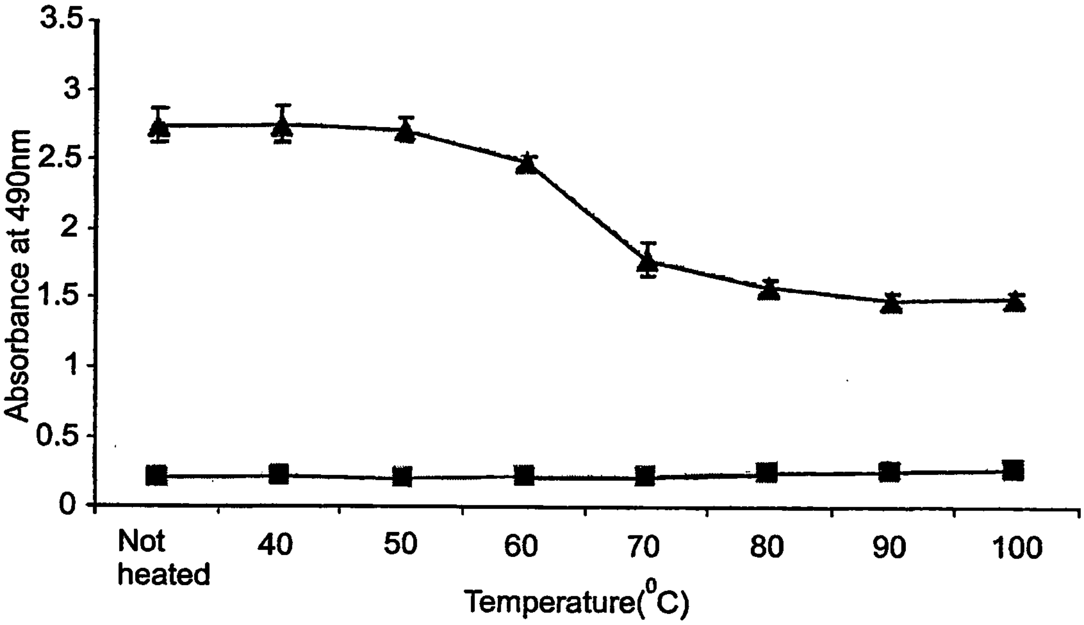


FIGURE 15

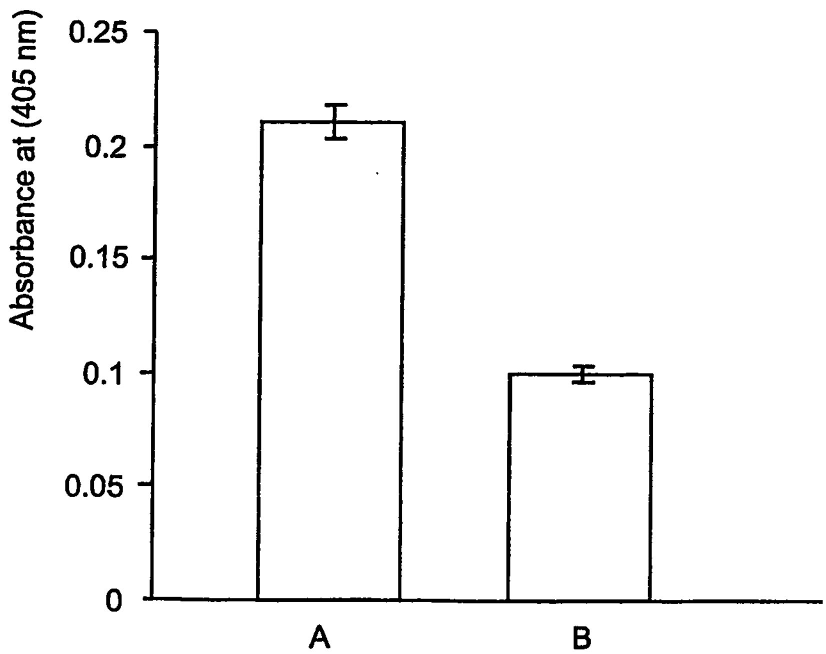


FIGURE 16

(i)

(ii)

(iii)

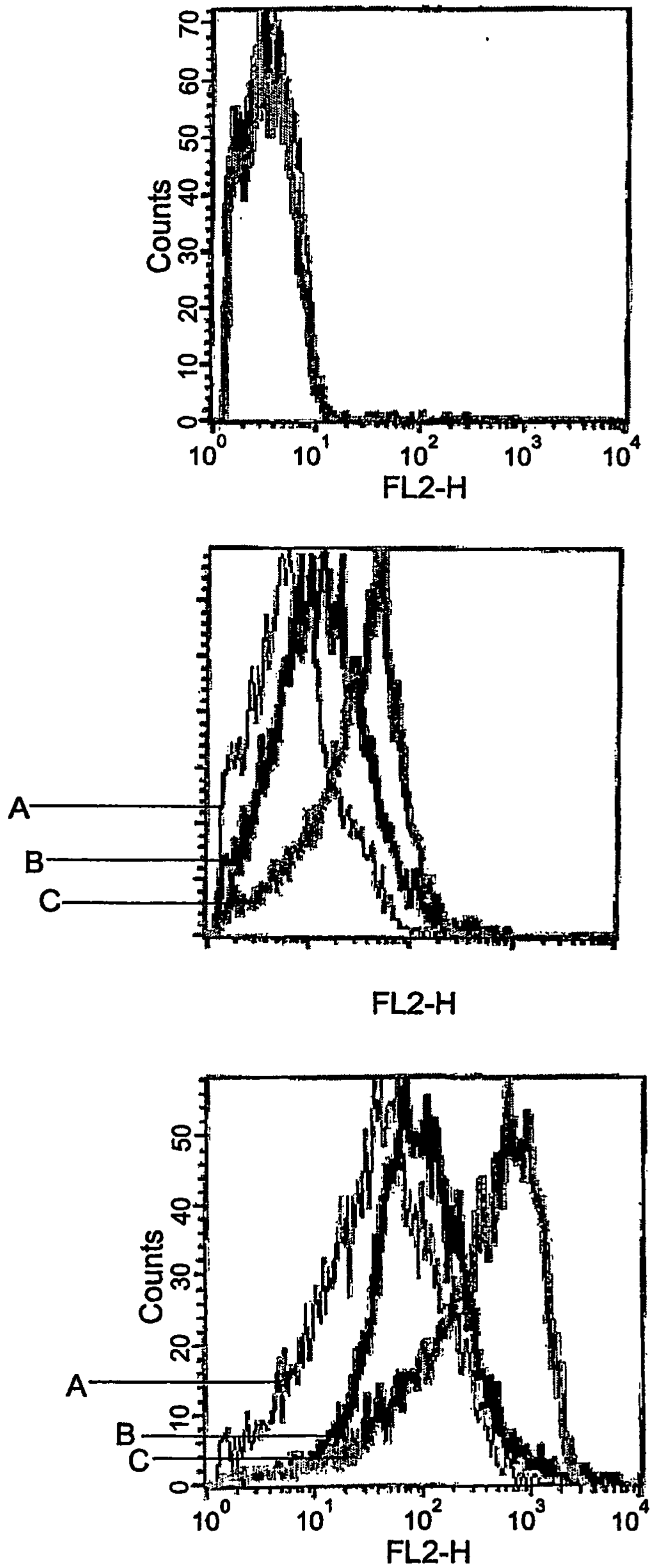


FIGURE 17

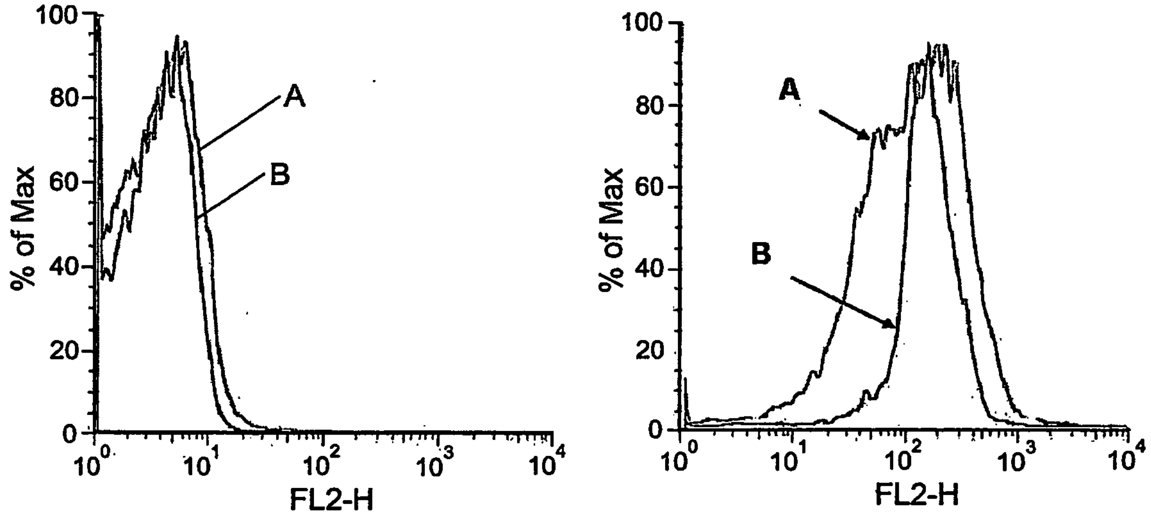


# FIGURE 18

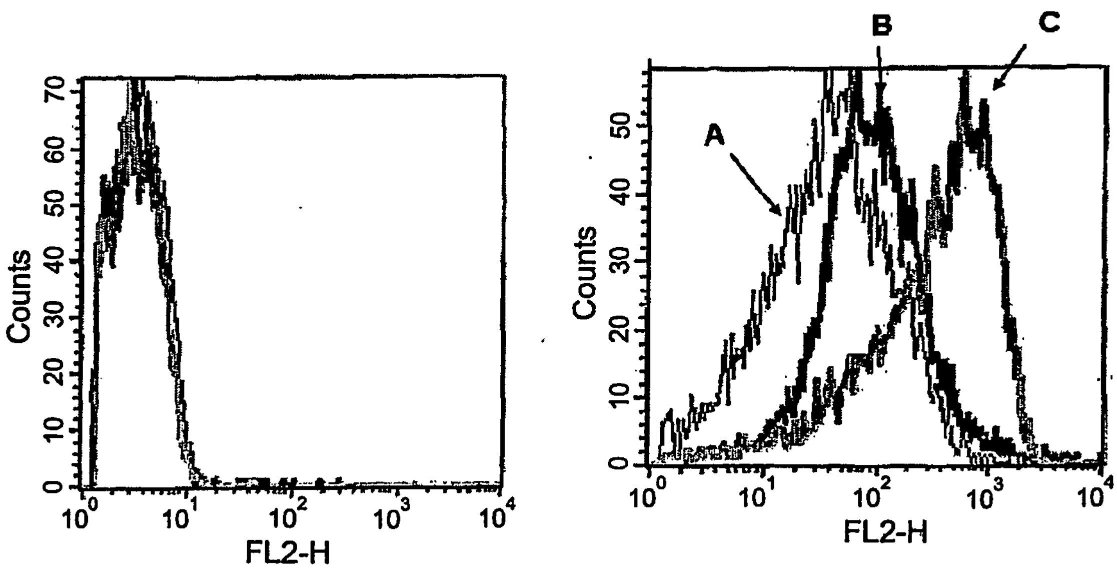


# FIGURE 19

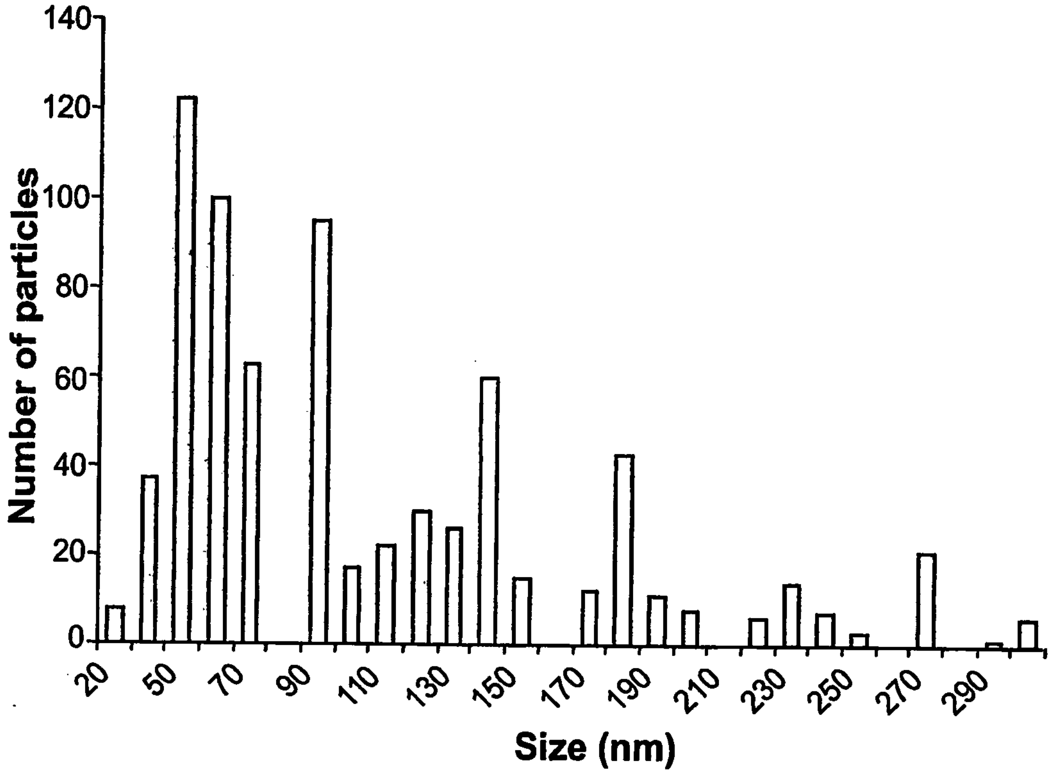
Number of particles
140
120
100
80
60
40
20
0
20
50
70
90
110
130
150
170
190
210
230
250
270
290
Size (nm)

FIGURE 20

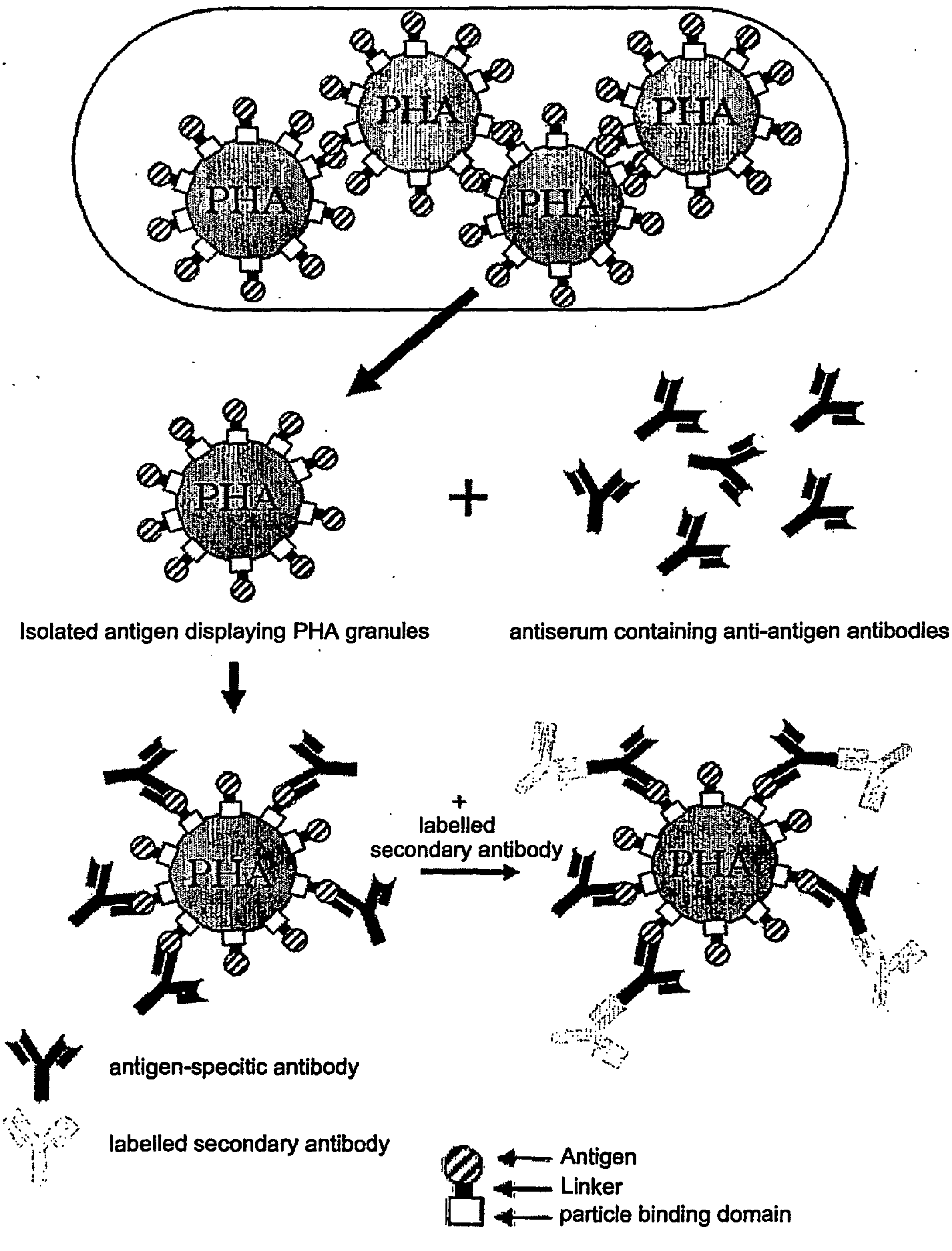
PHA
PHA
PHA
PHA
PHA
Isolated antigen displaying PHA granules
antiserum containing anti-antigen antibodies
PHA
+
labelled
secondary antibody
PHA
antigen-specitic antibody
labelled secondary antibody
Antigen
Linker
particle binding domain

# FIGURE 21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 04020623 A, Rehm
- WO 2004048922 A
- WO 2004020623 A
- EP 36776 A
- WO 0200894 A
- EP 139383 A
- US 4943529 A
- EP 402226 A
- EP 183070 A
- EP 244234 A
- EP 394538 A
- WO 9100357 A
- US 6232107 B


### Non-patent literature cited in the description


- Construction of a functional Slayer fusion protein comprising an immunoglobulin G-binding domain for development of specific adsorbents for extracorporeal blood purification. **VOLLENKLE C et al.** APPLIED AND ENVIRONMENTAL MICROBIOLOGY. AMERICAN SOCIETY FOR MICROBIOLOGY, 01 March 2004, vol. 70, 1514-1521
- **REHM B. H. A.** *Biochem J.,* 2003, vol. 376 (1), 15-33
- **PETERS, V. ; REHM, B.H.A.** *FEMS Microbiol. Lett.,* 2005, vol. 248, 93-100
- **QI Q. ; STEINBÜCHEL A. ; REHM B.H.A.** *Appl. Microbiol. Biotechnol.,* 2000, vol. 54, 37-43
- *International Immunology,* 2000, vol. 12 (9), 1255-1265
- *Biol. Pharm. Bull.,* 2004, vol. 27 (2), 219-221
- **HANLEY, S.Z. et al.** *FEBS Letters,* 1999, vol. 447, 99-105
- **MOLDES C. et al.** *Appl Environ Microbiol.,* June 2004, vol. 70 (6), 3205-12
- **SAEGUSA, H. et al.** *J. Bacteriol.,* 2001, vol. 183 (1), 94-100
- **PRIETO, M.A. et al.** *J. Bacteriol.,* 1999, vol. 181 (3), 858-868
- **REHM, B.** *Biotechnol Lett.,* February 2006, vol. 28 (4), 207-13
- **TATIANA A. TATUSOVA ; THOMAS L. MADDEN.** Blast 2 sequences - a new tool for comparing protein and nucleotide sequences. *FEMS Microbiol Lett.,* 1999, vol. 174, 247-250
- **NEEDLEMAN, S. B. ; WUNSCH, C. D.** *J. Mol. Biol.,* 1970, vol. 48, 443-453
- **RICE,P. ; LONGDEN,I. ; BLEASBY,A.** EMBOSS: The European Molecular Biology Open Software Suite. *Trends in Genetics,* June 2000, vol. 16 (6), 276-277
- **HUANG, X.** On Global Sequence Alignment. *Computer Applications in the Biosciences,* 1994, vol. 10, 227-235
- Molecular Cloning, A Laboratory Manual. Cold Spring Harbor Press, 1987
- **AUSUBEL et al.** Current Protocols in Molecular Biology. Greene Publishing, 1987
- **BOLTON ; MCCARTHY.** *PNAS,* 1962, vol. 84, 1390
- **NIELSEN et al.** *Science,* 06 December 1991, vol. 254 (5037), 1497-500
- **GIESEN et al.** *Nucleic Acids Res.,* 01 November 1998, vol. 26 (21), 5004-6
- **BOWIE et al.** *Science,* 1990, vol. 247, 1306
- **SAMBROOK et al.** Molecular Cloning: A Laboratory Manual. Cold Spring Harbor Press, 1987
- **URLAUB et al.** *Proc. Natl. Acad. Sci. USA,* 1980, vol. 77, 4216
- **STINCHCOMB et al.** *Nature,* 1979, vol. 282, 39
- **KINGSMAN et al.** *Gene,* 1979, vol. 7, 141
- **TSCHEMPER et al.** *Gene,* 1980, vol. 10, 157
- **JONES.** *Genetics,* 1977, vol. 85, 12
- **CHANG et al.** *Nature,* 1978, vol. 275, 615
- **GOEDDEL et al.** *Nature,* 1989, vol. 281, 544
- **GOEDDEL.** *Nucleic Acids Res.,* 1980, vol. 8, 4057
- **DEBOER et al.** *Proc. Natl. Acad. Sci. USA,* 1983, vol. 80, 21-25
- **HITZEMAN et al.** *J. Biol. Chem.,* 1980, vol. 255, 2073
- **HESS et al.** *J. Adv. Enzyme Reg.,* 1968, vol. 7, 149
- **HOLLAND.** *Biochemistry,* 1978, vol. 17, 4900

- Parameters Influencing the Productivity of Recombinant E. coli Cultivations. **FRIEHS ; REARDON.** Advances in Biochemical Engineering Technology. Springer Verlag, 1991, vol. 48
- **BRUNSCHWIG ; DARZINS.** *Gene,* 1992, vol. 111, 35-41
- **BARNARD et al.** *Protein Expr Purif.,* December 2004, vol. 38 (2), 264-71
- **LEUNG, D. W. et al.** *Technique,* 1989, vol. 1, 11-15
- **CALDWELL, R. C. ; JOYCE G. F.** *PCR Methods Applic.,* 1992, vol. 2, 28-33
- **REIDHAAR-OLSON, J. F. ; SAUER, R. T. et al.** *Science,* 1988, vol. 241, 53-57
- **STEMMER, W. P.** *PNAS, USA,* 1994, vol. 91, 10747-10751
- **ARKIN, A. P. ; YOUVAN, D. C.** *PNAS, USA,* 1992, vol. 89, 7811-7815
- **DELEGRAVE, S. ; YOUVAN, D. C.** *Biotechnology Research,* 1993, vol. 11, 1548-1552
- **ARNOLD, F. H.** *Current Opinion in Biotechnology,* 1993, vol. 4, 450-455
- **SELIGER H ; HINZ M ; HAPP E.** Arrays of immobilized oligonucleotides--contributions to nucleic acids technology. *Curr Pharm Biotechnol.,* 2003, vol. 4 (6), 379-95
- **GAO X ; GULARI E ; ZHOU X.** In situ synthesis of oligonucleotide microarrays. *Biopolymers,* 2004, vol. 73 (5), 579-96
- **MADISON, L. L. et al.** Metabolic Erigineering of Poly(3-hydroxyalkanoates): From DNA to Plastic. *Microbiology and Molecular Biology Reviews,* 1999, vol. 63 (1), 21-53
- **REHM B. H. A.** Polyester synthases: natural catalysts for plastics. *Biochem J.,* 2003, vol. 376 (1), 15-33
- **BROCKELBANK JA. et al.** *Appl Environ Microbiol.,* 25 August 2006
- **PETERS V ; REHM BH.** *Appl Environ Microbiol.,* March 2006, vol. 72 (3), 1777-83
- Biopolyester particles produced by microbes or using polyester synthases: self assembly and potential applications. **REHM BHA.** Microbial Biotechnology: biological self-assembly systems and biopolymer-based nanostructures. Horizon Bioscience, 2006
- **BEACH ; NURSE.** *Nature,* 1981, vol. 290, 140
- **FLEER et al.** *Bio/Technology,* 1991, vol. 9, 968-975
- **LOUVENCOURT et al.** *J. Bacteriol.,* 1983, vol. 154 (2), 737-742
- **VAN DEN BERG et al.** *Bio/Technology,* 1990, vol. 8, 135
- **SREEKRISHNA et al.** *J. Basic Microbiol.,* 1988, vol. 28, 265-278
- **CASE et al.** *Proc. Natl. Acad. Sci. USA,* 1979, vol. 76, 5259-5263
- **BALLANCE et al.** *Biochem. Biophys. Res. Commun.,* 1983, vol. 112, 284-289
- **TILBURN et al.** *Gene,* 1983, vol. 26, 205-221
- **YELTON et al.** *Proc. Natl. Acad. Sci. USA,* 1984, vol. 81, 1470-1474
- **KELLY ; HYNES.** *EMBO J.,* 1985, vol. 4, 475-479
- **C. ANTHONY.** *The Biochemistry of Methylotrophs,* 1982, 269
- **GRAHAM et al.** *J. Gen Virol.,* 1977, vol. 36, 69
- **MATHER.** *Biol. Reprod.,* 1980, vol. 23, 243-251
- **MATHER et al.** *Annals N.Y. Acad. Sci.,* 1982, vol. 383, 44-68
- **DONINI et al.** *Journal of Molecular Biology,* 2003, vol. 330, 323-332
- **WOERN ; PLUECKTHUN.** *FEBS Letters,* 1998, vol. 427, 357-361
- **SHAKI-LOEWENSTEIN et al.** *Journal of Immunological Methods,* 2005
- **MARTINEAU et al.** *Journal of Molecular Biology,* 1998, vol. 280, 117-127
- **CODY et al.** *Biochemistry,* 1993, vol. 32 (5), 1212-8
- **ORMO et al.** *Science,* 1996, vol. 273 (5280), 1392-6
- **PRASHER et al.** *Gene,* 1992, vol. 111 (2), 229-33
- **KENNEDY et al.** *Journal of Biological Chemistry,* 1999, vol. 274, 13281-91
- **NOLAN et al.** *Proceedings of the National Academy of Sciences,* 1988, vol. 85, 2603-7
- **JEFFERSON et al.** *EMBO Journal,* 1987, vol. 6 (13), 3901-7
- **CULLEN et al.** *Methods in Enzymology,* 1992, vol. 216, 362-8
- **WILKINSON ; HARRISON.** *Nature Bio/Technology,* 1991, vol. 9, 443-448
- **WITTRUP et al.** *Nature Bio/Technology,* 1988, vol. 6, 423-426
- **KOSCHORRECK M. et al.** *BMC Genomics,* 2005, vol. 6, 49-59
- *Nature Bio/Technology,* 1991, vol. 9, 443-448
- **PERLER, F. B.** *Nucleic Acids Res.,* 2002, vol. 30, 383-384
- **YUAN et al.** *Arch Biochem Biophys.,* 01 October 2001, vol. 394 (1), 87-98
- **SPIEKERMANN et al.** *Arch Microbiol.,* 1999, vol. 171 (2), 73-80
- **HOFFMANN, N. ; AMARA, A.A. ; BR. BEERMANN ; QI, Q., B. ; HINZ, H.-J. ; REHM, B.H.A.** *J. Biol. Chem,* 2002, vol. 277, 42926-42936
- **PETERS, V. ; B. H. A. REHM.** *FEMS Microbiol. Lett.,* 2005, vol. 248, 93-100
- **PETERS, V. ; B. H. A. REHM.** *Appl. Environ. Microbiol.,* 2006, vol. 72, 1777-83

- **SØRENSEN HP ; SPERLING-PETERSEN HU ; MORTENSEN KK.** *Protein Exp Purif.,* December 2003, vol. 32 (2), 252-259
- Reconstitution of conformationally dependent epitopes on the N-terminal extracellular domain of the human muscle acetylcholine receptor alpha subunit expressed in Escherichia coli: implications for myasthenia gravis therapeutic approaches. *International Immunology,* 2000, vol. 12 (9), 1255-1265
- Expression of the Soluble Extracellular Domain of Human Thrombopoietin Receptor Using a Maltose-Binding Protein-Affinity Fusion System. *Biol. Pharm. Bull.,* 2004, vol. 27 (2), 219-221
- **REHM, B.H.A. et al.** *Biochem. Biophys. Acta,* 2002, vol. 1594, 178-190

SEQUENCE LISTING

<110> Rehm, Bernd
Backstrom, Bjorn

<120> Polymer Particles and uses thereof

<130> 536268

<160> 24

<170> PatentIn version 3.3

<210> 1
<211> 468
<212> DNA
<213> synthetic

<400> 1

```
tctagacata tggcaccaac gtccagctcg accagcagtt ctaccgcaga agcccagcag      60
cagcagcagc agcagcaaca gcagcagcaa catctggaac agctgctgat ggatctgcag     120
gaactgctga gtcgtatgga aaattatcgt aatctgaaac tgccacgtat gctgacgttt     180
aaattctatc tgccaaaaca ggctacggaa ctgaaagatc tgcagtgcct ggaagatgaa     240
ctgggtccac tgcgtcacgt cctggatctg acccagtcca aatcctttca gctggaagac     300
gctgaaaatt ttatctccaa tatccgtgtg acggtggtga aactgaaagg ttctgacaac     360
acgtttgaat gccaatttga tgacgaatca gctaccgtgg tcgattttct gcgtcgttgg     420
atcgcctttt gccagtcgat tatttcgaca agcccacagt aaggatcc                  468
```

<210> 2
<211> 372
<212> DNA
<213> murine

<400> 2

tctagacata tgggtcagtt tcgtgtgatt ggcccaggtt atccaatccg tgcactggta 60
ggtgatgagg cagaactgcc atgtcgtatt tccccaggca aaaatgctac cggtatggaa 120
gtaggttggt atcgttcccc attttcccgt gtggtgcatc tgtatcgtaa cggcaaagat 180
caggatgccg aacaggcccc agaatatcgt ggtcgtacag agctgctgaa agaaacgatt 240
agcgaaggca aagttactct gcgtattcag aatgtgcgtt ttagcgatga aggtggctat 300
acctgtttct tccgtgatca ctcttaccag gaagaagccg ccatggaact gaaagttgaa 360
gattaaggat cc 372

<210> 3
<211> 49
<212> DNA
<213> synthetic

<400> 3
aaaaatctag aaaaaggaga tatacgtatg atcctcaccc cggaacaag 49

<210> 4
<211> 66
<212> DNA
<213> synthetic

<400> 4

aaaaacatat ggtggtgatg gtgatgcgag ccgcgtttat catcatcatc ggcagccgtc 60
gtcttc 66

<210> 5
<211> 653
<212> DNA
<213> Ralstonia eutropha

<400> 5

aaaaatctag aaaaaggaga tatacgtatg atcctcaccc cggaacaagt tgcagcagcg 60
caaaaggcca acctcgaaac gctgttcggc ctgaccacca aggcgtttga aggcgtcgaa 120
aagctcgtcg agctgaacct gcaggtcgtc aagacttcgt tcgcagaagg cgttgacaac 180
gccaagaagg cgctgtcggc caaggacgca caggaactgc tggccatcca ggccgcagcc 240
gtgcagccgg ttgccgaaaa gaccctggcc tacacccgcc acctgtatga aatcgcttcg 300
gaaacccaga gcgagttcac caaggtagcc gaggctcaac tggccgaagg ctcgaagaac 360
gtgcaagcgc tggtcgagaa cctcgccaag aacgccccgg ccggttcgga atcgaccgtg 420
gccatcgtga agtcggcgat ctccgctgcc aacaacgcct acgagtcggt gcagaaggcg 480
accaagcaag cggtcgaaat cgctgaaacc aacttccagg ctgcggctac ggctgccacc 540
aaggctgccc agcaagccag cgccacggcc cgtacggcca cggcaaagaa gacgacggct 600
gccgatgatg atgataaacg cggctcgcat caccatcacc accatatgtt ttt 653

<210> 6
<211> 38
<212> DNA
<213> synthetic

<400> 6
gtaatcatat ggggtaccga gctcgaattc gcgtctac 38

<210> 7
<211> 44
<212> DNA
<213> synthetic

<400> 7
gcgcgcatat gactttacaa atacatacag ggggtattaa tttg 44

<210> 8
<211> 38
<212> DNA
<213> synthetic

<400> 8
gtacacatat ggcgcaacac gatgaagccg tagacaac 38

<210> 9
<211> 415
<212> DNA
<213> synthetic

<400> 9

gtacacatat ggcgcaacac gatgaagccg tagacaacaa attcaacaaa gaacaacaaa 60
acgcgttcta tgagatctta catttaccta acttaaacga agaacaacga aacgccttca 120
tccaaagttt aaaagatgac ccaagccaaa gcgctaacct tttagcagaa gctaaaaagc 180
taaatgatgc tcaggcgccg aaagtagaca acaaattcaa caaagaacaa caaaacgcgt 240
tctatgagat cttacattta cctaacttaa acgaagaaca acgaaacgcc ttcatccaaa 300
gtttaaaaga tgacccaagc caaagcgcta acctttttagc agaagctaaa aagctaaatg 360
atgctcaggc gccgaaagta gacgcgaatt cgagctcggt accccatatg attac 415

<210> 10
<211> 556
<212> DNA
<213> synthetic

<400> 10

gcgcgcatat gatgacttta caaatacata cagggggtat taatttgaaa aagaaaaaca 60
tttattcaat tcgtaaacta ggtgtaggta ttgcatctgt aactttaggt acattactta 120
tatctggtgg cgtaacacct gctgcaaatg ctgcgcaaca cgatgaagcc gtagacaaca 180
aattcaacaa agaacaacaa aacgcgttct atgagatctt acatttacct aacttaaacg 240
aagaacaacg aaacgccttc atccaaagtt taaaagatga cccaagccaa agcgctaacc 300
ttttagcaga agctaaaaag ctaaatgatg ctcaggcgcc gaaagtagac aacaaattca 360
acaaagaaca acaaaacgcg ttctatgaga tcttacattt acctaactta aacgaagaac 420
aacgaaacgc cttcatccaa agtttaaaag atgacccaag ccaaagcgct aaccttttag 480
cagaagctaa aaagctaaat gatgctcagg cgccgaaagt agacgcgaat tcgagctcgg 540
taccccatat gattac 556

<210> 11
<211> 698
<212> PRT
<213> synthetic

<400> 11

Met Ala Gln His Asp Glu Ala Val Asp Asn Lys Phe Asn Lys Glu Gln
1 5 10 15

Gln Asn Ala Phe Tyr Glu Ile Leu His Leu Pro Asn Leu Asn Glu Glu
20 25 30

Gln Arg Asn Ala Phe Ile Gln Ser Leu Lys Asp Asp Pro Ser Gln Ser
35 40 45

Ala Asn Leu Leu Ala Glu Ala Lys Lys Leu Asn Asp Ala Gln Ala Pro
50 55 60

Lys Val Asp Asn Lys Phe Asn Lys Glu Gln Gln Asn Ala Phe Tyr Glu
65 70 75 80

Ile Leu His Leu Pro Asn Leu Asn Glu Glu Gln Arg Asn Ala Phe Ile
85 90 95

Gln Ser Leu Lys Asp Asp Pro Ser Gln Ser Ala Asn Leu Leu Ala Glu
100 105 110

Ala Lys Lys Leu Asn Asp Ala Gln Ala Pro Lys Val Asp Ala Asn Ser
115 120 125

Ser Ser Val Pro His Met His His His His His His Ser Gln Lys Asn
130 135 140

Asn Asn Glu Leu Pro Lys Gln Ala Ala Glu Asn Thr Leu Asn Leu Asn
145 150 155 160

Pro Val Ile Gly Ile Arg Gly Lys Asp Leu Leu Thr Ser Ala Arg Met
165 170 175

Val Leu Leu Gln Ala Val Arg Gln Pro Leu His Ser Ala Arg His Val
180 185 190

Ala His Phe Ser Leu Glu Leu Lys Asn Val Leu Leu Gly Gln Ser Glu
195 200 205

Leu Arg Pro Gly Asp Asp Asp Arg Arg Phe Ser Asp Pro Ala Trp Ser
210 215 220

Gln Asn Pro Leu Tyr Lys Arg Tyr Met Gln Thr Tyr Leu Ala Trp Arg
225 230 235 240

Lys Glu Leu His Ser Trp Ile Ser His Ser Asp Leu Ser Pro Gln Asp
245 250 255

Ile Ser Arg Gly Gln Phe Val Ile Asn Leu Leu Thr Glu Ala Met Ser
260 265 270

Pro Thr Asn Ser Leu Ser Asn Pro Ala Ala Val Lys Arg Phe Phe Glu
275 280 285

Thr Gly Gly Lys Ser Leu Leu Asp Gly Leu Gly His Leu Ala Lys Asp
290 295 300

Leu Val Asn Asn Gly Gly Met Pro Ser Gln Val Asp Met Asp Ala Phe
305 310 315 320

Glu Val Gly Lys Asn Leu Ala Thr Thr Glu Gly Ala Val Val Phe Arg
325 330 335

```
Asn Asp Val Leu Glu Leu Ile Gln Tyr Arg Pro Ile Thr Glu Ser Val
            340                 345                 350

His Glu Arg Pro Leu Leu Val Val Pro Pro Gln Ile Asn Lys Phe Tyr
        355                 360                 365

Val Phe Asp Leu Ser Pro Asp Lys Ser Leu Ala Arg Phe Cys Leu Arg
    370                 375                 380

Asn Gly Val Gln Thr Phe Ile Val Ser Trp Arg Asn Pro Thr Lys Ser
385                 390                 395                 400

Gln Arg Glu Trp Gly Leu Thr Thr Tyr Ile Glu Ala Leu Lys Glu Ala
                405                 410                 415

Ile Glu Val Val Leu Ser Ile Thr Gly Ser Lys Asp Leu Asn Leu Leu
            420                 425                 430

Gly Ala Cys Ser Gly Gly Ile Thr Thr Ala Thr Leu Val Gly His Tyr
        435                 440                 445

Val Ala Ser Gly Glu Lys Lys Val Asn Ala Phe Thr Gln Leu Val Ser
    450                 455                 460

Val Leu Asp Phe Glu Leu Asn Thr Gln Val Ala Leu Phe Ala Asp Glu
465                 470                 475                 480

Lys Thr Leu Glu Ala Ala Lys Arg Arg Ser Tyr Gln Ser Gly Val Leu
                485                 490                 495

Glu Gly Lys Asp Met Ala Lys Val Phe Ala Trp Met Arg Pro Asn Asp
            500                 505                 510

Leu Ile Trp Asn Tyr Trp Val Asn Asn Tyr Leu Leu Gly Asn Gln Pro
        515                 520                 525

Pro Ala Phe Asp Ile Leu Tyr Trp Asn Asn Asp Thr Thr Arg Leu Pro
    530                 535                 540

Ala Ala Leu His Gly Glu Phe Val Glu Leu Phe Lys Ser Asn Pro Leu
545                 550                 555                 560

Asn Arg Pro Gly Ala Leu Glu Val Ser Gly Thr Pro Ile Asp Leu Lys
                565                 570                 575

Gln Val Thr Cys Asp Phe Tyr Cys Val Ala Gly Leu Asn Asp His Ile
            580                 585                 590

Thr Pro Trp Glu Ser Cys Tyr Lys Ser Ala Arg Leu Leu Gly Gly Lys
        595                 600                 605
```

Cys Glu Phe Ile Leu Ser Asn Ser Gly His Ile Gln Ser Ile Leu Asn
610 615 620

Pro Pro Gly Asn Pro Lys Ala Arg Phe Met Thr Asn Pro Glu Leu Pro
625 630 635 640

Ala Glu Pro Lys Ala Trp Leu Glu Gln Ala Gly Lys His Ala Asp Ser
645 650 655

Trp Trp Leu His Trp Gln Gln Trp Leu Ala Glu Arg Ser Gly Lys Thr
660 665 670

Arg Lys Ala Pro Ala Ser Leu Gly Asn Lys Thr Tyr Pro Ala Gly Glu
675 680 685

Ala Ala Pro Gly Thr Tyr Val His Glu Arg
690 695

<210> 12
<211> 744
<212> PRT
<213> synthetic

<400> 12

Met Thr Leu Gln Ile His Thr Gly Gly Ile Asn Leu Lys Lys Lys Asn
1               5                   10                  15

Ile Tyr Ser Ile Arg Lys Leu Gly Val Gly Ile Ala Ser Val Thr Leu
            20                  25                  30

Gly Thr Leu Leu Ile Ser Gly Gly Val Thr Pro Ala Ala Asn Ala Ala
        35                  40                  45

Gln His Asp Glu Ala Val Asp Asn Lys Phe Asn Lys Glu Gln Gln Asn
    50                  55                  60

Ala Phe Tyr Glu Ile Leu His Leu Pro Asn Leu Asn Glu Glu Gln Arg
65                  70                  75                  80

Asn Ala Phe Ile Gln Ser Leu Lys Asp Asp Pro Ser Gln Ser Ala Asn
                85                  90                  95

Leu Leu Ala Glu Ala Lys Lys Leu Asn Asp Ala Gln Ala Pro Lys Val
            100                 105                 110

Asp Asn Lys Phe Asn Lys Glu Gln Gln Asn Ala Phe Tyr Glu Ile Leu
        115                 120                 125

His Leu Pro Asn Leu Asn Glu Glu Gln Arg Asn Ala Phe Ile Gln Ser
    130                 135                 140

Leu Lys Asp Asp Pro Ser Gln Ser Ala Asn Leu Leu Ala Glu Ala Lys

145 150 155 160

Lys Leu Asn Asp Ala Gln Ala Pro Lys Val Asp Ala Asn Ser Ser Ser
165 170 175

Val Pro His Met His His His His His His Ser Gln Lys Asn Asn Asn
180 185 190

Glu Leu Pro Lys Gln Ala Ala Glu Asn Thr Leu Asn Leu Asn Pro Val
195 200 205

Ile Gly Ile Arg Gly Lys Asp Leu Leu Thr Ser Ala Arg Met Val Leu
210 215 220

Leu Gln Ala Val Arg Gln Pro Leu His Ser Ala Arg His Val Ala His
225 230 235 240

Phe Ser Leu Glu Leu Lys Asn Val Leu Leu Gly Gln Ser Glu Leu Arg
245 250 255

Pro Gly Asp Asp Asp Arg Arg Phe Ser Asp Pro Ala Trp Ser Gln Asn
260 265 270

Pro Leu Tyr Lys Arg Tyr Met Gln Thr Tyr Leu Ala Trp Arg Lys Glu
275 280 285

Leu His Ser Trp Ile Ser His Ser Asp Leu Ser Pro Gln Asp Ile Ser
290 295 300

Arg Gly Gln Phe Val Ile Asn Leu Leu Thr Glu Ala Met Ser Pro Thr
305 310 315 320

Asn Ser Leu Ser Asn Pro Ala Ala Val Lys Arg Phe Phe Glu Thr Gly
325 330 335

Gly Lys Ser Leu Leu Asp Gly Leu Gly His Leu Ala Lys Asp Leu Val
340 345 350

Asn Asn Gly Gly Met Pro Ser Gln Val Asp Met Asp Ala Phe Glu Val
355 360 365

Gly Lys Asn Leu Ala Thr Thr Glu Gly Ala Val Val Phe Arg Asn Asp
370 375 380

Val Leu Glu Leu Ile Gln Tyr Arg Pro Ile Thr Glu Ser Val His Glu
385 390 395 400

Arg Pro Leu Leu Val Val Pro Pro Gln Ile Asn Lys Phe Tyr Val Phe
405 410 415

Asp Leu Ser Pro Asp Lys Ser Leu Ala Arg Phe Cys Leu Arg Asn Gly

420 425 430

Val Gln Thr Phe Ile Val Ser Trp Arg Asn Pro Thr Lys Ser Gln Arg
435 440 445

Glu Trp Gly Leu Thr Thr Tyr Ile Glu Ala Leu Lys Glu Ala Ile Glu
450 455 460

Val Val Leu Ser Ile Thr Gly Ser Lys Asp Leu Asn Leu Leu Gly Ala
465 470 475 480

Cys Ser Gly Gly Ile Thr Thr Ala Thr Leu Val Gly His Tyr Val Ala
485 490 495

Ser Gly Glu Lys Lys Val Asn Ala Phe Thr Gln Leu Val Ser Val Leu
500 505 510

Asp Phe Glu Leu Asn Thr Gln Val Ala Leu Phe Ala Asp Glu Lys Thr
515 520 525

Leu Glu Ala Ala Lys Arg Arg Ser Tyr Gln Ser Gly Val Leu Glu Gly
530 535 540

Lys Asp Met Ala Lys Val Phe Ala Trp Met Arg Pro Asn Asp Leu Ile
545 550 555 560

Trp Asn Tyr Trp Val Asn Asn Tyr Leu Leu Gly Asn Gln Pro Pro Ala
565 570 575

Phe Asp Ile Leu Tyr Trp Asn Asn Asp Thr Thr Arg Leu Pro Ala Ala
580 585 590

Leu His Gly Glu Phe Val Glu Leu Phe Lys Ser Asn Pro Leu Asn Arg
595 600 605

Pro Gly Ala Leu Glu Val Ser Gly Thr Pro Ile Asp Leu Lys Gln Val
610 615 620

Thr Cys Asp Phe Tyr Cys Val Ala Gly Leu Asn Asp His Ile Thr Pro
625 630 635 640

Trp Glu Ser Cys Tyr Lys Ser Ala Arg Leu Leu Gly Gly Lys Cys Glu
645 650 655

Phe Ile Leu Ser Asn Ser Gly His Ile Gln Ser Ile Leu Asn Pro Pro
660 665 670

Gly Asn Pro Lys Ala Arg Phe Met Thr Asn Pro Glu Leu Pro Ala Glu
675 680 685

Pro Lys Ala Trp Leu Glu Gln Ala Gly Lys His Ala Asp Ser Trp Trp

690 695 700

Leu His Trp Gln Gln Trp Leu Ala Glu Arg Ser Gly Lys Thr Arg Lys
705 710 715 720

Ala Pro Ala Ser Leu Gly Asn Lys Thr Tyr Pro Ala Gly Glu Ala Ala
725 730 735

Pro Gly Thr Tyr Val His Glu Arg
740

<210> 13
<211> 28
<212> DNA
<213> primer

<400> 13
gcactagtat gaccacggtc tcgattac 28

<210> 14
<211> 23
<212> DNA
<213> primer

<400> 14
taactagtct gctgaacggc gtc 23

<210> 15
<211> 2286
<212> DNA
<213> synthetic

<400> 15

atgaccacgg tctcgattac ggccagcgct tcggcagacc cctccaagga ctcgaaggcc 60
caggtctcgg ccgccgaggc cggcatcacc ggcacctggt acaaccagct cggctcgacc 120
ttcatcgtga ccgcgggcgc cgacggcgcc ctgaccggaa cctacgagtc ggccgtcggc 180
aacgccgaga gccgctacgt cctgaccggt cgttacgaca gcgccccggc caccgacggc 240
agcggcaccg ccctcggttg gacggtggcc tggaagaata actaccgcaa cgcccactcc 300
gcgaccacgt ggagcggcca gtacgtcggc ggcgccgagg cgaggatcaa cacccagtgg 360
ctgctgacct ccggcaccac cgaggccaac gcctggaagt ccacgctggt cggccacgac 420
accttcacca aggtgaagcc gtccgccgcc tccatcgacg cggcgaagaa ggccggcgtc 480
aacaacggca acccgctcga cgccgttcag cagactagtg cgaccggcaa aggcgcggca 540
gcttccacgc aggaaggcaa gtcccaacca ttcaaggtca cgccggggcc attcgatcca 600
gccacatggc tggaatggtc ccgccagtgg cagggcactg aaggcaacgg ccacgcggcc 660
gcgtccggca ttccgggcct ggatgcgctg gcaggcgtca agatcgcgcc ggcgcagctg 720
ggtgatatcc agcagcgcta catgaaggac ttctcagcgc tgtggcaggc catggccgag 780
ggcaaggccg aggccaccgg tccgctgcac gaccggcgct tcgccggcga cgcatggcgc 840
accaacctcc catatcgctt cgctgccgcg ttctacctgc tcaatgcgcg cgccttgacc 900

gagctggccg atgccgtcga ggccgatgcc aagacccgcc agcgcatccg cttcgcgatc 960
tcgcaatggg tcgatgcgat gtcgcccgcc aacttccttg ccaccaatcc cgaggcgcag 1020
cgcctgctga tcgagtcggg cggcgaatcg ctgcgtgccg gcgtgcgcaa catgatggaa 1080
gacctgacac gcggcaagat ctcgcagacc gacgagagcg cgtttgaggt cggccgcaat 1140
gtcgcggtga ccgaaggcgc cgtggtcttc gagaacgagt acttccagct gttgcagtac 1200
aagccgctga ccgacaaggt gcacgcgcgc ccgctgctga tggtgccgcc gtgcatcaac 1260
aagtactaca tcctggacct gcagccggag agctcgctgg tgcgccatgt ggtggagcag 1320
ggacatacgg tgtttctggt gtcgtggcgc aatccggacg ccagcatggc cggcagcacc 1380
tgggacgact acatcgagca cgcggccatc cgcgccatcg aagtcgcgcg cgacatcagc 1440
ggccaggaca agatcaacgt gctcggcttc tgcgtgggcg gcaccattgt ctcgaccgcg 1500
ctggcggtgc tggccgcgcg cggcgagcac ccggccgcca gcgtcacgct gctgaccacg 1560
ctgctggact ttgccgacac gggcatcctc gacgtctttg tcgacgaggg ccatgtgcag 1620
ttgcgcgagg ccacgctggg cggcggcgcc ggcgcgccgt gcgcgctgct gcgcggcctt 1680
gagctggcca ataccttctc gttcttgcgc ccgaacgacc tggtgtggaa ctacgtggtc 1740
gacaactacc tgaagggcaa cacgccggtg ccgttcgacc tgctgttctg gaacggcgac 1800
gccaccaacc tgccggggcc gtggtactgc tggtacctgc gccacaccta cctgcagaac 1860
gagctcaagg taccgggcaa gctgaccgtg tgcggcgtgc cggtggacct ggccagcatc 1920
gacgtgccga cctatatcta cggctcgcgc gaagaccata tcgtgccgtg gaccgcggcc 1980
tatgcctcga ccgcgctgct ggcgaacaag ctgcgcttcg tgctgggtgc gtcgggccat 2040
atcgccggtg tgatcaaccc gccggccaag aacaagcgca gccactggac taacgatgcg 2100
ctgccggagt cgccgcagca atggctggcc ggcgccatcg agcatcacgg cagctggtgg 2160
ccggactgga ccgcatggct ggccgggcag gccggcgcga aacgcgccgc gcccgccaac 2220
tatggcaatg cgcgctatcg cgcaatcgaa cccgcgcctg ggcgatacgt caaagccaag 2280
gcatga 2286

<210> 16
<211> 1209
<212> DNA
<213> Homo sapiens

<400> 16

atgatcctca ccccggaaca agttgcagca gcgcaaaagg ccaacctcga aacgctgttc 60
ggcctgacca ccaaggcgtt tgaaggcgtc gaaaagctcg tcgagctgaa cctgcaggtc 120
gtcaagactt cgttcgcaga aggcgttgac aacgccaaga aggcgctgtc ggccaaggac 180
gcacaggaac tgctggccat ccaggccgca gccgtgcagc cggttgccga aaagaccctg 240
gcctacaccc gccacctgta tgaaatcgct tcggaaaccc agagcgagtt caccaaggta 300
gccgaggctc aactggccga aggctcgaag aacgtgcaag cgctggtcga gaacctcgcc 360

aagaacgccc cggccggttc ggaatcgacc gtggccatcg tgaagtcggc gatctccgct 420
gccaacaacg cctacgagtc ggtgcagaag gcgaccaagc aagcggtcga aatcgctgaa 480
accaacttcc aggctgcggc tacggctgcc accaaggctg cccagcaagc cagcgccacg 540
gcccgtacgg ccacggcaaa gaagacgacg gctgccgagc tcgagtccga acatgagacc 600
cgtctggtgg caaagctatt taaagactac agcagcgtgg tgcggccagt ggaagaccac 660
cgccaggtcg tggaggtcac cgtgggcctg cagctgatac agctcatcaa tgtggatgaa 720
gtaaatcaga tcgtgacaac caatgtgcgt ctgaaacagc aatgggtgga ttacaaccta 780
aaatggaatc cagatgacta tggcggtgtg aaaaaaattc acattccttc agaaaagatc 840
tggcgcccag accttgttct ctataacaat gcagatggtg actttgctat tgtcaagttc 900
accaaagtgc tcctgcagta cactggccac atcacgtgga cacctccagc catctttaaa 960
agctactgtg agatcatcgt cacccacttt ccctttgatg aacagaactg cagcatgaag 1020
ctgggcacct ggacctacga cggctctgtc gtggccatca acccggaaag cgaccagcca 1080
gacctgagca acttcatgga gagcggggag tgggtgatca aggagtcccg gggctggaag 1140
cactccgtga cctattcctg ctgccccgac accccctacc tggacatcac ctaccacttc 1200
gtcatgtag 1209

<210> 17
<211> 2037
<212> DNA
<213> Homo sapiens

<400> 17

atgatcctca ccccggaaca agttgcagca gcgcaaaagg ccaacctcga aacgctgttc 60
ggcctgacca ccaaggcgtt tgaaggcgtc gaaaagctcg tcgagctgaa cctgcaggtc 120
gtcaagactt cgttcgcaga aggcgttgac aacgccaaga aggcgctgtc ggccaaggac 180
gcacaggaac tgctggccat ccaggccgca gccgtgcagc cggttgccga aaagaccctg 240
gcctacaccc gccacctgta tgaaatcgct tcggaaaccc agagcgagtt caccaaggta 300
gccgaggctc aactggccga aggctcgaag aacgtgcaag cgctggtcga gaacctcgcc 360
aagaacgccc cggccggttc ggaatcgacc gtggccatcg tgaagtcggc gatctccgct 420
gccaacaacg cctacgagtc ggtgcagaag gcgaccaagc aagcggtcga aatcgctgaa 480
accaacttcc aggctgcggc tacggctgcc accaaggctg cccagcaagc cagcgccacg 540
gcccgtacgg ccacggcaaa gaagacgacg gctgccgagc tcgagatgaa ttcgagctcg 600
aacaacaaca acaataacaa taacaacaac ctcgggatcg agggaaggat ttcagaattc 660
atgagccaag atgtctcctt gctggcatca gactcagagc ccctgaagtg tttctcccga 720
acatttgagg acctcacttg cttctgggat gaggaagagg cagcgcccag tgggacatac 780
cagctgctgt atgcctaccc gcgggagaag ccccgtgctt gcccccctgag ttcccagagc 840
atgccccact ttggaacccg atacgtgtgc cagtttccag accaggagga agtgcgtctc 900
ttctttccgc tgcacctctg ggtgaagaat gtgttcctaa accagactcg gactcagcga 960

gtcctctttg tggacagtgt aggcctgccg gctcccccca gtatcatcaa ggccatgggt 1020
gggagccagc caggggaact tcagatcagc tgggaggagc cagctccaga aatcagtgat 1080
ttcctgaggt acgaactccg ctatggcccc agagatccca agaactccac tggtcccacg 1140
gtcatacagc tgattgccac agaaacctgc tgccctgctc tgcagaggcc tcactcagcc 1200
tctgctctgg accagtctcc atgtgctcag cccacaatgc cctggcaaga tggaccaaag 1260
cagacctccc caagtagaga agcttcagct ctgacagcag agggtggaag ctgcctcatc 1320
tcaggactcc agcctggcaa ctcctactgg ctgcagctgc gcagcgaacc tgatgggatc 1380
tccctcggtg gctcctgggg atcctggtcc ctccctgtga ctgtggacct gcctggagat 1440
gcagtggcac ttggactgca atgctttacc ttggacctga agaatgttac ctgtcaatgg 1500
cagcaacagg accatgctag ctcccaaggc ttcttctacc acagcagggc acggtgctgc 1560
cccagagaca ggtaccccat ctgggagaac tgcgaagagg aagagaaaac aaatccagga 1620
ctacagaccc cacagttctc tcgctgccac ttcaagtcac gaaatgacag cattattcac 1680
atccttgtgg aggtgaccac agccccgggt actgttcaca gctacctggg ctcccctttc 1740
tggatccacc aggctgtgcg cctccccacc ccaaacttgc actggaggga gatctccagt 1800
gggcatctgg aattggagtg gcagcaccca tcgtcctggg cagcccaaga gacctgttat 1860
caactccgat acacaggaga aggccatcag gactggaagg tgctggagcc gcctctcggg 1920
gcccgaggag ggaccctgga gctgcgcccg cgatctcgct accgtttaca gctgcgcgcc 1980
aggctcaacg gccccaccta ccaaggtccc tggagctcgt ggtcggaccc aacttaa 2037

<210> 18
<211> 44
<212> DNA
<213> primer

<400> 18
ggctactcga gatgaattcg agctcgaaca acaacaacaa taac 44

<210> 19
<211> 40
<212> DNA
<213> primer

<400> 19
cagcttcgaa ttaagttggg tccgaccacg agctccaggg 40

<210> 20
<211> 25
<212> DNA
<213> adapter

<400> 20
tatggctctg cactagtcac tgcca 25

<210> 21
<211> 24
<212> DNA
<213> adaptor reverse

<400> 21
tatggcagtg actagtgcag agca 24

<210> 22
<211> 33
<212> DNA
<213> oligonucleotide

<400> 22
ggactagtat gaccatgatt acggattcac tgg 33

<210> 23
<211> 36
<212> DNA
<213> oligonucleotide

<400> 23
caactagttt tttgacacca gaccaactgg taattg 36

<210> 24
<211> 20
<212> DNA
<213> T7 promoter

<400> 24
taatacgact cactataggg 20